# EUROPÄISCHE PATENTANMELDUNG

(11) **EP 0 694 543 A1**
(43) Veröffentlichungstag der Anmeldung: **31.01.1996**
(21) Anmeldenummer: 95110624.4
(22) Anmeldetag: 07.07.1995
(51) Int. Cl.: C07D 413/04, C07D 413/14, A61K 31/42

(54) **6-gliedrige stickstoffhaltige Heteroaryl-Oxazolidinone**

(30) Priorität: 20.07.1994 DE 4425612
(71) Anmelder: BAYER AG, D-51368 Leverkusen (DE)
(72) Erfinder: Riedl, Bernd, Dr., D-42115 Wuppertal (DE); Häbich, Dieter, Dr., D-42115 Wuppertal (DE); Stolle, Andreas, Dr., D-42115 Wuppertal (DE); Wild, Hanno, Dr., Orange, Connecticut 06477 (US); Endermann, Rainer, Dr., D-42113 Wuppertal (DE); Bremm, Klaus Dieter, Dr., D-45661 Recklinghausen (DE); Kroll, Hein-Peter, Dr., D-42115 Wuppertal (DE); Labischinski, Harald, Prof.Dr., D-42109 Wuppertal (DE); Schaller, Klaus, Dr., D-42109 Wuppertal (DE); Werling, Hans-Otto, Dr., D-42113 Wuppertal (DE)

(57) **Zusammenfassung**

Die Erfindung betrifft 6-gliedrige stickstoffhaltige Heteroaryloxazolidinone, Verfahren zu ihrer Herstellung und ihre Verwendung als Arzneimittel, insbesondere als antibakterielle Arzneimittel.

## Beschreibung

Die vorliegende Erfindung betrifft 6-gliedrige stickstoffhaltige Heteroaryloxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, US 4 801 600, US 4 921 869, US 4 965 268, EP 312 000 und C.H. Park et al., J. Med. Chem. 35, 1156 (1992) sind N-Aryloxazolidinone mit antibakterieller Wirkung bekannt.

Außerdem werden Verbindungen der allgemeinen Formel (I) (D = Pyridyl und R¹ = Hydroxy) von einer allgemeinen Zwischenproduktformel in der PCT WO 93/22298 umfaßt, wobei dort weder konkrete Stoffvertreter noch eine pharmakologische Wirkung beschrieben werden.

Die vorliegende Erfindung betrifft 6-gliedrige stickstoffhaltige Heteroaryloxazolidinone der allgemeinen Formel (I) 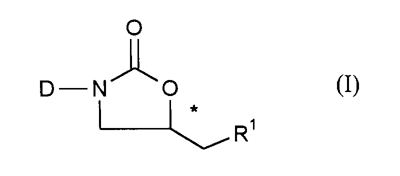
in welcher
- R¹: für Azido, Hydroxy oder für eine Gruppe der Formel -OR², O-SO₂R³ oder -NR⁴R⁵ steht,
worin
- R²: geradkettiges oder verzweigtes Acyl mit bis zu 8 Kohlenstoffatomen oder eine Hydroxyschutzgruppe bedeutet,
- R³: geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
- R⁴ und R⁵: gleich oder verschieden sind und
Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten,
oder
- R⁴ oder R⁵: eine Gruppe der Formel -CO-R⁶ bedeutet,
worin
- R⁶: Cycloalkyl mit 3 bis 6 Kohlenstoffatomen, geradkettiges oder verzweigtes Alkyl mit bis zu 8 Kohlenstoffatomen, Phenyl oder Wasserstoff bedeutet,

- D: für einen über ein Kohlenstoffatom direkt gebundenen 6-gliedrigen, aromatischen Heterocyclus mit mindestens einem Stickstoffatom steht, oder für einen über ein Kohlenstoffatom, jeweils 6-gliedrigen, bi- oder tricyclischen aromatischen Rest mit mindestens einem stickstoffhaltigem Ring steht, oder
für β-Carbolin-3-yl oder für über den 6-Ring direkt gebundenes Indolizinyl steht, wobei die Cyclen gegebenenfalls jeweils bis zu 3-fach gleich oder verschieden durch Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR⁷R⁸ substituiert sein kann,
worin
- R⁷ und R⁸: gleich oder verschieden sind und
Wasserstoff Formyl, geradkettiges oder verzweigtes Alkyl oder Acyl mit jeweils bis zu 4 Kohlenstoffatomen, Phenyl oder Cycloalkyl mit 3 bis 6 Kohlenstoffatomen bedeuten,
oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch Phenyl, Pyrimidyl oder geradkettiges oder verzweigtes Alkyl oder Acyl mit jeweils bis zu 3 Kohlenstoffatomen substituiert sein kann,
und/oder
die Cyclen gegebenenfalls durch eine Gruppe der Formel -NR⁷'R⁸' substituiert sind,
worin
- R^{7'} und R^{8'}: gleich oder verschieden sind und die oben angegebene Bedeutung von R⁷ und R⁸ haben und mit dieser gleich oder verschieden sind,
und/oder
die Cyclen gegebenenfalls durch (C₂-C₈)-Alkenylphenyl, Phenyl oder durch einen 5- oder 6-gliedrigen gesättigten oder ungesättigten Heterocyclus mit bis zu 3 Heteroatomen aus der Reihe S, N und/oder O substituiert sind, die ihrerseits gegebenenfalls durch eine Gruppe der Formel -CO-NR⁹R¹⁰, -NR¹¹R¹², -NR¹³-SO₂-R¹⁴, R¹⁵R¹⁶N-SO₂- R¹⁷-S(O)ₐ- R¹⁸-N=CH- oder durch den Rest -CH(OH)-SO₃R²⁰ substituiert sind,
worin
- a: eine Zahl 0, 1 oder 2 bedeutet,
- R⁹, R¹⁰, R¹³, R¹⁵ und R¹⁶: gleich oder verschieden sind und Wasserstoff geradkettiges oder verzweigtes Alkyl mit bis zu 6 Kohlenstoffatomen, Tolyl oder Phenyl bedeuten,
- R¹¹ und R¹²: gleich oder verschieden sind und die oben angegebene Bedeutung von R⁷ und R⁸ haben und mit dieser gleich oder verschieden sind,
- R¹⁴ und R¹⁷: gleich oder verschieden sind und die oben angegebene Bedeutung von R³ haben und mit dieser gleich oder verschieden sind,
- R¹⁸: Hydroxy, Benzyloxy oder einen Rest der Formel -NH-CO-NH₂, 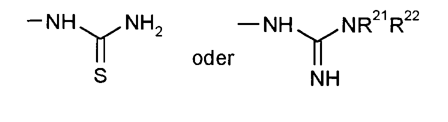 bedeutet,
worin
- R²¹ und R²²: gleich oder verschieden sind und Wasserstoff oder geradkettiges oder verzweigtes Alkyl mit bis zu 4 Kohlenstoffatomen bedeuten, das seinerseits durch Phenyl oder Pyridyl substituiert sein kann
- R²⁰: Wasserstoff oder ein Natriumion bedeutet,
und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, Azido durch geradkettiges oder verzweigtes Alkoxy oder Acyl mit bis zu 5 Kohlenstoffatomen oder durch eine Gruppe der Formel -NR²³R²⁴, R²⁵-S-, R²⁶-SO₂O- oder 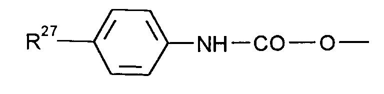 substituiert sein kann,
worin
- R²³ und R²⁴: die oben angegebene Bedeutung von R⁷ und R⁸ haben und mit dieser gleich oder verschieden sind,
oder einen Rest der Formel -P(O) (OR²⁸) (OR²⁹) oder R³⁰-SO₂- bedeuten,
worin
- R²⁸ und R²⁹: gleich oder verschieden sind und Wasserstoff oder geradkettiges oder verzweigtes Alkyl mit bis zu 3 Kohlenstoffatomen bedeuten
- R³⁰: Methyl, Phenyl oder Tolyl bedeutet,
- R²⁵: einen Rest der Formel 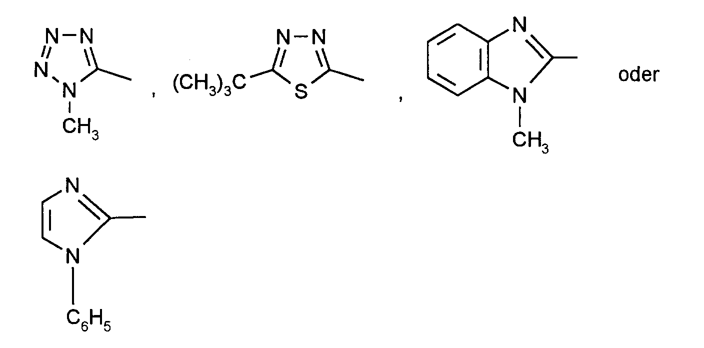 bedeutet,
- R²⁶: geradkettiges oder verzweigtes Alkyl mit bis zu 3 Kohlenstoffatomen bedeutet,
- R²⁷: geradkettiges oder verzweigtes Alkoxycarbonyl mit bis zu 6 Kohlenstoffatomen oder Carboxy bedeutet
und/oder
die Cyclen gegebenenfalls durch einen Rest der Formel 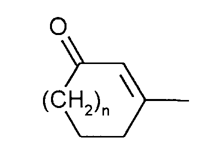 substituiert sind,
worin
- n: eine Zahl 0, 1 oder 2 bedeutet
und deren Salze und N-Oxide.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren in bekannter Weise in die stereoisomer einheitlichen Bestandteile trennen.

Physiologisch unbedenkliche Salze der 6-gliedrigen Heteroaryl-oxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Als Salze können außerdem C₁-C₄-Alkylhalogenide, insbesondere C₁-C₄-Alkyljodide fungieren.

Heterocyclus unter dem Substituenten D im Fall der direkten Anbindung an das Oxazolidinongerüst steht im Rahmen der Erfindung im allgemeinen für einen über ein Kohlenstoffatom direkt gebundenen 6-gliedrigen, aromatischen Heterocyclus mit mindestens einem Stickstoffatom, oder für einen über ein Kohlenstoffatom, jeweils 6-gliedrigen, bi- oder tricyclischen aromatischen Rest mit mindestens einem stickstoffhaltigem Ring, oder für β-Carbolin-3-yl oder für über den 6-Ring direkt gebundenes Indolizinyl. Beispielsweise seien genannt Cinnolinyl, Pteridinyl, Phenanthridinyl, Acridinyl, Phenanthrolinyl, Chinazolinyl, Chinoxalinyl, Naphthyridinyl, Phthalazinyl, Chinolyl, Isochinolyl, 4H-Chinolizinyl, Phenazinyl, Pyridyl, Pyrazinyl, Pyrimidinyl, Pyridazinyl, Indolizinyl, β-Carbolin-3-yl und über den 6-Ring direkt gebundenes Indolizinyl.

Im weiteren Substitutionsfeld steht Heteroyclus auch für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Dazu gehören auch über N-gebundene, 5- bis 6-gliedrige gesättigte Heterocyclen, die außerdem als Heteroatome bis zu 2 Sauerstoff-, Schwefel- und/oder Stickstoffatome enthalten können, wie beispielsweise Piperidyl, Morpholinyl oder Piperazin oder Pyrrolidinyl. Besonders bevorzugt sind Piperidyl und Pyrrolidinyl.

Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, AIlyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl oder Tetrahydropyranyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Bevorzugt sind Verbindungen der allgemeinen Formel (I),
in welcher
- R¹: für Azido, Hydroxy oder für eine Gruppe der Formel -OR², -OSO₂R³ oder -NR⁴R⁵ steht,
worin
- R²: geradkettiges oder verzweigtes Acyl mit bis zu 6 Kohlenstoffatomen oder Benzyl bedeutet,
- R³: geradkettiges oder verzweigtes Alkyl mit bis zu 3 Kohlenstoffatomen, Phenyl oder Toluolyl bedeutet,
- R⁴ und R⁵: gleich oder verschieden sind und
Cyclopropyl, Cycylopentyl, Cyclohexyl, Wasserstoff, Phenyl oder geradkettiges oder verzweigtes Alkyl mit bis zu 6 Kohlenstoffatomen, tert. Butoxycarbonyl oder Benzyloxycarbonyl bedeuten,
oder
- R⁴ oder R⁵: eine Gruppe der Formel -CO-R⁶ bedeutet,
worin
- R⁶: Cyclopropyl, Cyclobutyl, Cyclopentyl, Cyclohexyl oder geradkettiges oder verzweigtes Alkyl mit bis zu 6 Kohlenstoffatomen, Phenyl oder Wasserstoff bedeutet,
- D: für Cinnolinyl, Pteridinyl, Acridinyl, Chinazolinyl, Chinoxalinyl, Naphthyridinyl, Phthalazinyl, Chinolyl, Isochinolyl, Pyridyl, Pyrazinyl, Pyrimidinyl oder Pyridazinyl steht, wobei die Cyclen gegebenenfalls jeweils bis zu 3-fach gleich oder verschieden durch durch Carboxy, Fluor, Chlor, Brom, Jod, Cyano, Mercapto, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR⁷R⁸ gegebenenfalls substituiert sein kann,
worin
- R⁷ und R⁸: gleich oder verschieden sind und
Wasserstoff, Formyl geradkettiges oder verzweigtes Alkyl oder Acyl mit jeweils bis zu 3 Kohlenstoffatomen, Phenyl, Cyclopropyl, Cyclopentyl oder Cyclohexyl bedeuten bedeuten,
oder gemeinsam mit dem Stickstoffatom einen Morpholinyl-, Pyrrolidinyl-, Piperazinyl- oder Piperidylring bilden, die gegebenenfalls, auch über die freie N-Funktion, durch Phenyl, Pyrimidyl, Methyl, Ethyl oder Acetyl substituiert sind,
und/oder die Cyclen gegebenenfalls durch einen Rest der Formel durch eine Gruppe der Formel -NR^{7'}R^{8'} substituiert sind,
worin
- R^{7'} und R^{8'}: die oben angegebene Bedeutung von R⁷ und R⁸ haben und mit dieser gleich oder verschieden sind,
und/oder
die Cyclen gegebenenfalls durch (C₂-C₄)-Alkenylphenyl, Phenyl, Pyridyl oder Thienyl substituiert sind, die ihrerseits gegebenenfalls durch eine Gruppe der Formel -CO-NR⁹R¹⁰, -NR¹¹R¹², -NR¹³-SO₂-R¹⁴, R¹⁵R¹⁶N-SO₂-, R¹⁷-S(O)ₐ-, R¹⁸-N=CH- oder durch den Rest -CH(OH)-SO₃R²⁰ substituiert sind,
worin
- a: eine Zahl 0, 1 oder 2 bedeutet,
- R⁹, R¹⁰, R¹³, R¹⁵ und R¹⁶: gleich oder verschieden sind und
Wasserstoff, geradkettiges oder verzweigtes Alkyl mit bis zu 4 Kohlenstoffatomen, Tolyl oder Phenyl bedeuten,
- R¹¹ und R¹²: gleich oder verschieden sind und die oben angegebene Bedeutung von R⁷ und R⁸ haben und mit dieser gleich oder verschieden sind,
- R¹⁴ und R¹⁷: gleich oder verschieden sind und die oben angegebene Bedeutung von R³ haben und mit dieser gleich oder verschieden sind,
- R¹⁸: Hydroxy, Benzyloxy oder einen Rest der Formel
-NH-CO-NH₂, 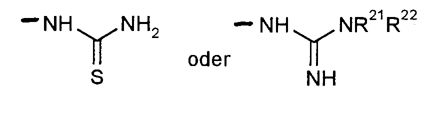 bedeutet,
worin
- R²¹ und R²²: gleich oder verschieden sind und Wasserstoff oder geradkettiges oder verzweigtes Alkyl mit bis zu 3 Kohlenstoffatomen bedeutet, das seinerseits durch Phenyl oder Pyridyl substituiert sein kann,
- R²⁰: Wasserstoff oder ein Natriumion bedeutet
und/oder ihrerseits gegebenenfalls bis zu 2-fach gleich oder verschieden durch Carboxy, Fluor, Chlor, Brom, Jod, Cyano, Mercapto, Trifluormethyl, Formyl, Nitro, Phenyl, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, Azido durch geradkettiges oder verzweigtes Alkoxy oder Acyl mit bis zu 4 Kohlenstoffatomen oder durch eine Gruppe der Formel -NR²³R²⁴, R²⁵-S-, R²⁶-SO₂O- oder 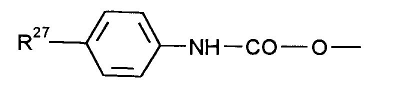 substituiert sein kann,
worin
- R²³und R²⁴: die oben angegebene Bedeutung von R⁷ und R⁸ haben und mit dieser gleich oder verschieden sind,
oder einen Rest der Formel (P) (O) (OR²⁸) (OR²⁹) oder R³⁰-SO₂- bedeuten,
worin
- R²⁸ und R²⁹: gleich oder verschieden sind und Wasserstoff, Methyl oder Ethyl bedeuten,
- R³⁰: Methyl, Phenyl oder Tolyl bedeutet,
- R²⁵: einen Rest der Formel 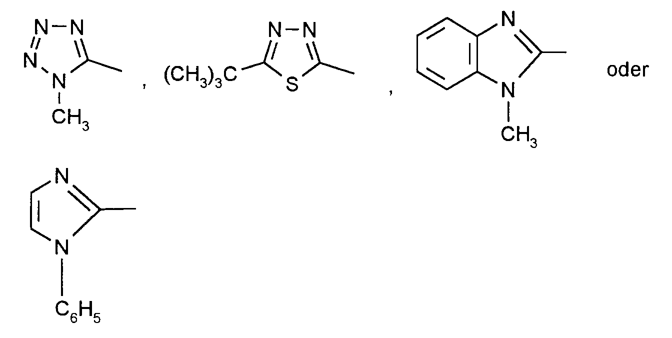 bedeutet,
- R²⁶: Methyl, Ethyl, Propyl oder Isopropyl bedeutet,
- R²⁷: geradkettiges oder verzweigtes Alkoxycarbonyl mit bis zu 5 Kohlenstoffatomen oder Carboxy bedeutet
und/oder
die Cyclen gegebenenfalls durch einen Rest der Formel 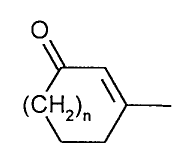 substituiert sind,
worin
- n: eine Zahl 0, 1 oder 2 bedeutet
und deren Salze und N-Oxide.

Besonders bevorzugt sind Verbindungen der allgemeinen Formel (I),
in welcher
- R¹: für Azido, Hydroxy oder für eine Gruppe der Formel -OR², -OSO₂R³ oder -NR⁴R⁵ steht,
worin
- R²: geradkettiges oder verzweigtes Acyl mit bis zu 6 Kohlenstoffatomen bedeutet,
- R³: Methyl, Ethyl, Phenyl oder Toluolyl bedeutet,
- R⁴ und R⁵: gleich oder verschieden sind und
Cyclopropyl, Cyclopentyl, Cyclohexyl, Wasserstoff, Phenyl oder geradkettiges oder verzweigtes Alkyl mit bis zu 5 Kohlenstoffatomen bedeuten,
oder
- R⁴ oder R⁵: eine Gruppe der Formel -CO-R⁶ bedeutet,
worin
- R⁶: Cyclopropyl, Cyclopentyl, Cyclohexyl oder geradkettiges oder verzweigtes Alkyl mit bis zu 4 Kohlenstoffatomen, Wasserstoff oder Phenyl bedeutet,
- D: für Cinnolinyl, Chinoxalinyl, Naphthyridinyl, Phthalazinyl, Chinolyl, Isochinolyl, Pyridyl, Pyrazinyl, Pyrimidinyl oder Pyridazinyl steht, wobei die Cyclen gegebenenfalls jeweils bis zu 2-fach gleich oder verschieden durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR⁷R⁸ gegebenenfalls substituiert sein kann,
worin
- R⁷ und R⁸: gleich oder verschieden sind und Wasserstoff, Formyl, Acetyl, Methyl oder Cylopropyl bedeuten,
oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl, Phenyl, Pyrimidyl oder Acetyl substituiert sind,
und/oder die Cyclen gegebenenfalls durch eine Gruppe der Formel -NR⁷'R^{8'} substituiert sind,
worin
- R^{7'} und R^{8'}: die oben angegebene Bedeutung von R⁷ und R⁸ haben und mit dieser gleich oder verschieden sind,
und/oder
die Cyclen gegebenenfalls durch 2-Phenylvinyl, Phenyl, Pyridyl oder Thienyl substituiert sind, die ihrerseits gegebenenfalls durch eine Gruppe der Formel -CO-NR⁹R¹⁰ , -NR¹¹R¹², R¹⁸-N=CH- oder durch den Rest -CH(OH)-SO₃-R²⁰ substituiert sind,
worin
- R⁹ und R¹⁰: gleich oder verschieden sind und Wasserstoff oder Methyl bedeuten,
- R¹¹ und R¹²: gleich oder verschieden sind und die oben angegebene Bedeutung von R⁷ und R⁸ haben und mit dieser gleich oder verschieden sind,
- R¹⁸: Hydroxy, Benzyloxy oder einen Rest der Formel -NH-CO-NH₂, 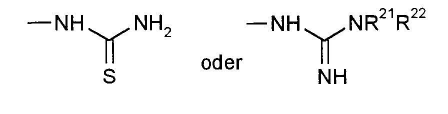 oder bedeutet,
worin
- R²¹ und R²²: gleich oder verschieden sind und Wasserstoff, Methyl oder Ethyl bedeuten, die ihrerseits durch Phenyl oder Pyridyl substituiert sein können,
- R²⁰: Wasserstoff oder ein Natriumion bedeutet
und/oder ihrerseits gegebenenfalls bis zu 2-fach gleich oder verschieden durch Carboxy, Fluor, Chlor, Brom, Jod, Cyano, Formyl, Trifluormethyl, Nitro, Phenyl, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, Azido, durch geradkettiges oder verzweigtes Alkoxy oder Acyl mit bis zu 4 Kohlenstoffatomen oder durch eine Gruppe der Formel -NR²³R²⁴ , R²⁵-S-, R²⁶-SO₂O- oder 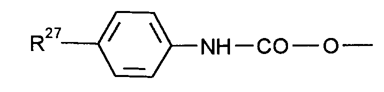 substituiert sein kann,
worin
- R²³ und R²⁴: die oben angegebene Bedeutung von R⁷ und R⁸ haben und mit dieser gleich oder verschieden sind, oder einen Rest der Formel P(O) (OCH₃)₂ oder R³⁰-SO₂- bedeuten
worin
- R³⁰: Methyl, Phenyl oder Tolyl bedeutet,
- R²⁵: einen Rest der Formel 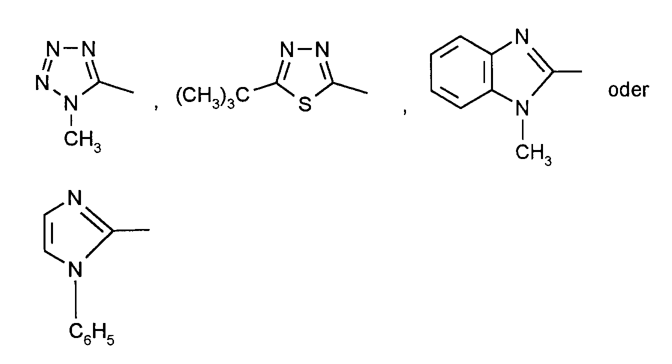 bedeutet,
- R²⁶: Methyl, Ethyl oder Propyl bedeutet,
- R²⁷: geradkettiges oder verzweigtes Alkoxycarbonyl mit bis zu 4 Kohlenstoffatomen bedeutet
und/oder
die Cyclen gegebenenfalls durch einen Rest der Formel 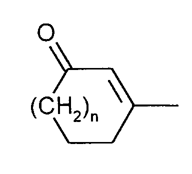 substituiert sind,
worin
- n: eine Zahl 0, 1 oder 2 bedeutet
und deren Salze und N-Oxide.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formeln (II) oder (III)

   D-N=C=O (II)

   oder

   D-CO-N₃ (III)

   in welchen
   - D: die oben angegebene Bedeutungen hat,
   mit Lithiumbromid/(C₄H₉)₃ P(O) und Epoxiden der allgemeinen Formel (IV) 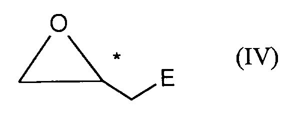 in welcher
   - E: für C₁-C₆-Acyloxy steht,
   in inerten Lösemitteln, gegebenenfalls in Anwesenheit einer Base
   umsetzt,
   und im Fall R¹ = OH durch eine typische Esterverseifung oder durch eine typische Umesterung die Hydroxyfunktion freisetzt,
   oder
[B] Verbindungen der allgemeinen Formel (V)

   D-NH-CO₂-L (V)

   in welcher
   - D: die oben angegebene Bedeutung hat
   und
   - L: für eine typische Schutzgruppe, vorzugsweise Benzyl steht,
   in inerten Lösemitteln und in Anwesenheit einer Base, beispielsweise Lithiumalkylen oder Lithium-N-alkyl- oder Lithium-N-silylalkylamiden, vorzugsweise N-Butyllithium, mit Epoxiden der allgemeinen Formel (IV) umsetzt,
   oder
[C] im Fall R¹ = OH, zunächst Verbindungen der allgemeinen Formel (III) durch Abspaltung von Stickstoff in Alkoholen in die Verbindungen der allgemeinen Formel (Va)

   D-NH-CO₂-T (Va)

   in welcher
   - D: die oben angegebene Bedeutung hat
   und
   - T: für geradkettiges oder verzweigtes C₂-C₆-Alkyl, vorzugsweise n-Butyl steht,
   überführt,
   und in einem zweiten Schritt wie unter [A] beschrieben in inerten Lösemitteln und in Anwesenheit einer Base, vorzugsweise Lithium-N-alkyl-oder N-Silylalkylamiden oder n-Butyllithium und Epoxiden der allgemeinen Formel (IV) umsetzt,
   oder
[D] Verbindungen der allgemeinen Formel (VI) 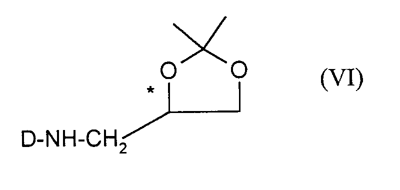 in welcher
   - D: die oben angegebene Bedeutung hat,
   entweder direkt mit Säuren und Kohlensäurediethylester
   umsetzt,
   oder zunächst durch Umsetzung der Verbindungen der allgemeinen Formel (VI) mit Säuren die Verbindungen der allgemeinen Formel (VII) 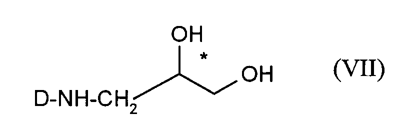 in welcher
   - D: die oben angegebene Bedeutung hat
   herstellt,
   und anschließend in Anwesenheit eines Hilfsmittels in inerten Lösemitteln cyclisiert,
   oder
[E] zunächst Verbindungen der allgemeinen Formel (Ia) 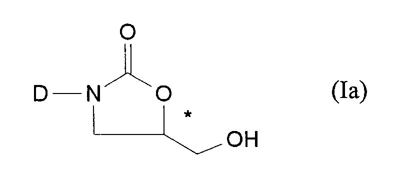 in welcher
   - D: die oben angegebene Bedeutung hat,
   durch Umsetzung mit (C₁-C₄)-Alkyl- oder Phenylsulfonsäurechloriden in inerten Lösemitteln und in Anwesenheit einer Base in die entsprechenden Verbindungen der allgemeinen Formel (Ib) 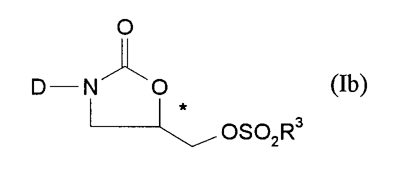 in welcher
   - D und R³: die oben angegebene Bedeutung haben,
   überführt,
   anschließend mit Natriumazid in inerten Lösemitteln die Azide der allgemeinen Formel (Ic) 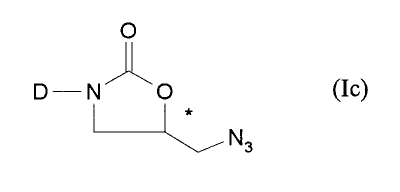 in welcher
   - D: die oben angegebene Bedeutung hat,
   herstellt,
   in einem weiteren Schritt durch Umsetzung mit (C₁-C₄-O)₃-P oder PPh₃, vorzugsweise (CH₃O)₃P in inerten Lösemitteln und mit Säuren in die Amine der allgemeinen Formel (Id) 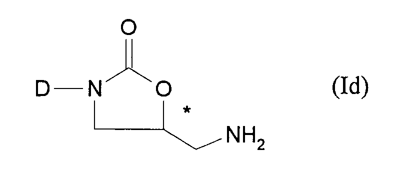 in welcher
   - D: die oben angegebene Bedeutung hat,
   überführt,
   und durch Umsetzung mit Acetanhydrid oder anderen Acylierungsmitteln der allgemeinen Formel (VIII)

   R³¹-CO-R⁶ (VIII)

   in welcher
   - R⁶: die oben angegebene Bedeutung hat
   und
   - R³¹: für Halogen, vorzugsweise für Chlor oder für den Rest -OCOR⁶ steht,
   in inerten Lösemitteln die Verbindungen der allgemeinen Formel (Ie) 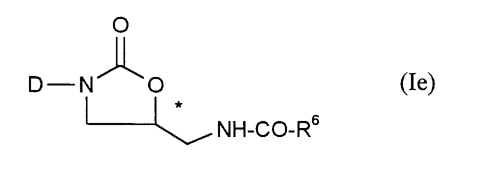 in welcher
   - D und R⁶: die oben angegebene Bedeutung haben
   herstellt,
   oder
[F] Verbindungen der allgemeinen Formel (Ie) durch eine Halogenierung, gegebenenfalls in Anwesenheit eines Silberkatalysators, in die Verbindungen der allgemeinen Formel (If) 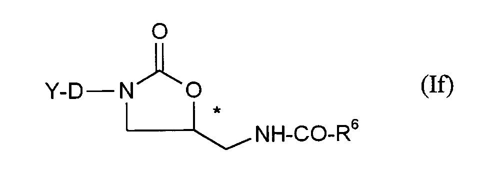 in welcher
   - Y: für Halogen, vorzugsweise für Brom oder Jod steht,
   und
   - D und R⁶: die oben angegebene Bedeutung haben,
   überführt,
   oder
[G] Verbindungen der allgemeinen Formel (If) mit Verbindungen der allgemeinen Formel (IX)

   D'-R³² (IX)

   in welcher
   - D': für einen der unter D oben aufgeführten, gegebenenfalls substituierten, monocyclischen Heterocyclen, Phenyl oder (C₂-C₈)-Alkenylphenyl steht,
   und
   - R³²: für den Boronsäurerest -B(OH)₂ oder
   für einen zinnorganischen Rest der Formel -SnR³³R³⁴R³⁵ steht,
   worin
   R³³, R³⁴ und R³⁵ gleich oder verschieden sind und C₁-C₄-Alkyl bedeuten,
   in inerten Lösemitteln und in Anwesenheit eines Palladiumkatalysators umsetzt,
   und im Fall der N-Oxide eine Oxidation durchführt,
   und im Fall R⁴, R⁵, R⁹, R¹⁰, R¹¹, R¹², R¹³, R¹⁵, R¹⁶, R¹⁸ und R¹⁹ ≠ H eine Alkylierung nach üblichen Methoden durchführt,
   und gegebenenfalls weitere Substituenten oder bereits vorhandene funktionelle Gruppen nach üblichen Methoden, wie beispielsweise Redoxreaktionen, Substitutionsreaktionen und/oder Verseifungen oder Ein- und Abbau von Schutzgruppen, einführt bzw. derivatisiert.

Die erfindungsgemäßen Verfahren können durch folgende Formelschemata beispielhaft erläutert werden:

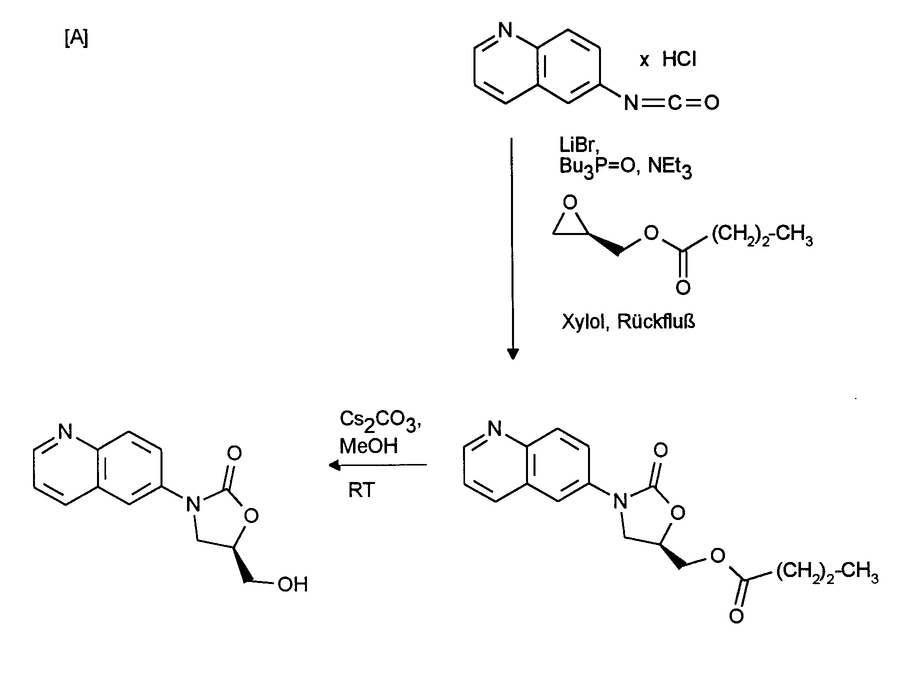

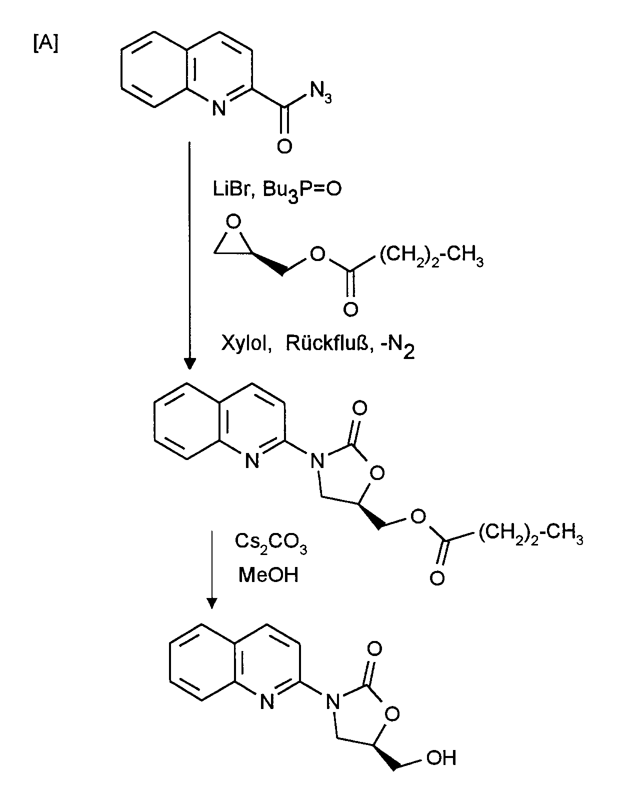

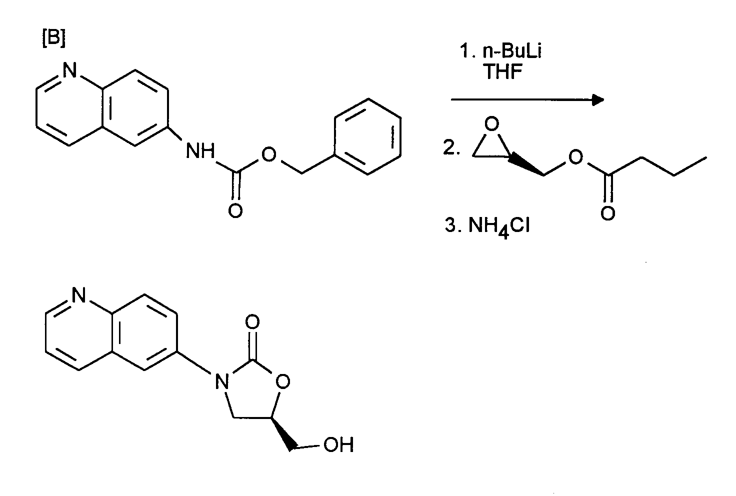

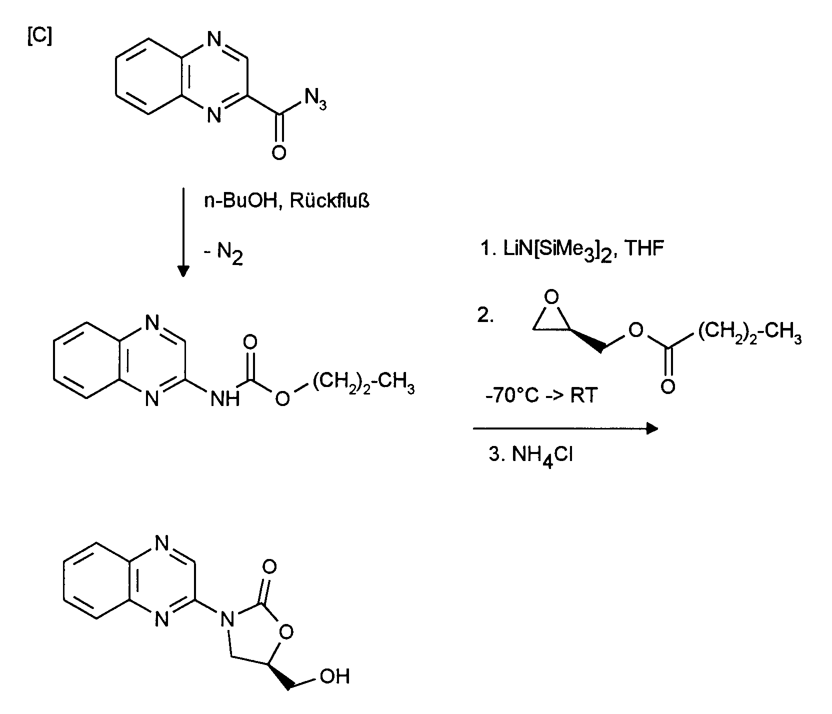

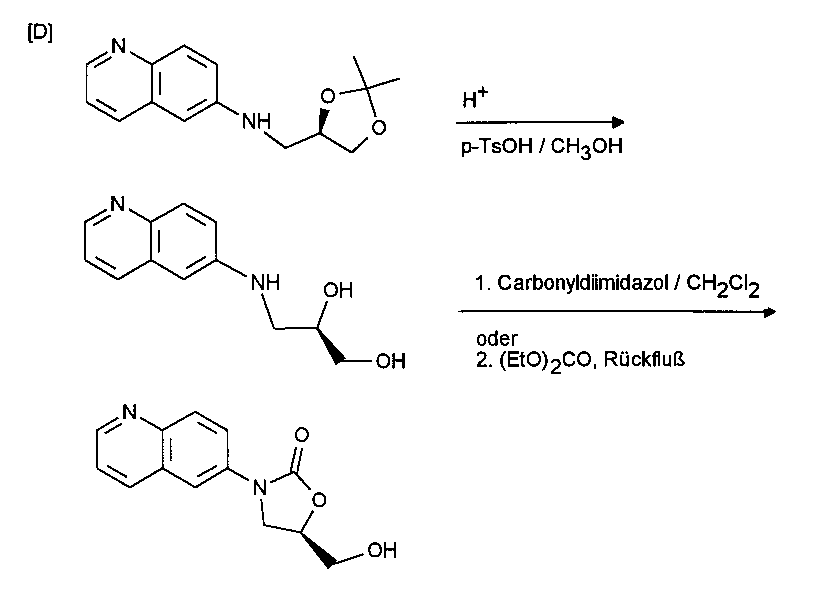

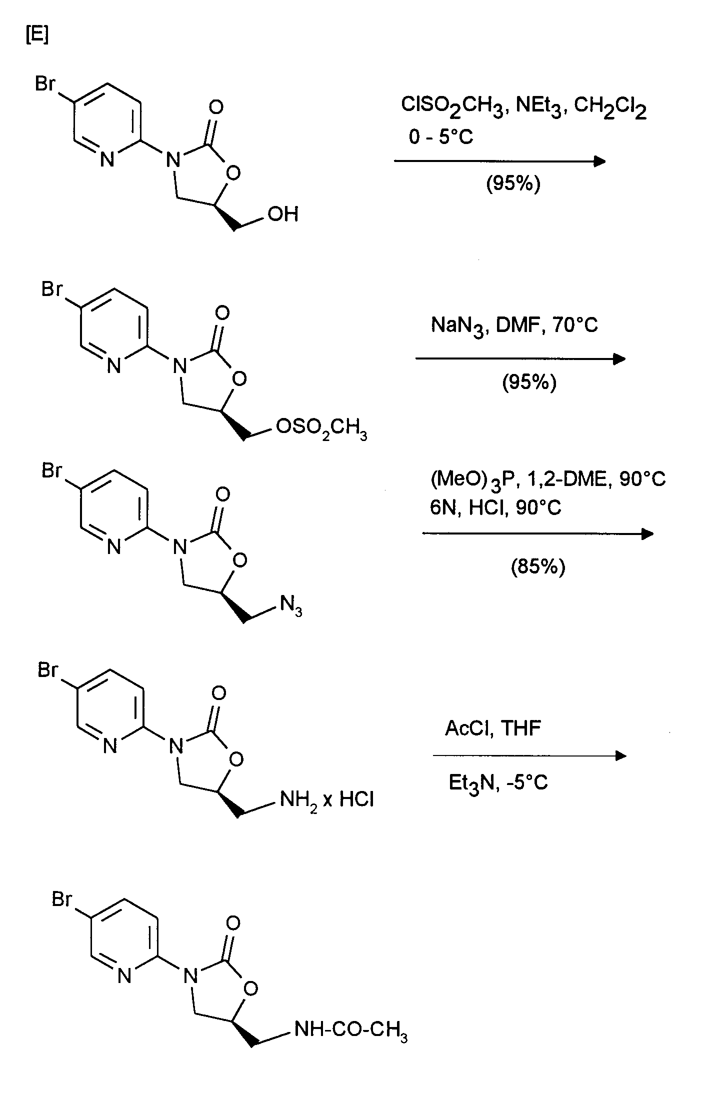

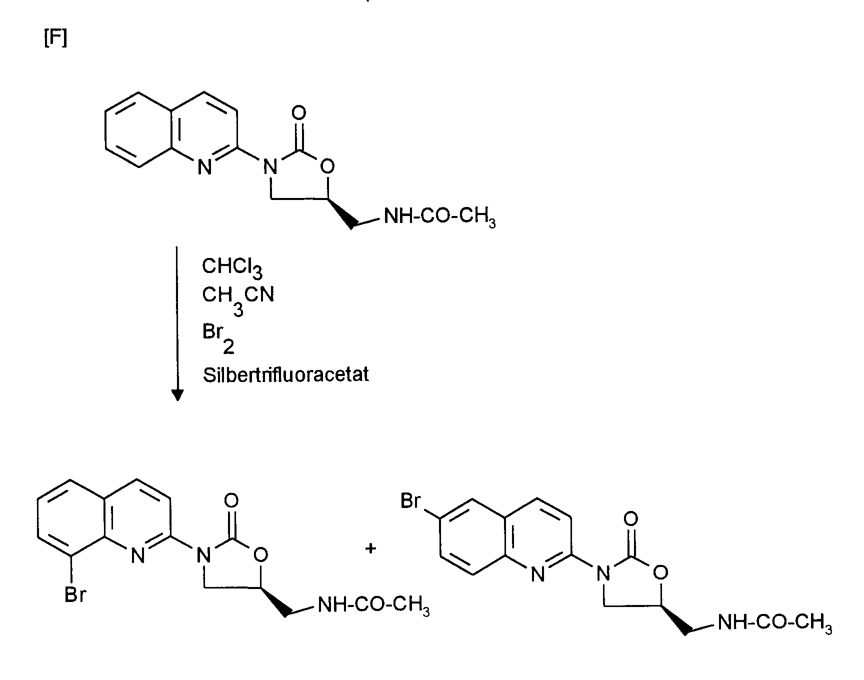

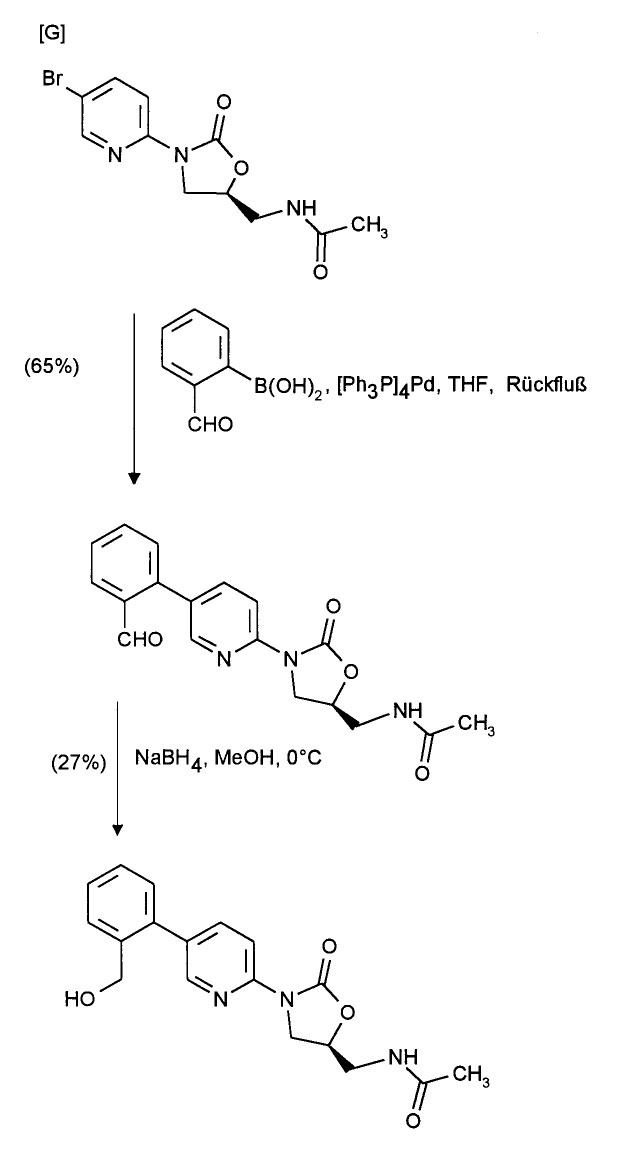

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formeln (II), (III), (IV) und (Va) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [A] erfolgt bevorzugt in Xylol oder Dichlorbenzol, gegebenenfalls in Gegenwart von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [B] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran und Lithium-bis-trimethylsilylamid oder n-Butyllithium, in einem Temperaturbereich von -100°C bis +20°C, vorzugsweise von -75°C bis -40°C.

Für das Verfahren [C] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [D] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (VI) eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester oder Chlorameisensäuretrichlormethylester.

Als Lösemittel eignen sich die oben aufgeführten Halogenkohlenwasserstoffe. Bevorzugt ist Methylenchlorid.

Die Cyclisierungen erfolgen im allgemeinen in einem Temperaturbereich von -20°C bis 100°C, vorzugsweise bei -20°C bis Raumtemperatur.

Die Acylierung [E] erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis 50°C, bevorzugt von -10°C bis Raumtemperatur.

Die Kupplungsreaktionen [G] mit den Boronsäure- und Zinnarylverbindungen erfolgt ebenfalls in einem der oben aufgeführten Ether oder Kohlenwasserstoffe, vorzugsweise Tetrahydrofuran oder Toluol und in Anwesenheit eines Palladiumkomplexes.

Als Palladiumkomplexe eignen sich beispielsweise Pd[P(C₆H₅)₃]₄, [(C₆H₅)₃P]₂PdCl₂ oder (C₆H₅CN)₂PdCl₂. Bevorzugt ist [(C₆H₅)₃P]₄Pd.

Die Umsetzung erfolgt in einem Temperaturbereich von Raumtemperatur bis 150°C, vorzugsweise bei der Siedetemperatur des jeweiligen Lösemittels.

Die Reduktionen erfolgen im allgemeinen mit Hydriden in inerten Lösemitteln oder mit Boranen, Diboranen oder ihren Komplexverbindungen.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Die Reduktion erfolgt im allgemeinen in einem Temperaturbereich von -50°C bis zum jeweiligen Siedepunkt des Lösemittels, bevorzugt von -20°C bis +90°C.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Oxidation zum N-oxid erfolgt im allgemeinen in einem der oben aufgeführten Lösemittel, vorzugsweise in Methylenchlorid mit Oxidationsmitteln wie beispielsweise Metachlorperbenzoesäure, Wasserstoffperoxid oder Peressigsäure, vorzugsweise mit Metachlorperbenzoesäure in einem Temperaturbereich von 0°C bis 80°C, bevorzugt von 0°C bis 40°C.

Die Abspaltung der Hydroxyschutzgruppen erfolgt im allgemeinen nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Abspaltung der Aminoschutzgruppe erfolgt im allgemeinen, ebenfalls nach üblichen Methoden, abspaltet und zwar vorzugsweise Boc mit Salzsäure in Dioxan, Fmoc mit Piperidin und Z mit HBr/HOAc oder durch Hydrogenolyse.

Die oben aufgeführten anderen Derivatisierungsreaktionen erfolgen im allgemeinen nach denen in Compendium of Organic Synthetic Methods, T.T. Harrison und S. Harrison, Wiley Interscience, publizierten Methoden.

Bevorzugt werden Redoxreaktionen, reduktive Aminierung, Umesterung und die Halogenisierung von Methylgruppen mit N-Bromsuccinimid (NBS) oder N-Chlorsuccinimid (NCS) aufgeführt, die im folgenden beispielhaft erläutert werden.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimehylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dichlormethan, Dimethylsulfoxid und Dimethylformamid.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung und die Sulfoamidierung erfolgen im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Tetrahydrofuran. Ebenso ist es möglich, Gemische der Lösemittel einzusetzen. Besonders bevorzugt sind Dichlormethan und Tetrahydrofuran.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexyfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder 4-Dimethylaminopyridin.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C und Normaldruck.

Die Verbindungen der allgemeinen Formeln (IV), (VIII) und (IX) sind bekannt oder können nach üblichen Methoden hergestellt werden.

Die Verbindungen der allgemeinen Formel (VII) sind größtenteils neu und können beispielsweise wie oben beschrieben hergestellt werden.

Die Verbindungen der allgemeinen Formel (II) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (III) sind teilweise bekannt oder neu und können dann beispielsweise hergestellt werden, indem man ausgehend von den entsprechenen Carbonsäuren entweder mit Chlorameisensäureisobutylester / Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei -10°C bis Raumtemperatur umsetzt.

Die Verbindungen der allgemeinen Formeln (V) und (Va) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die Verbindungen der allgemeinen Formel (VII) sind größtenteils neu und können wie oben beschrieben hergestellt werden.

Die Verbindungen der allgemeinen Formel (Ia) sind neu und können beispielsweise wie unter [A], [B], [D] oder [E] beschrieben hergestellt werden.

Die Verbindungen der allgemeinen Formel (Ib), (Ic), (Id), (Ie) und (If) sind neu und können wie oben beschrieben hergestellt werden.

Die Verbindungen der allgemeinen Formel (VI) sind größtenteils bekannt oder neu und können beispielsweise hergestellt werden, indem man ausgehend von den freien Aminen (Ia) entweder mit dem Acetonid von Glycerinaldehyd in Methanol und in Anwesenheit von Natriumacetat / Natriumcyanborhydrid oder von Natriumboranat und Methanol in einem Temperaturbereich von -20°C bis +40°C, bevorzugt von -10°C bis 20°C und Normaldruck umsetzt.

Die Einführung des Halogenatoms Y (Verbindungen der allgemeinen Formel (If)) erfolgt im Fall von Brom und Iod entweder mit elementarem Brom oder Iod, oder im Fall von Brom oder Jod in Anwesenheit eines Silber-Salzes, in einem der oben aufgeführten Lösemittel, vorzugsweise Methylenchlorid, Acetonitril oder Chloroform, in einem Temperaturbereich von -30°C bis +60°C, bevorzugt von 0°C bis +30°C und Normaldruck.

Als Silber-Salze eignen sich beispielsweise Silbertetrafluoroborat, Silbertrifluormethansulfonat oder Silbertrifluoracetat.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

Die MHK-Werte wurden mit Hilfe der Mikrodilutionsmethode in BH-Medium bestimmt. Jede Prüfsubstanz wurde im Nährmedium gelöst. In der Mikrotiterplatte wurde durch serielle Verdünnung eine Konzentrationsreihe der Prüfsubstanzen angelegt. Zur Inokulation wurden Übernachtkulturen der Erreger verwandt, die zuvor im Nährmedium 1:250 verdünnt wurden. Zu 100 µl der verdünnten, wirkstoffhaltigen Nährlösungen wurden je 100 µl Inokulationslösung gegeben.

Die Mikrotiterplatten wurden bei 37°C bebrütet und nach ca. 20 Stunden abgelesen. Der MHK-Wert (µg/ml) gibt die niedrigste Wirkstoffkonzentration an, bei der kein Wachstum zu erkennen war.

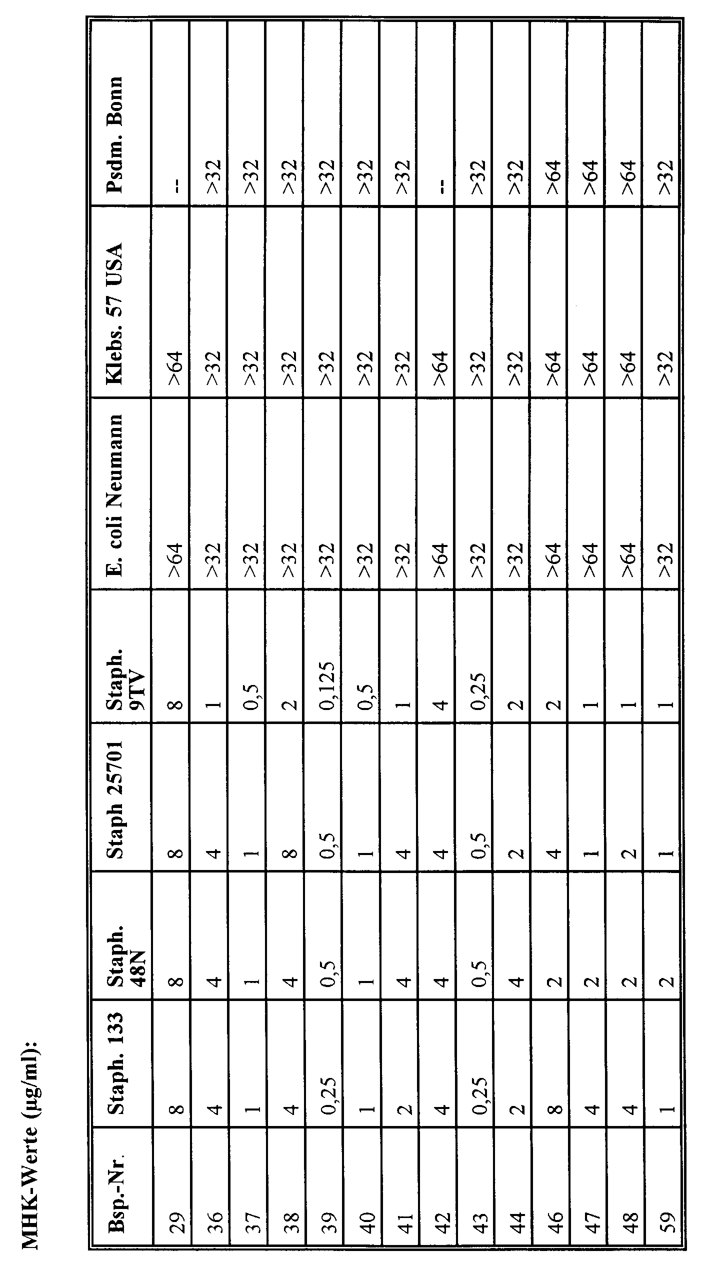

Die erfindungsgemäßen Verbindungen der allgemeinen Formeln (I), (Ia), (Ib), (Ic), (Id), (Id), (Ie) und (If) weisen bei geringer Toxizität ein breites antibakterielles Spektrum, speziell gegen gram-positive Bakterien sowie Mycobacterien, Corynebacterien, Haemophilus Influenzae und Anaerobae Keime auf. Diese Eigenschaften ermöglichen ihre Verwendung als chemotherapeutische Wirkstoffe in der Human- und Tiermedizin.

Die erfindungsgemäßen Verbindungen sind gegen ein breites Spektrum von Mikroorganismen wirksam. Mit ihrer Hilfe können gram-positive Bakterien und bakterienähnliche Mikroorganismen, wie Mycoplasmen, bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit anderen Antibiotika kombiniert werden.

### Anhang zum experimentellen Teil

### Liste der verwendeten Laufmittelgemische zur Chromatographie:

- I: Dichormethan : Methanol
- II: Toluol : Ethylacetat
- III: Acetonitril : Wasser
- IV: Ethylacetat
- V: Petrolether : Ethylacetat

### Abkürzungen:

- Z: Benzyloxycarbonyl
- Boc: tert.Butyloxycarbonyl
- DMF: Dimethylformamid
- Ph: Phenyl
- Me: Methyl
- THF: Tetrahydrofuran
- CDI: Carbonylimidazol
- DCE: Dichlorethan

### Ausgangsverbindungen

### 5-Brom-2-isocyanato-pyridin Hydrochlorid

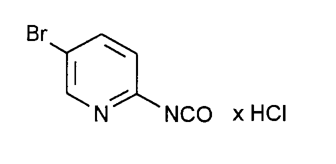

Zu einer gerührten Lösung von 100 g (0,58 mol) 2-Amino-5-brompyridin in 400 ml 1,2-Dichlorethan tropft man in der Siedehitze 78,0 ml (0,64 mol) Chlorameisensäuretrichlorethylester. Nach der Zugabe wird 2h im Rückfluß gekocht, dann darf sich das Gemisch auf Raumtemperatur abkühlen. Der entstandene Niederschlag wird durch Filtration abgetrennt, mit 100 ml 1,2-Dichlorethan gut gewaschen und im Hochvakuum über Natriumhydroxid getrocknet. Man erhält 98,3 g (72%) der Titelverbindung als gelben Feststoff.
Schmp.: 248-254°C (Zers.)
R_{f} = 0,23 (Ethylacetat)
MS (EI) m/z = 198 (M)⁺
Wie für Beispiel I beschrieben, erhielt man aus den entsprechenden heteroaromatischen Aminen durch Umsetzung mit Chlorameisensäuretrichlormethylester die Hydrochloride folgender Isocyanate:

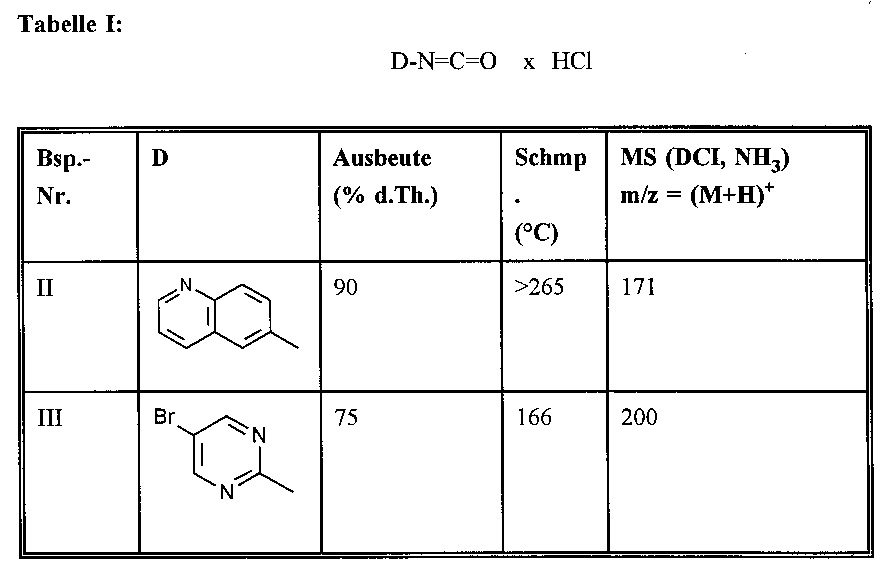

### Beispiel IV

### Chinolin-2-carbonsäureazid

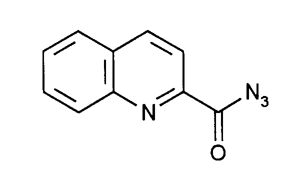

Eine auf -10°C gekühlte, gerührte Suspension von 30,0 g (0.17 mol) Chinolin-2-carbonsäure in 385 ml wasserfreiem Tetrahydrofuran wird mit 47 ml (0,34 mol) Triethylamin versetzt und 10 min. bei -10°C gerührt, wobei eine klare Lösung entsteht. Danach tropft man 73,0 ml (0,34 mol) Diphenylphosphorylazid zu und läßt die Reaktionsmischung 20 h bei 0°C im Kühlschrank stehen. Danach wird die Mischung in 350 ml eiskalte verdünnte NaHCO₃-Lösung eingerührt. Der entstandene Niederschlag wird durch Filtration abgetrennt, mit Wasser gewaschen und an der Luft getrocknet. Man erhält 88,9 g (86%) der Titelverbindung als hellen Feststoff.
R_{f} = 0,35 (Toluol : Ethylacetat 9:1)

### Beispiel V

### Chinoxalin-2-carbonsäureazid

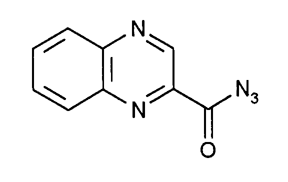

Wie für Beispiel IV beschrieben erhielt man aus 2,60 g (15,0 mmol) Chinoxalin-2-carbonsäure 2,87 g (96%) des entsprechenden Säureazids, als braunes Pulver. R_{f} = 0,65 (Dichlormethan : Ethylacetat 9:1)

### Beispiel VI

### 6-Benzyloxycarbonylamino-chinolin

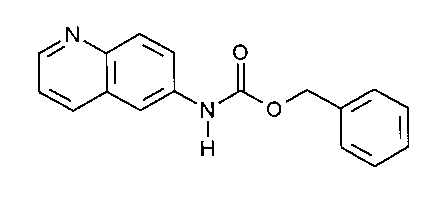

Zu einer auf 0°C gekühlten, gerührten Lösung von 10,0 g (69,36 mmol) 6-Aminochinolin in 160 ml Wasser und 80 ml THF werden innerhalb von 30 min 13,0 ml (76,28 mmol) Chlorameisensäurebenzylester getropft, wobei pH = 10 durch gleichzeitige Zugabe einer 4 N NaOH Lösung gehalten wird. Man rührt noch 2 h bei 0°C nach, dampft das THF im Vakuum ab und extrahiert den Rückstand mit 3 x 50 ml Ethylacetat. Die vereinigten organischen Extrakte werden über MgSO₄ getrocknet, das Lösemittel wird im Vakuum abgedampft und der Rückstand durch Chromatographie an 450 g Kieselgel (Toluol : Ethylacetat 1:4) gereinigt. Man erhält 11,60 g (60%) der Titelverbindung als Kristalle.
Schmp.: 122°C
R_{f} = 0,43 (Toluol : Ethylacetat 1:4)
MS (EI) m/z = 278 (M⁺)
¹H-NMR (300 MHz, D₆-DMSO): δ = 5,22 (s, 2H, CH₂O); 7,3 - 7,5 (m, 6H, Ph, Chinolin-H); 7,78 (dd, J = 1,5, 9 Hz, 1H, Chinolin-H); 7,96 (d, J = 9 Hz, 1H, Chinolin-H); 8,17 (d, J = 1,5 Hz, 1H, Chinolin H-5); 8,25 (d, J = 9 Hz, 1H, Chinolin-H); 8,77 (m, 1H, Chinolin H-2).

Wie für Beispiel VI beschrieben erhielt man aus den entsprechenden Heteroaromaten durch Umsetzung mit Chlorameisensäurebenzylester die in der Tabelle II aufgeführten Verbindungen:

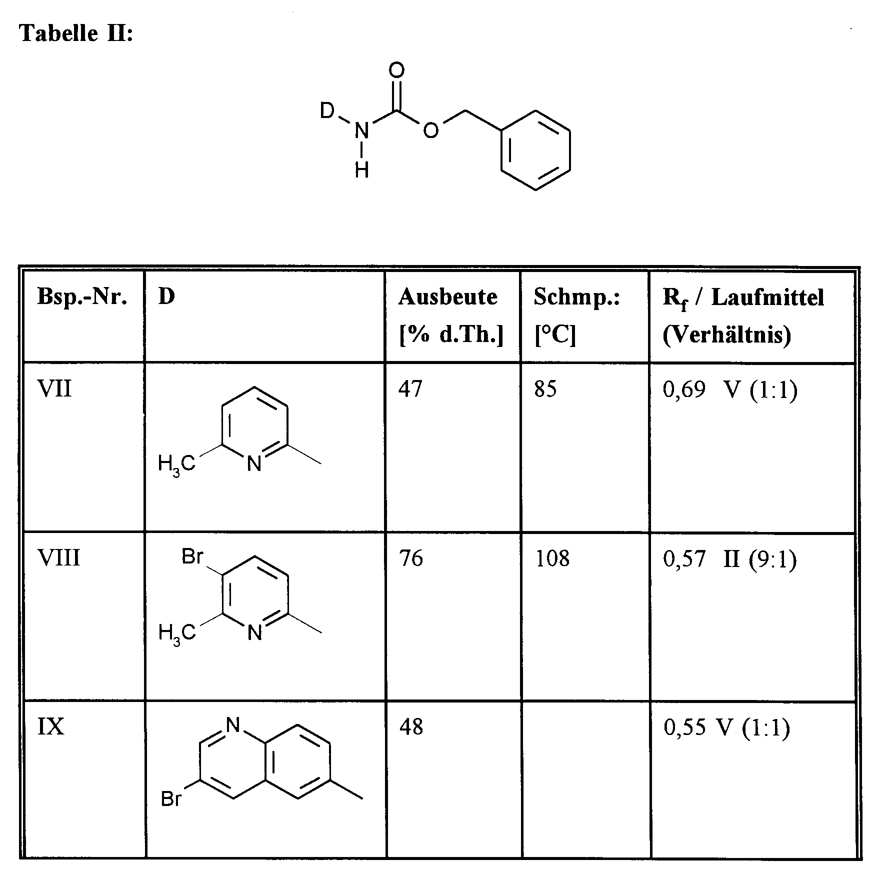

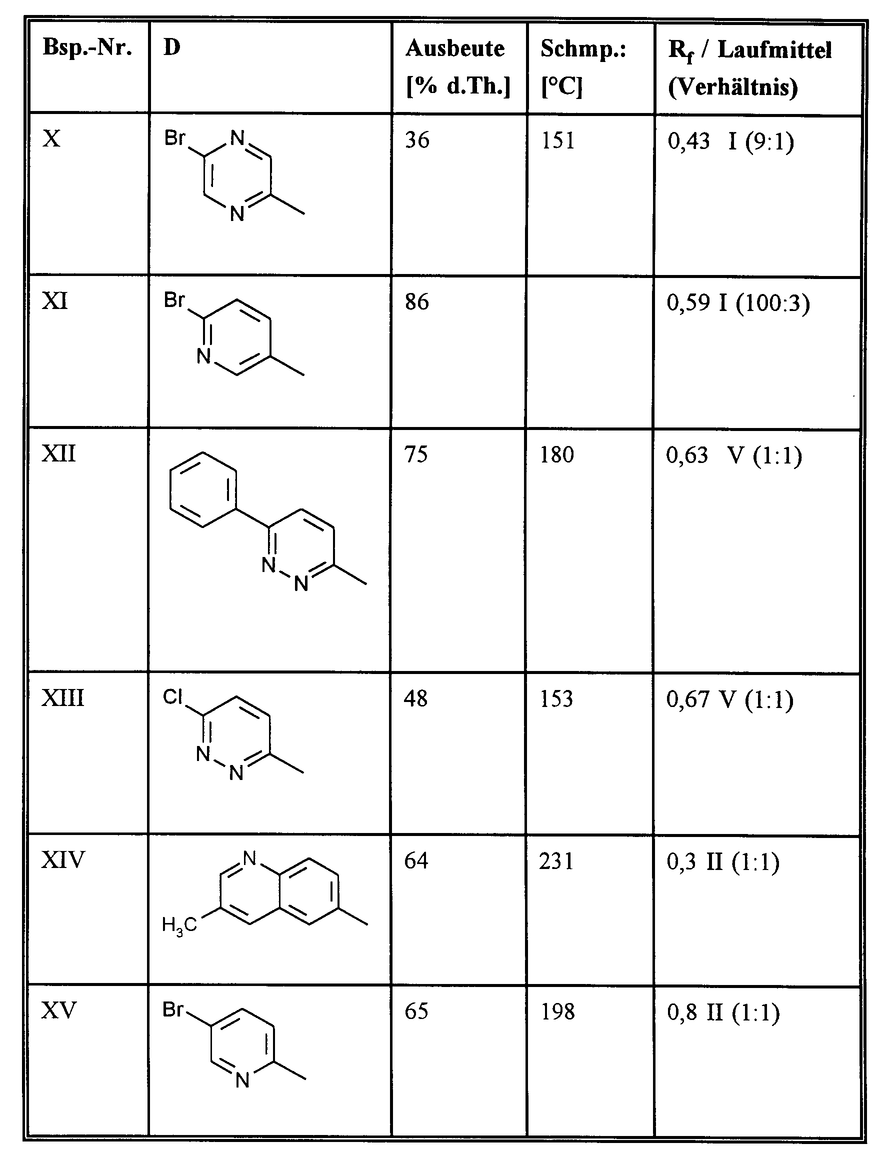

### Beispiel XVI

### N-Boc-2-amino-6-brom-1,8-naphthyridin

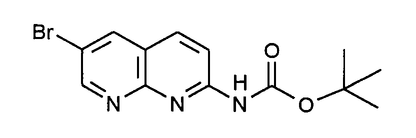

Unter Argon werden 200 mg (0,893 mmol) 2-Amino-6-brom-1,8-naphthyridin (C. Reichardt; W. Scheibelein, Tetrahedron Lett. 1977, 2087) in 3 ml absolutem DMF gelöst und zu einer auf 0°C gekühlten Suspension von 28,2 mg 80 %igem NaH (0,937 mMol) in 2 ml absolutem DMF addiert, ohne daß die Temperatur +5°C übersteigt. Nach 10 Minuten rühren addiert man 0,21 g (0,937 mmol) (Boc)₂O und läßt über Nacht auf Raumtemperatur kommen. Der Ansatz wird mit Wasser versetzt und 3 mal mit je 30 ml Essigester extrahiert. Die organische Phase wird 1 mal mit je 30 ml Wasser gewaschen, über MgSO₄ getrocknet und eingedampft. Nach Säulenchromatographie an Kieselgel mit CH₂Cl₂:CH₃OH=100:2 erhält man 114 mg (39 % der Theorie) der Titelverbindung als gelben Feststoff.
R_{f}-Wert (CH₂Cl₂:MeOH=100:2) : 0,42
Schmelzpunkt: >230°C

### Beispiel XVII

### N-Allyl-N-Boc-2-amino-6-brom-1,8-naphthyridin

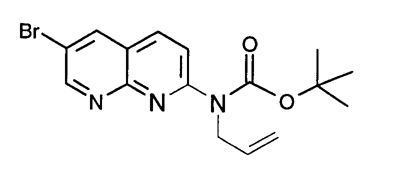

Unter Argon werden 9,7 mg NaH (80 % in Öl; 0,324 mmol) in 2 ml absolutem THF suspendiert und auf 0°C gekühlt. Man addiert langsam eine Lösung von 100 mg (0,308 mmol) der Verbindung aus Beispiel XVI in 3 ml absolutem THF, rührt 10 Minuten bei 0°C und weitere 15 Minuten bei Raumtemperatur nach.

Man addiert 10 mg Tetra-butylammoniumiodid und 32 µl (0,37 mmol) Allylbromid und läßt bei Raumtemperatur über Nacht rühren.

Man versetzt mit Wasser, extrahiert 3 mal mit 25 ml Essigester, trocknet MgSO₄ und engt ein. Das Rohprodukt wird durch Chromatographie an Kieselgel mit CH₂Cl₂:MeOH=100:1,5 gereinigt. Man erhält 84 mg (75 % der Theorie) der Titelverbindung.
R_{f}-Wert (CH₂Cl₂:MeOH=100:2) : 0,22
Schmelzpunkt: 114°C

### Beispiel XVIII

### 3-(6-Brom-1,8-naphthyridin-2-yl)-5-iodomethyl-2-(tert.-butyloxy)-oxazoliniumiodid

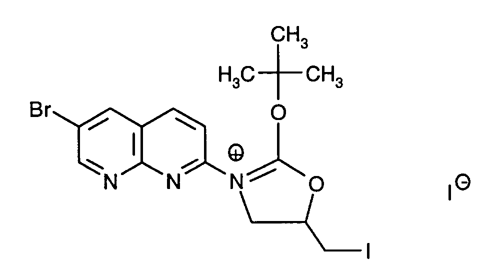

In einem abgedunkelten Kolben werden 74 mg (0,203 mmol) der Verbindung aus Beispiel XVII in 5 ml Chloroform unter Argon gelöst. Man gibt 129 mg (0,508 mmol) Iod zu und läßt über Nacht rühren.

Man versetzt mit 5 ml 20 %iger Natriumthiosulfat-Lösung, trennt die organische Phase ab und engt ein. Der Rückstand wird mit Wasser verrührt, abgesaugt und mit Wasser gewaschen.

Man trocknet am Hochvakuum und erhält 99 mg Produkt.

Schmelzpunkt: 210°C unter Zersetzung
¹³C-NMR(DMSO, 75 MHz): 156,2 (d); 153,2 (s); 148,3 (s); 148,2 (d); 142,5 (s); 141,1 (d); 120,5 (s); 118,5 (s); 113,6 (d); 86,5 (s); 59,0 (d); 52,6 (t); 27,4 (q); 7,9 (t);
MS (FAB): 492 (62), 490 (50), 436 (100).

### Herstellungsbeispiele

### Beispiel 1

### (5R)-3-(5-Brom-pyridin-2-yl)-5-butyryloxy-methyl-oxazolidin-2-on

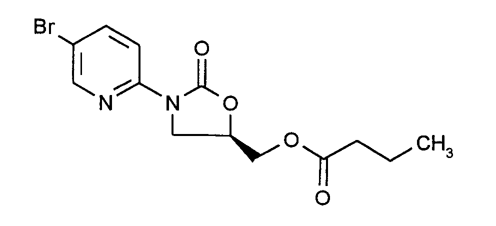

Eine Suspension von 2,17 g (25 mmol) Lithiumbromid und 5,46 g (25 mmol) Tributylphophinoxid in 73 ml Xylol wird 1 h am Wasserabscheider gekocht. Dazu wird in der Siedehitze ein Gemisch von 58,5 ml (0,42 mol) Triethylamin und 66,6 g (0,42mol) (R)-Glycidylbutyrat getropft. Gleichzeitig werden innerhalb von 20 min 98,2 g (0,42 mol) der Verbindung aus Beispiel I portionsweise zugegeben. Nach beendeter Zugabe wird noch 1 h unter Rückfluß nachgerührt. Man laßt auf Raumtemperatur abkühlen und dampft das Lösemittel im Vakuum ab. Nach Chromatographie des Rückstands an 1 kg Kieselgel (Toluol : Ethylacetat 95:5) erhält man 37,9 g (26%) der Titelverbindung als Öl.
R_{f} = 0,43 (Toluol : Ethylacetat 4:1)
MS (FAB) m/z = 343 (M+H)⁺
¹H-NMR (250 MHz, D₆-DMSO): δ = 0,81 (t, J = 7 Hz, 3H, CH₃CH₂); 1,5 (m, 2H, CH₃CH₂CH₂CO); 2,29 (t, J = 7 Hz, 2H, CH₃CH₂CH₂CO); 3,91 (dd, J = 7 Hz, 10 Hz, 1H, H-4 trans); 4,25 (dd, J = 9 Hz, 10 Hz, 1H, H-4 cis); 4,36 (m, 2H, CH₂O); 4,97 (m, 1H, H-5); 8,08 (d, J = 1 Hz, 2H, Pyridyl H-3,4); 8,50 (d, J = 1 Hz, pyridyl H-6).

### Beispiel 2

### (5R)-3-(Chinolin-2-yl)-5-butyryloxymethyl-oxazolidin-2-on

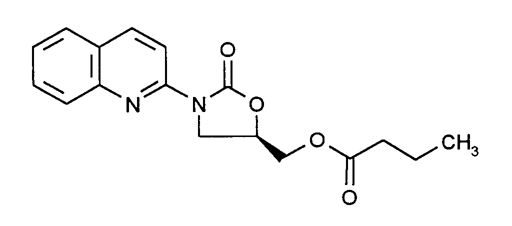

Eine Suspension von 51 mg (0,06 mmol) Lithiumbromid und 126 mg (0,06 mmol) Tributylphosphinoxid in 10 ml 1,3-Dichlorbenzol werden 1 h am Wasserabscheider gekocht. Dazu wird in der Siedehitze (Bad 220°C) innerhalb von 10 min ein Gemisch von 1,42 ml (10,0 mmol) (R)-Glycidylbutyrat und 19,82 g (10,0 mmol) des Säureazids aus Herstellungsbeispiel IV in 17 ml 1,3-Dichlorbenzol getropft (heftige Gasentwicklung !). Nach beendeter Zugabe wird noch 30 min im Rückfluß nachgerührt, dann läßt man die Mischung auf Rautemperatur abkühlen. Das Lösemittel wird im Hochvakuum abgedampft und der Rückstand durch Chromatographie an 175 g Kieselgel (Toluol : Ethylacetat 9:1) gereinigt. Man erhält 2,51 g (80%) der Titelverbindung als helles Öl.
R_{f} = 0,20 (Dichlormethan)
R_{f} = 0,34 (Toluol : Ethylacetat 9:1)
MS (FAB) m/z = 315 (M+H)⁺
¹H-NMR (250 MHz, CD₃OD) δ = 0,82 (t, J = 7 Hz, 3H, CH₃CH₂); 1,57 (m, 2H, CH₃CH₂CH₂CO); 2,29 (t, J = 7 Hz, 2H, CH₃CH₂CH₂CO); 4,25 (dd, J = 6,5, 10 Hz, 1H, H-4 trans); 4,4 - 4,5 (m, 3H, H-4, CH₂O); 5,00 (m, 1H, H-5); 7,48 (m, 1H, H arom); 7,68 (m, 1H, H arom); 7,83 (d, J = 7 Hz, 2H, Chinolin H-6,7); 8,25 (d, J = 8 Hz, 1H, Chinolin H-3); 8,36 (d, J = 8 Hz, 1H, Chinolin H-4).

### Beispiel 3

### (5R)-3-(Chinolin-6-yl)-5-hydroxymethyl-oxazolidin-2-on

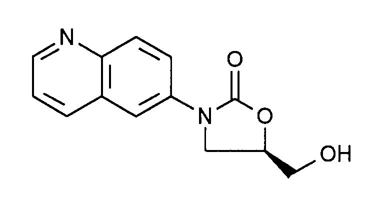

Eine auf -78°C gekühlte, gerührte Lösung von 3,28 g (11,78 mmol) 6-Benzyloxycarbonylamino-chinolin und 1 mg 1,10-Phenanthrolinhydrat in 30 ml wasserfreiem THF wird bis zum Farbumschlag langsam mit 4,70 ml (11,78 mmol) einer 2,5 M Lösung von n-Butyllithium in n-Hexan vesetzt. Danach tropft man 1,67 ml (11,78 mmol) (R)-Glycidylbutyrat zu und erlaubt der Reaktionsmischung sich innerhalb von 16 h auf Raumtemperatur zu erwärmen. Danach werden innerhalb von 15 min 30 ml gesättigte wässrige NH₄Cl-Lösung zugetropft. Die Wasserphase wird mit 3 x 60 ml Ethylacetat extrahiert, die organischen Phasen werden vereinigt, mit 2 x 50 ml NaCl-Lösung gewaschen und über MgSO₄ getrocknet. Nach Abdampfen des Lösemittels im Vakuum, Verreiben des Rückstands mit Ether und Umkristallisation aus 25 ml Ethanol erhält man 1,30 g (45%) der Titelverbindung als farblose Kristalle.
Schmp.: 165°C
R_{f} = 0,08 (Toluol : Ethylacetat 1:4)
MS (DCI, NH₃) m/z = 245 (M+H)⁺
¹H-NMR (250 MHz, D₆-DMSO) δ = 3,6 - 3,8 (m, 2H, CH₂O); 4,00 (dd, J = 7, 10 Hz, 1H, H-4 trans); 4,25 (dd, J = 10, 10 Hz, 1H, H-4 cis); 4,78 (m, 1H, H-5); 5,25 (t, J = 6 Hz, 1H, OH); 7,52 (dd, J = 6, 9 Hz, 1H, Chinolin H-3); 7,92 (d, J = 1,5 Hz, 1H, Chinolin H-5); 8,02 (d, J = 10 Hz, 1H, Chinolin H-8); 8,3 (m, 2H, Chinolin H-4,7); 8,82 (m, 1H, Chinolin H-2).

### Beispiel 4

### (5R)-3-(5-Brom-pyridin-2-yl)-5-hydroxymethyl-oxazolidin-2-on

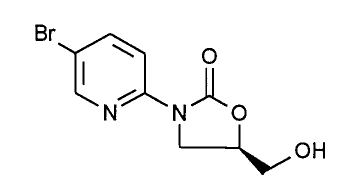

Eine Lösung von 19,6 g (57,3 mmol) der Verbindung aus Beispiel 1 in 125 ml wasserfreiem Methanol wird mit 185 mg (0,57 mmol) Caesiumcarbonat versetzt und 5 h bei Raumtemperatur gerührt. Das Lösemittel wird im Vakuum abgedampft und der Rückstand wird mit 30 ml Ether verrührt. Der Niederschlag wird durch Filtration abgetrennt, mit 25 ml Wasser und 5 ml Ether gewaschen und im Hochvakuum getrocknet. Man erhält 10,73 g (69%) der Titelverbindung als helle Kristalle.
Schmp.: 123-124°C
R_{f}-Wert: 0,09 (Toluol : Ethylacetat 4:1)
MS (DCI, NH₃) m/z = 273 (M+H)⁺
¹H-NMR (200 MHz, CD₃OD) δ = 3,68 (d, J = 5,9 Hz, 1 H, CH₂O); 3,87 (dd, J = 4, 9 Hz, 1H, CH₂O); 4,06 (dd, J = 7, 10 Hz, 1H, H-4 trans); 4,26 (dd, J = 9, 10 Hz, 1H, H-4 cis); 4,75 (m, 1H, H-5); 7,92 (dd, J = 1,5 Hz, 10 Hz, 1H, Pyridyl H-3); 8,12 (d, J = 10 Hz, 1H, Pyridyl H-4); 8,40 (d, J = 1,5 Hz, 1H, Pyridyl H-6).

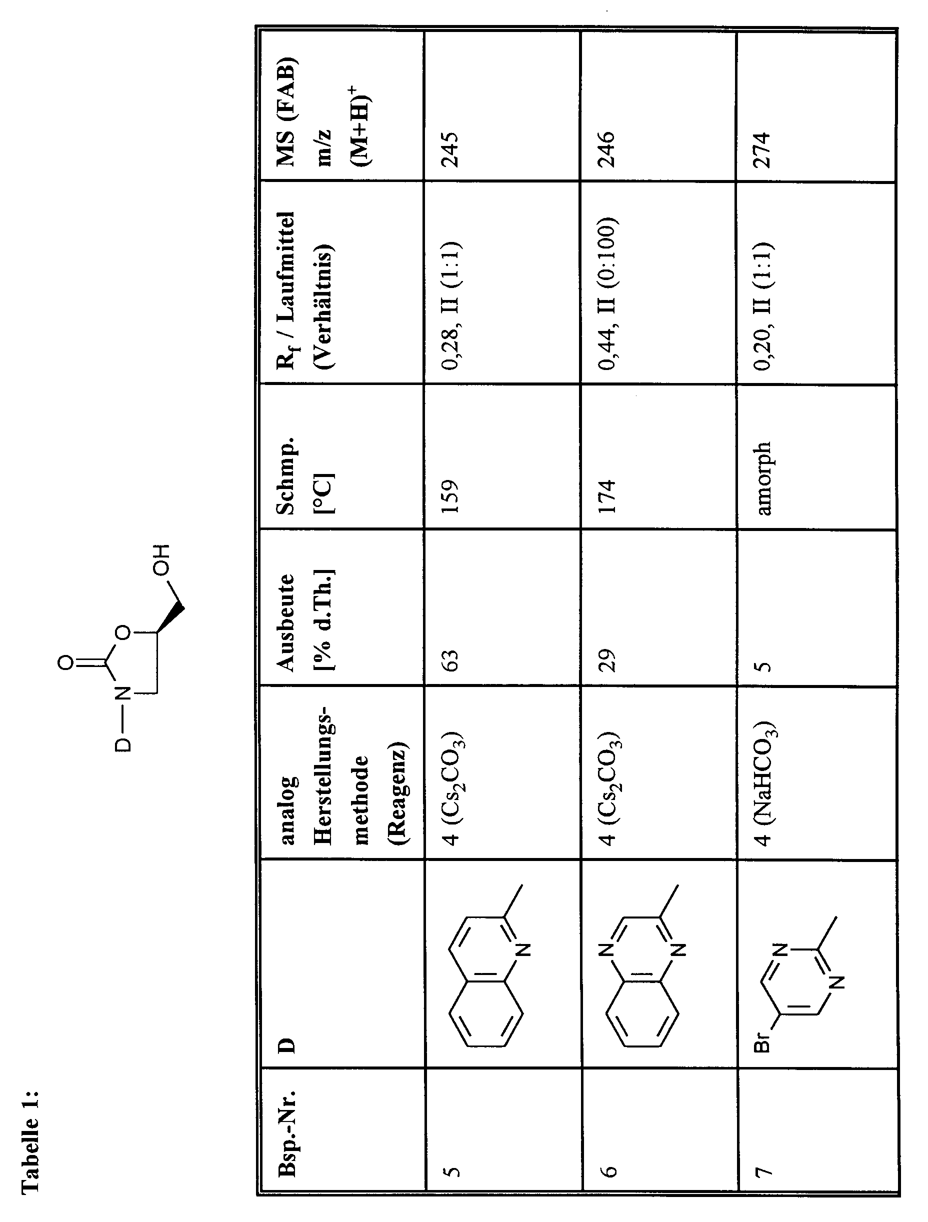

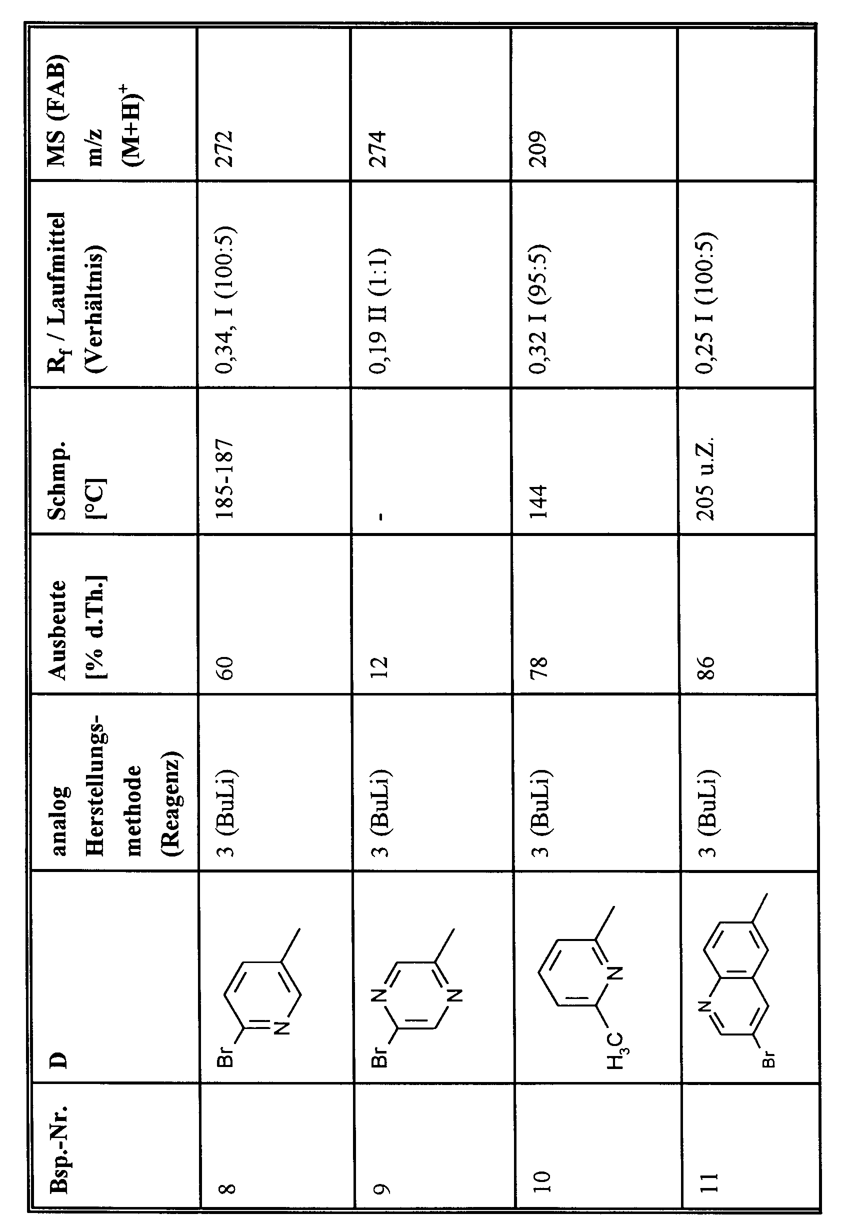

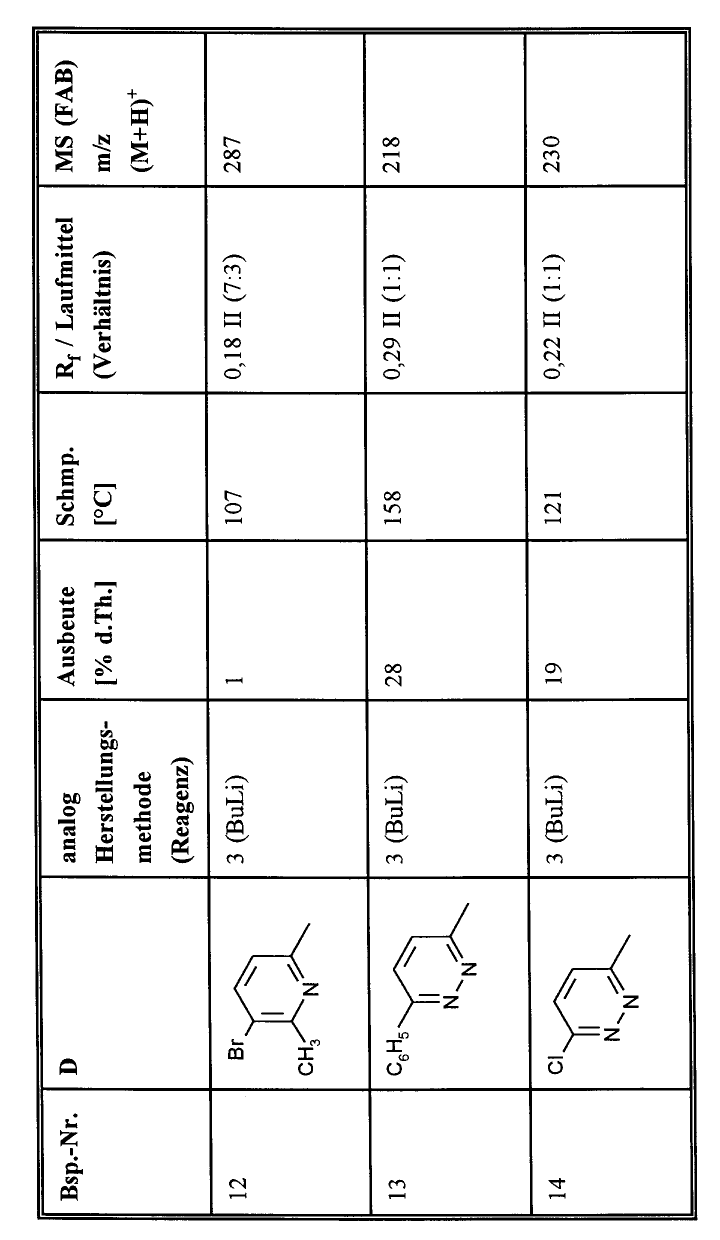

### Beispiel 15

### (5R)-3-(5-Brom-pyridin-2-yl)-5-methansulfonyloxy-methyl-oxazolidin-2-on

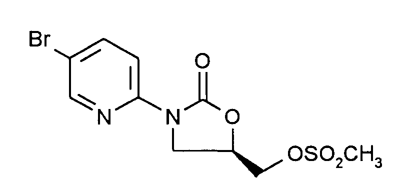

Eine auf 0°C gekühlte, gerührte Lösung von 10,5 g (38,44 mmol) der Verbindung aus Beispiel 4 und 6,40 ml (46,14 mmol) Triethylamin in 36 ml wasserfreiem Dichlormethan wird langsam mit 3,27 ml (42,28 mmol) Methansulfonsäurechlorid versetzt. Man rührt 10 min. bei 0-5°C nach und rührt das Gemisch in 50 ml Eiswasser ein. Die organische Phase wird abgetrennt, mit 20 ml gesättigter NaHCO₃-Lösung und 20 ml Eiswasser gewaschen und über MgSO₄ getrocknet. Das Lösemittel wird im Vakuum eingedampft und der Rückstand mit 50 ml Ether verrührt, abgesaugt und im Hochvakuum getrocknet. Man erhält 12,8 g (95%) der Titelverbindung als farblose Kristalle.
Schmp.: 138-138,5°C
R_{f} = 0,65 (Dichlormethan : Methanol 95:5)
MS (DCI, NH₃) m/z = 351 (M+H)⁺
¹H-NMR (250 MHz, D₆-DMSO) δ = 3,25 (s, 3H, OSO₂CH₃); 3,91 (dd, J = 7, 10 Hz, 1H, H-4 trans); 4,27 (dd, J = 10, 10 Hz, 1H, H-4 cis); 4,52 (m, 2H, CH₂O); 5,02 (m, 1H, H-5); 8,09 (s, 2H, Pyridyl H-3,4); 8,52 (s, 1H, Pyridyl H-6).

Wie für Beispiel 15 beschrieben erhält man aus den entsprechenden Alkoholen die folgenden Methansulfonate (Tabelle 2):

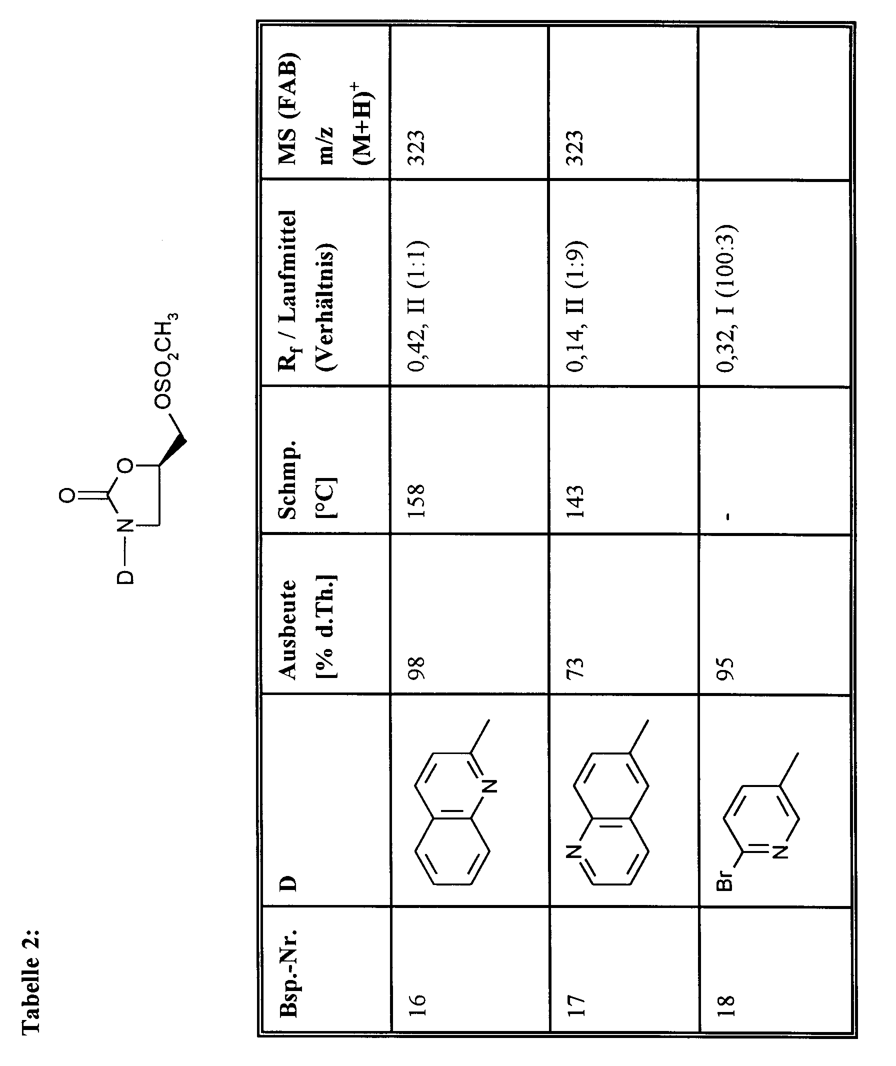

### Beispiel 19

### (5R)-3-(5-Brom-pyridin-2-yl)-5-azidomethyl-oxazolidin-2-on

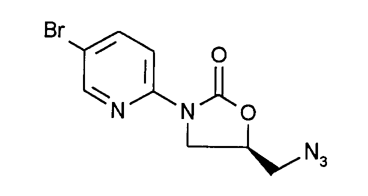

Eine gerührte Lösung von 12,5 g (35,6 mmol) der Verbindung aus Beispiel 15 in 48 ml wasserfreiem DMF wird mit 3,01 g (46,28 mmol) Natriumazid versetzt und 3 h bei 70°C gerührt. Man läßt auf Raumtemperatur abkühlen und rührt in 100 ml Eiswasser ein. Der entstandene Niederschlag wird durch Filtration abgetrennt, mit 50 ml Wasser und 20 ml Petrolether gewaschen und an der Luft getrocknet. Man erhält 10,1 g (95%) der Titelverbindung als helle Kristalle.
Schmp.: 64-67°C
R_{f} = 0,63 (Toluol : Ethylacetat 2:3)
MS (DCI, NH₃) m/z = 298 (M+H)⁺
¹H-NMR (250 MHz, D₆-DMSO) δ = 3,73 (m, 2H, CH₂N₃); 3,87 (dd, J = 6, 8 Hz, 1H, H-4 trans); 4,22 (dd, J = 8, 8 Hz, 1H, H-4 cis); 4,92 (m, 1H, H-5); 8,08 (s, 2H, Pyridyl H-3,4); 8,51 (s, 1H, Pyridyl H-6).

Wie für Beispiel 19 beschrieben erhält man aus den entsprechenden Methansulfonaten die folgenden Azide (Tabelle 3):

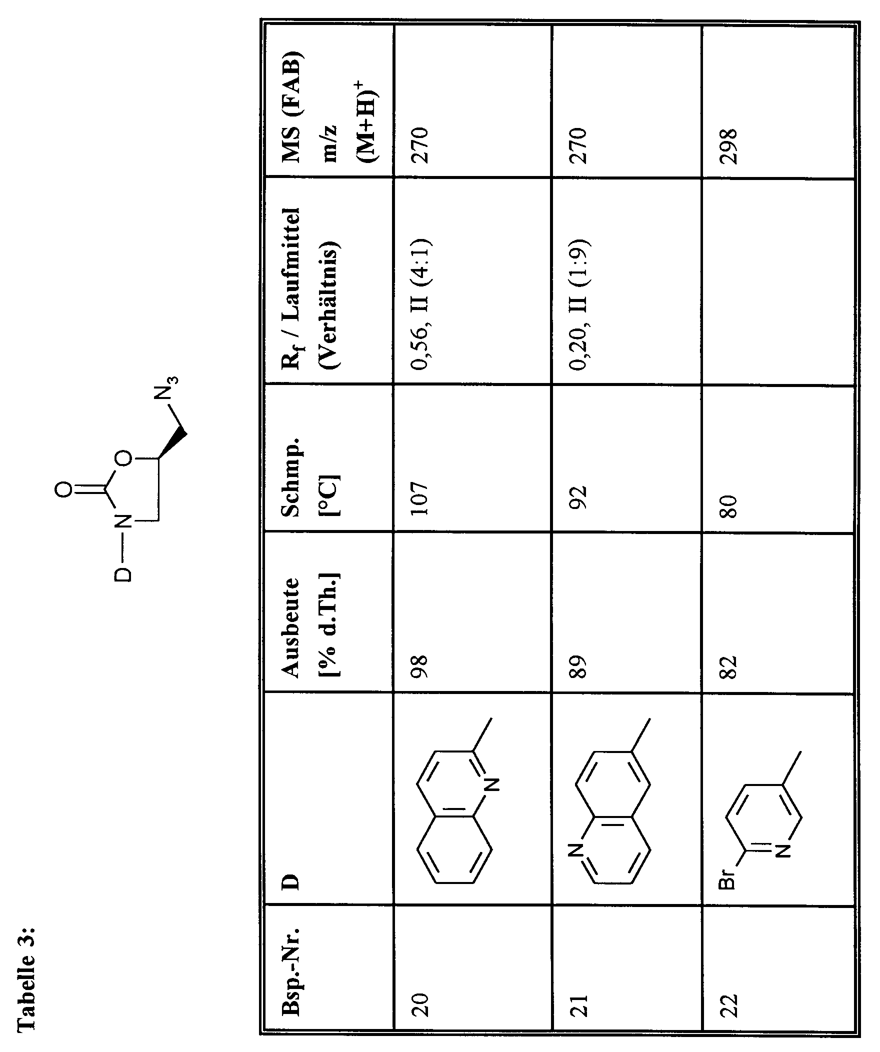

### Beispiel 23

### (5S)-3-(5-Brom-pyridin-2-yl)-5-aminomethyl-oxazolidin-2-on Hydrochlorid

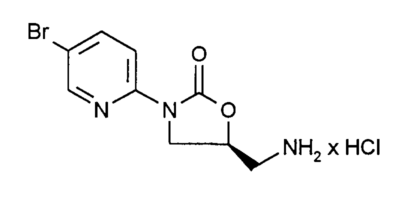

Eine gerührte Lösung von 10,1 g (33,9 mmol) der Verbindung aus Beispiel 19 in 16,5 ml 1,2-Dimethoxyethan wird auf 50°C erwärmt. Man tropft langsam 4,68 ml (4,70 mmol) Trimethylphosphit zu (Gasentwicklung) und rührt nach beendeter Zugabe nach 2 h bei 90°C nach. Nun tropft man 6,6 ml 6 N HCl zu und rührt nochmals 2 h bei 90°C nach. Man läßt auf Raumtemperatur abkühlen, trennt den Niederschlag durch Filtration ab, wäscht mit 2 x 10 ml 1,2-Dimethoxyethan und trocknet im Hochvakuum über NaOH. Man erhält 8,9 g (85%) der Titelverbindung als farblose Kristalle.
Schmp.: 260-262°C
R_{f} = 0,53 (Acetonitril : Wasser 4:1)
MS (EI) m/z = 271 (M⁺)
¹H-NMR (250 MHz, D₆-DMSO) δ = 3,28 (m, 2H, CH₂NH₂); 3,93 (dd, J 7, 9 Hz, 1H, H-4 trans); 4,28 (dd, J = 9, 9 Hz, 1H, H-4 cis); 5,00 (m, 1H, H-5); 8,05 (s, 2H, Pyridyl H-3,4); 8,5 (m, 3H, NH₂, Pyridyl H-6).

Wie für Beispiel 23 beschrieben erhält man durch Umsetzung der entsprechenden Azide die folgenden Produkte (Tabelle 4):

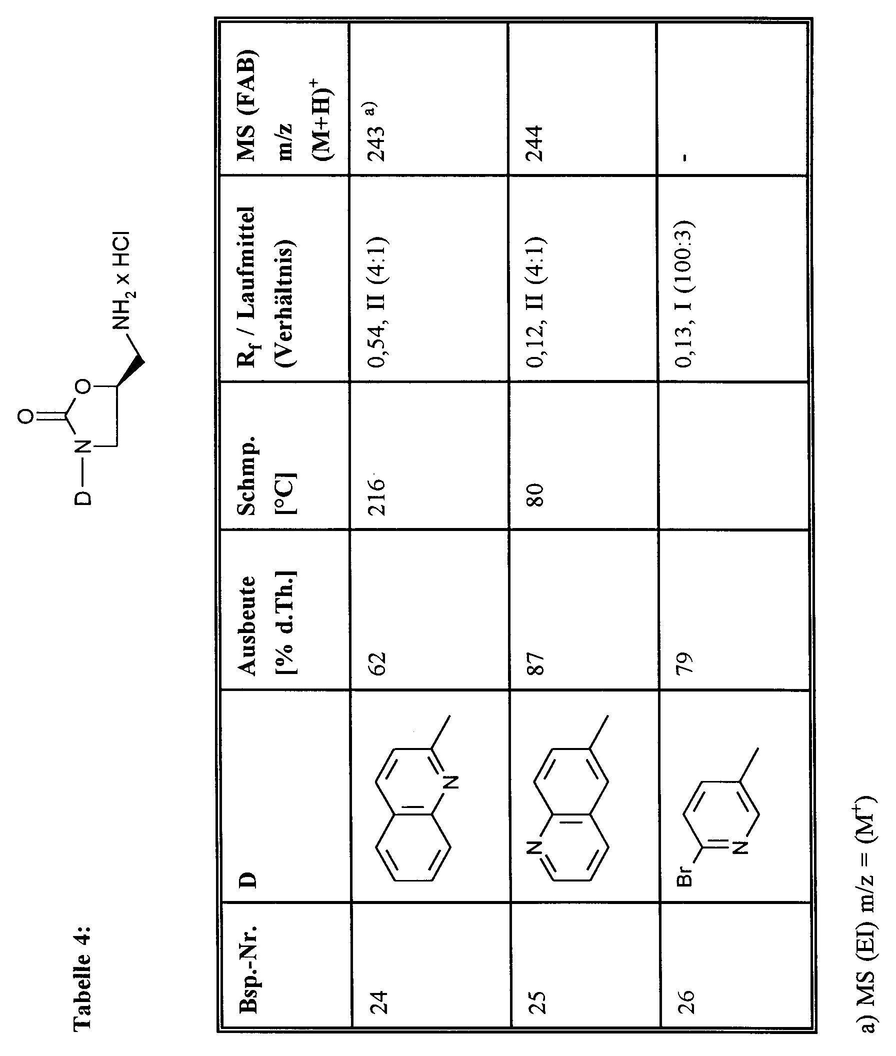

### Beispiel 27

### (5S)-3-(5-Brom-pyridin-2-yl)-5-acetylaminomethyl-oxazolidin-2-on

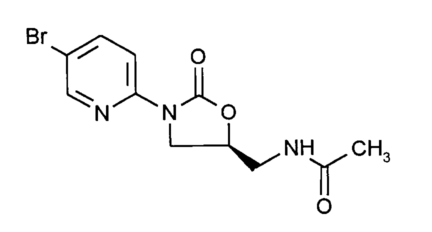

Eine gerührte Lösung von 8,90 g (28,84 mmol) der Verbindung aus Beispiel 23 in 35 ml THF wird mit einer Lösung von 1,03 g (25,73 mmol) Natriumhydroxid in 8,4 ml Wasser versetzt. Dazu tropft man bei 0-5°C langsam 2,68 ml (28,30 mmol) Acetanhydrid in 3 ml THF und hält pH = 9 durch gleichzeitige Zugabe einer 5 N wäßrigen NaOH-Lösung. Man rührt 1 h bei 0°C nach und dampft das Lösemittel im Vakuum ab. Der Rückstand wird mit 2 x 20 ml Wasser gut verrührt, abgetrennt und im Hochvakuum über Sicapent getrocknet. Man erhält 8,90 g (98%) der Titelverbindung als farblose Kristalle.
Schmp.: 166-168°C
R_{f} = 0,57 (Acetonitril : Wasser 95:5)
MS (EI) m/z= 313 (M⁺)
¹H-NMR (250 MHz, D₆-DMSO) δ = 1,82 (s, 3H, COCH₃); 3,42 (t, J = 6,5 Hz, 2H, CH₂N); 3,84 (dd, J = 7, 9 HZ, 1H, H-4 trans); 4,18 (dd, J 0 9, 10 Hz, 1H, H-4 cis); 4,75 (m, 1H, H-5); 8,05 (s, 2H, Pyridyl H-3,4); 8,23 (m, 1H, NHCO); 8,50 (s, 1H, Pyridyl H-6).

Wie für Beispiel 27 beschrieben erhält man durch Acylierung der entsprechenden Amine die folgenden Produkte (Tabelle 5):

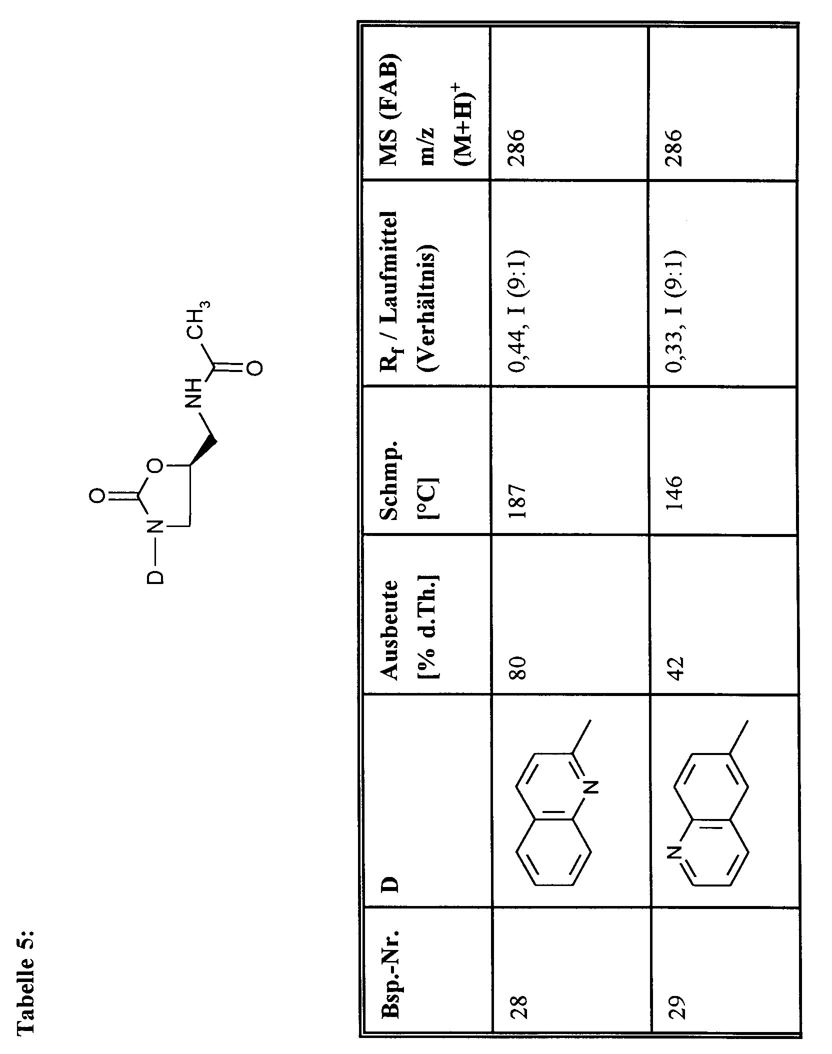

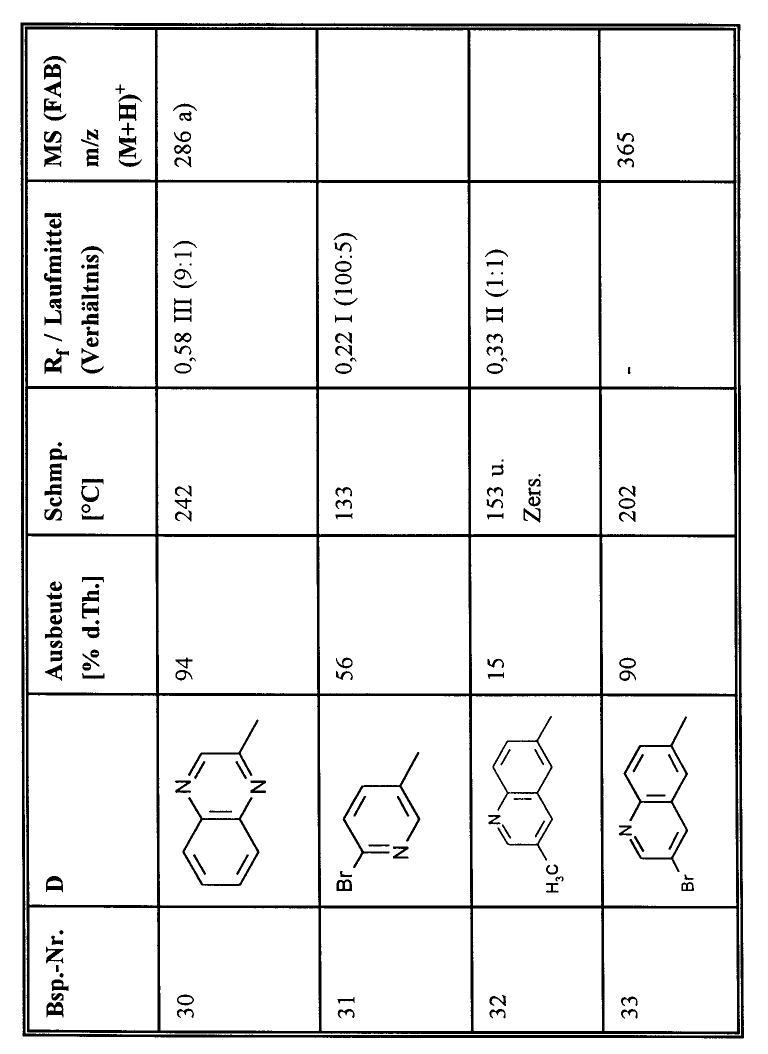

### Beispiel 34 und Beispiel 35

### (5S)-3-(6-Brom-chinolin-2-yl)-5-acetaminomethyl-oxazolidin-2-on und (5S)-3-(8-Brom-chinolin-2-yl)-5-acetaminomethyl-oxazolidin-2-on

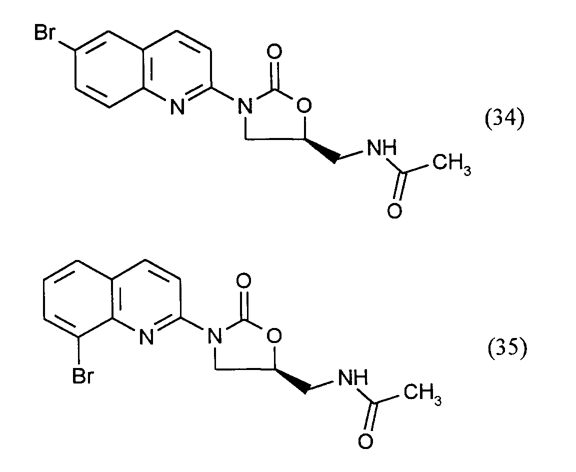

Zu einer gerührten, auf 0°C gekühlten Lösung von 4,38 g (15,20 mmol) der Verbindung aus Beispiel 28 in 87 ml Chloroform und 56 ml Acetonitril gibt man 4,36 g(19,73 mmol) Silbertrifluoroacetat. Danach tropft man innerhalb von 15 min 0,78 ml (15,20 mmol) einer Maßlösung von Brom in Chloroform zu. Das Eisbad wird entfernt und man rührt 4 h bei Raumtemperatur nach. Zur Aufarbeitung rührt man das Gemisch in 100 ml Ethylacetat ein, wäscht mit 2 x 50 ml gesättiger NaHCO₃-Lösung und 50 ml NaCl-Lösung und trocknet die organische Phase über MgSO₄. Das Lösemittel wird im Vakuuum abgedampft und der Rückstand mit 50 ml Ether / n-Pentan verrührt. Der Niederschlag wird durch Filtration abgetrennt, und im Hochvakuum getrocknet. Man erhält 5,43 g (98%) der Titelverbindung als Gemisch der Isomere. Trennung dieses Gemisches an 540 g Kieselgel (Ethylactat) ergab 2,70 g (43%) des unpolaren 8-Brom-Isomers als farblose Kristalle, Schmp.: 211°C
R_{f} = 0,29 (Ethylacetat)
MS (DCI, NH₃) m/z = 364 (M+H)⁺
¹H-NMR (250 MHz, D₆-DMSO) δ = 1,85 (s, 3H, COCH₃); 3,50 (m, 2H, CH₂NH); 4,01 (dd, J= 7, 10 Hz, 1H, H-4 trans); 4,45 (dd, J = 9, 10 Hz, 1H, H-4 cis); 4,85 (m, 1H, H-5); 7,45 (t, J = 7 Hz, 1H, Chinolin H-6); 7,99 (dd, J = 1, 7 Hz, 1H, Chinolin H-7); 8,11 (dd, J = 1, 7 Hz, 1H, Chinolin H-5); 8,29 (m, 1H, NHCO); 8,43 (m, 2H, Chinolin H-3,4).
und 1,02 g (16%) des polaren 6-Brom-Isomers, Schmp.: 210-213°C
R_{f} = 0,22 (Ethylacetat)
MS (DCI, NH₃) m/z = 364 (M+H)⁺
¹H-NMR (250 MHz, D₆-DMSO) δ = 1,85 (s, 3H, COCH₃); 3,48 (m, 2H, CH₂N); 4,00 (dd, J = 6, 10 Hz, 1H, H-4 trans); 4,36 (dd, J = 9, 10 Hz, 1H, H-4 cis); 4,80 (m, 1H, H-5); 7,8 (m, 2H, Chinolin H-7,8); 8,21 (d, J = 1 Hz, 1H, Chinolin H-5); 8,27 (m, 1H, NHCO); 8,37 (s, 2H, Chinolin H-3,4)
sowie 830 mg (13%) einer Mischfraktion beider Isomere.

### Beispiel 36

### (5S)-3-[5-(4-Methylphenyl)pyridin-2-yl]-5-acetyl-aminomethyl-oxazolidin-2-on

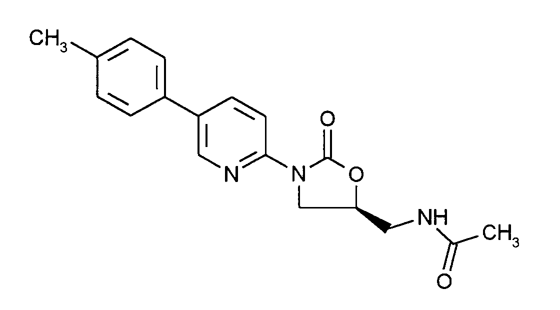

Eine gerührte Lösung von 943 mg (3,00 mmol) der Verbindung aus Beispiel 27 und 530 mg (3,90mmol) 4-Methylphenyl-boronsäure in 15,4 ml THF werden mit 104 mg (0,09 mmol) Tetrakis(triphenylphosphin)palladium versetzt und 1 h zum Rückfluß erhitzt. Danach gibt man 2,07 ml (4,14 mmol) einer 2 M Na₂CO₃-

Lösung zu und erhitzt 30 h am Rückfluß. Danach darf das Gemisch abkühlen, das Lösemittel wird im Vakuum abgedampft und der Rückstand wird durch Chromatographie an 88 g Kieselgel (Ethylacetat) gereinigt. Nach Umkristallisation aus Ethanol erhält man 582 mg (60%) der Titelverbindung als farblose Kristalle. Schmp.: 186-188°C
R_{f} = 0,18 (Ethylacetat)
MS (EI) m/z = 325 (M)⁺
¹H-NMR (200 MHz, D₆-DMSO): δ = 1,85 (s, 3H, COCH₃); 2,36 (s, 3H, CH₃); 3,45 (m, 2H, CH₂N); 3,91 (dd, J = 7, 10 Hz, 1H, H-4 trans); 4,25 (dd, J = 10, 10 Hz, 1H, H-4 cis); 4,77 (m, 1H, H-5); 7,30, 7,61 (AB, J_{AB} = 9 Hz, 4H, Tolyl-H); 8,15 (s, 2H, Pyridyl H-3,4); 8,28 (bt, J = 6 Hz, 1H, NHCO); 8,68 (m, 1H, Pyridyl H-6).

In Analogie zur Vorschrift des Beispiels 36 werden die in den Tabellen 6 und 7 aufgeführten Verbindungen hergestellt:

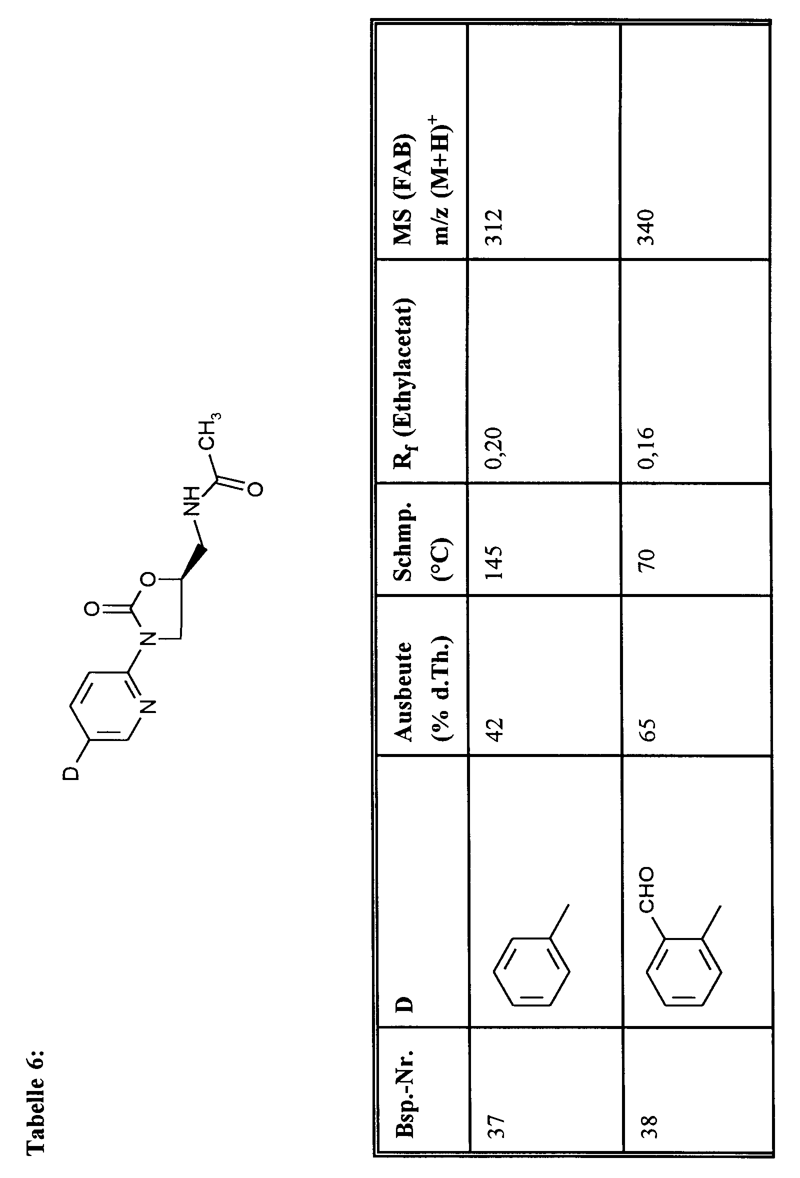

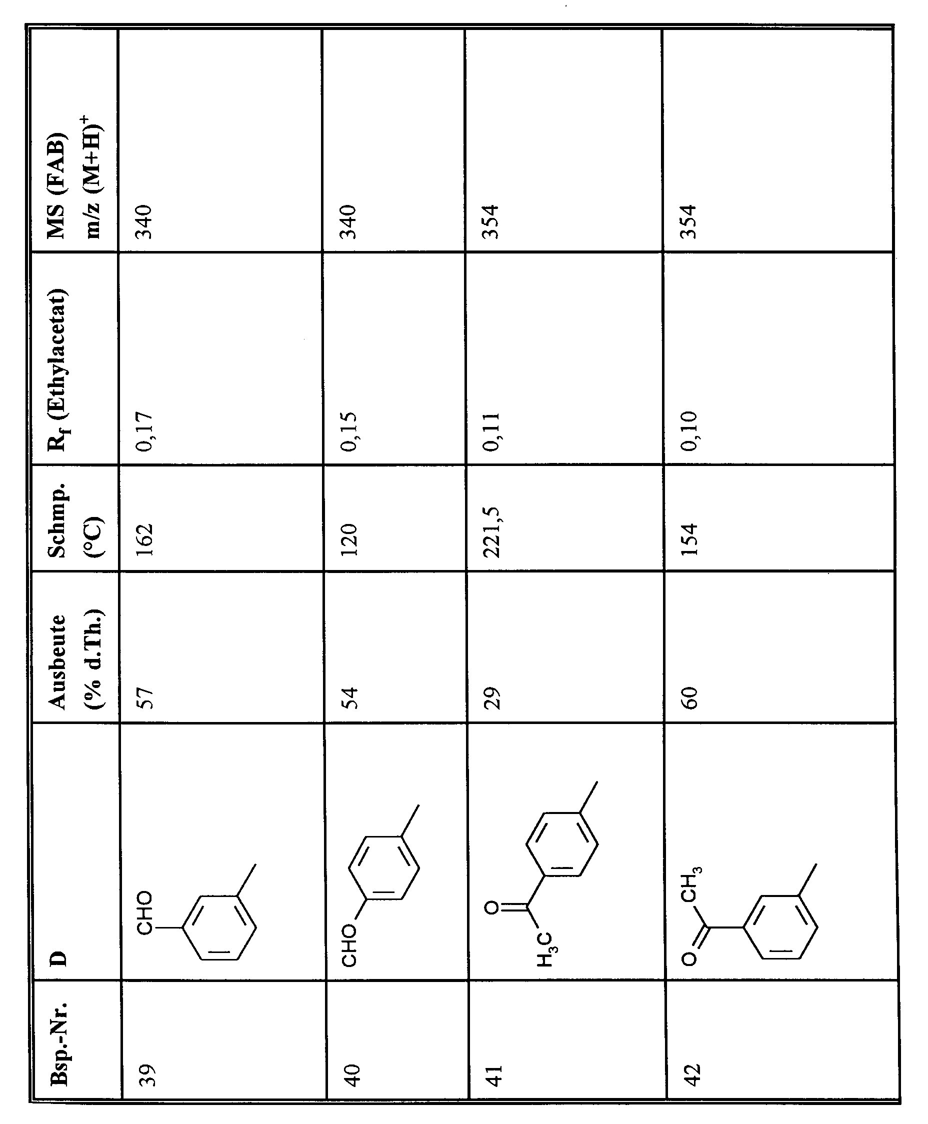

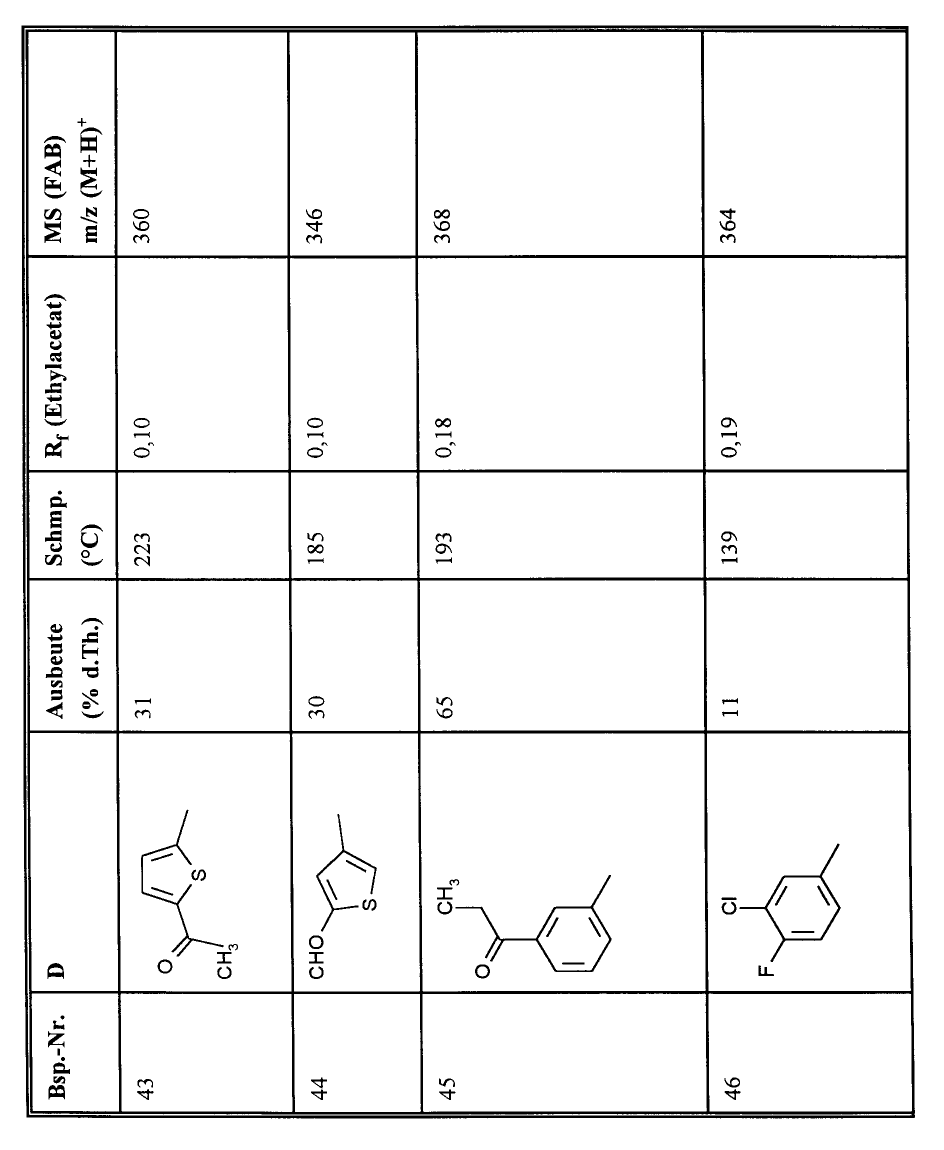

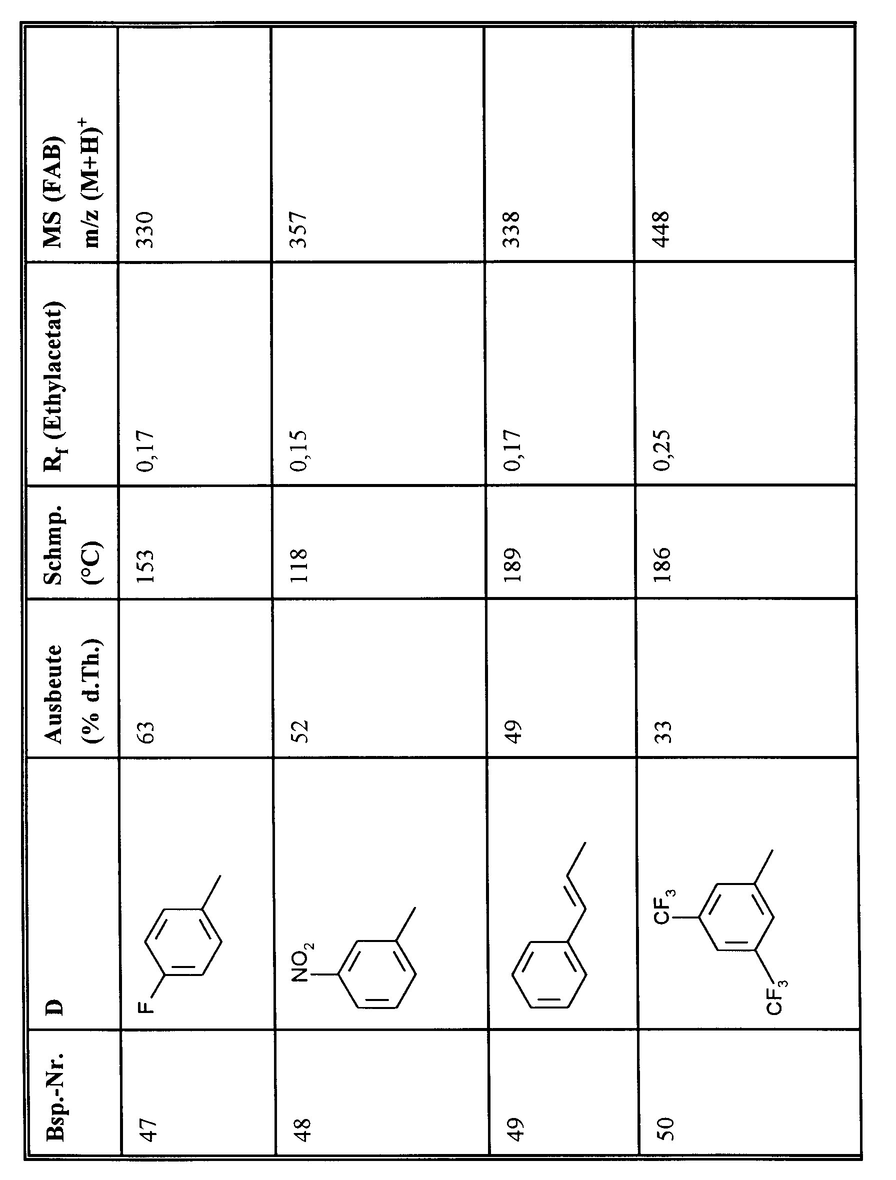

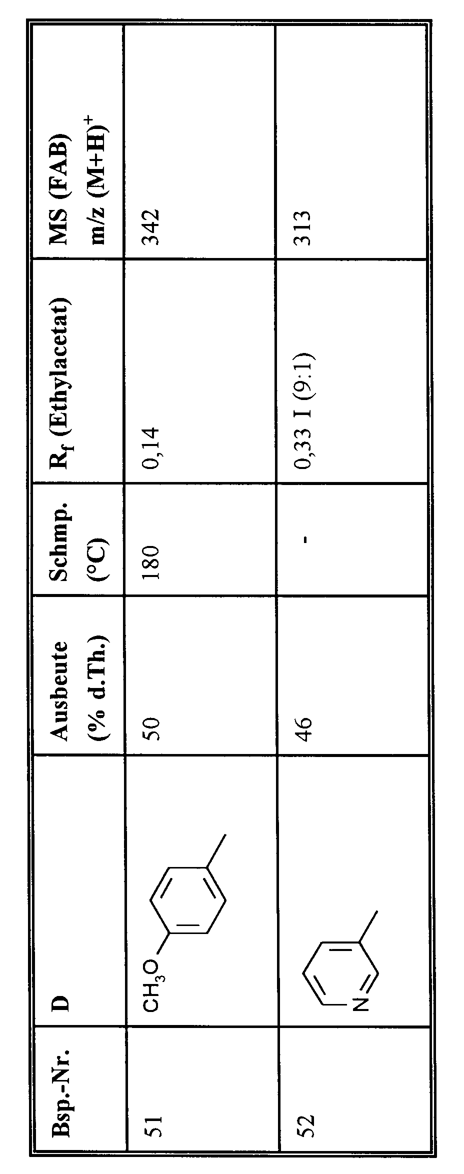

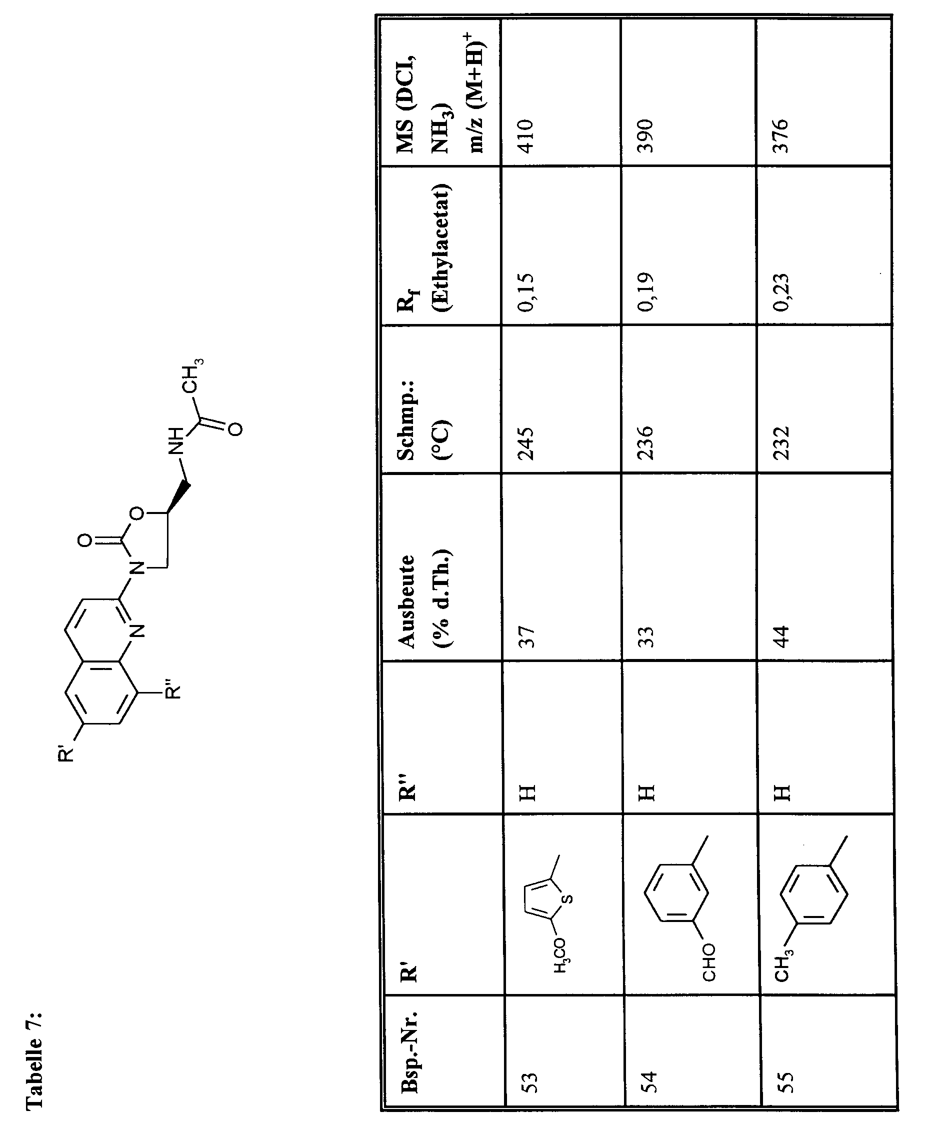

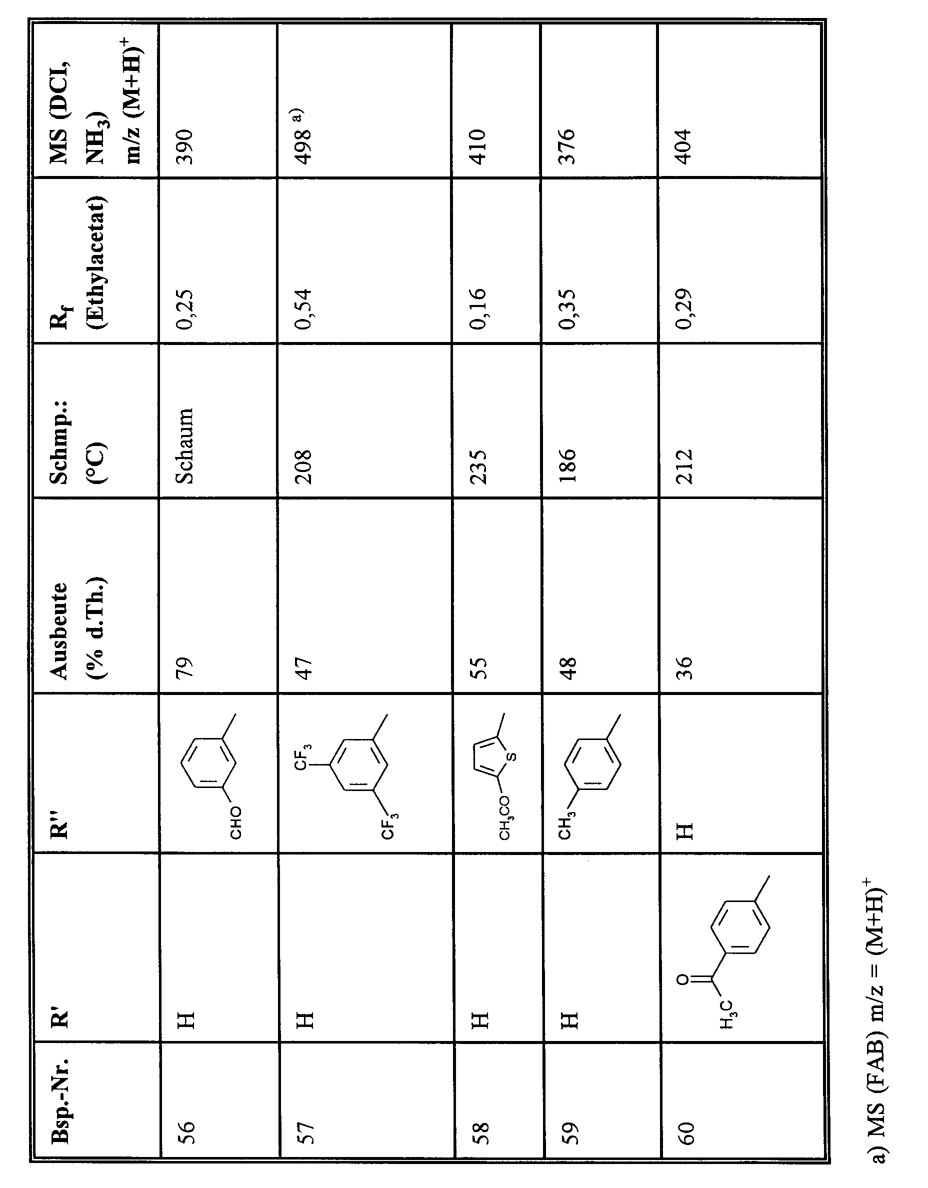

### Beispiel 61

### (5S)-3-[5-(2-Hydroxymethyl-phenyl)-pyridin-2-yl]-5-acetyl-aminomethyl-oxazolidin-2-on

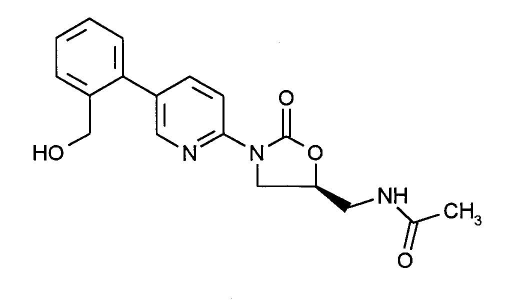

Eine gerührte, auf 0°C gekühlte Lösung von 88 mg (0,26 mmol) der Verbindung aus Beispiel 38 in 3 ml Methanol wird mit 8 mg (0,20 mmol) Natriumborhydrid versetzt und 4 h bei 0°C gerührt. Das Lösemittel wird im Vakuum abgedampft und der Rückstand wird durch Chromatographie an 9 g Kieselgel (Ethylacetat) gereinigt. Man erhält 25 mg (27%) der Titelverbindung als farblose Kristalle. Schmp.: ab 85°C Zersetzung
R_{f} = 0,06 (Ethylacetat)
MS (DCI, NH₃) m/z = 342 (M+H)⁺
¹H-NMR (250 MHz, D₆-DMSO) δ = 1,86 (s, 3H,COCH₃); 3,46 (m, 2H, CH₂N); 4,02 (dd, J = 8, 10 Hz, 1H, H-4 trans); 4,28 (dd, J = 10, 10 Hz, 1H, H-4 cis); 4,40 (d, J = 6 Hz, 2H, CH₂O); 4,76 (m, 1H, H-5); 5,21 (t, 1H, OH); 7,3 - 7,6 (m, 4H, H arom); 8,91 (dd, J = 1,5, 9 Hz, 1H, Pyridyl H-4); 8,12 (d, J = 9 Hz, 1H, Pyridyl H-3); 8,27 (m, 1H, CONH); 8,40 (d, J = 1,5 Hz, 1H, Pyridyl H-6).

### Beispiel 62

### (5S)-3-(Chinolin-6-yl)-5-acetylaminomethyl-oxazolidin-2-on Hydrochlorid

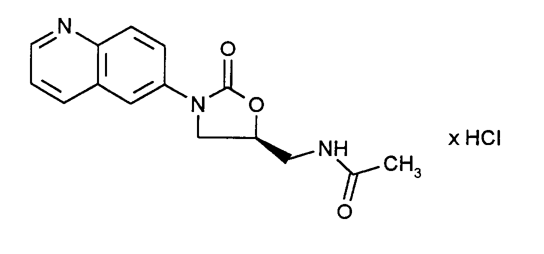

Zu einer gerührten Lösung von 285 mg (1,00 mmol) der Verbindung aus Beispiel 29 in 5 ml wasserfreiem Dioxan tropft man 5 ml einer 1 N Lösung von gasförmigem Chlorwasserstoff in Ether. Man rührt 30 min bei Raumtemperatur nach, gibt 20 ml Ether zu, rührt gut durch und trennt den Niederschlag durch Filtration ab. Der Niederschlag wird in 30 ml Wasser gelöst, die Lösung wird durch eine "Millipore-Membran" (0,2 µ) gepreßt und gefriergetrocknet. Man erhält 300 mg (93%) der Titelverbindung als farbloses Lyophilisat, welches im Hochvakuum über NaOH getrocknet wird.
¹H-NMR (300 MHz, D₂O): δ = 2,02 (s, 3H, COCH₃); 3,71 (m, 2H, CH₂N); 4,15 (dd, J = 10 Hz, 1H, H-4 trans); 4,43 (dd, J = 10 , 10 Hz, 1H, H-4 cis); 5,02 (m, 1H, H-5); 8,07 (dd, J = 6, 9 Hz, 1H, Chinolin H-3); 8,16 (d, J = 1 Hz, 1H, Chinolin H-5); 8,23 (d, J = 10 Hz, 1H, Chinolin H-8); 8,50 (dd, J = 1, 10 Hz, 1H, Chinolin H-7); 9,05 (m, 2H, Chinolin H-2, 4).

### Beispiel 63

### (5S)-3-(1-Methyl-chinolin-6-yl)-5-acetylaminomethyl-oxazolidin-2-on Jodid

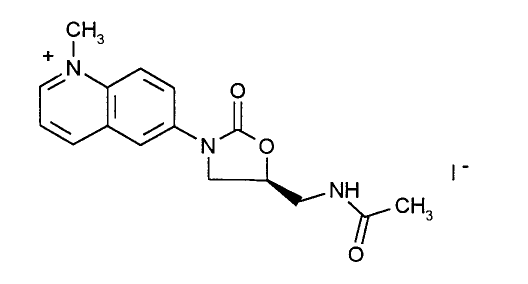

Eine gerührte Lösung von 314 mg (1,10 mmol) der Verbindung aus Beispiel 29 in 3 ml wasserfreiem Acetonitril wird mit 0,35 ml (5,05 mmol) Iodmethan versetzt und 2 h bei Raumtemperatur gerührt, wobei ein heller Niederschlag entsteht. Man gibt 50ml Ether zu, rührt für 10 min gut durch, trennt den Niederschlag durch Filtration ab, wäscht mit 5 ml Ether und trocknet im Hochvakuum. Man erhält 451 mg (96%) der Titelverbindung als helle Kristalle.
Schmp.: 196°C u.Z.
R_{f} = 0,06 (Acetonitril / Wasser 4:1)
MS (FAB): 300 (M⁺, 100) freies Kation
¹H-NMR (D₆-DMSO, TMS): 9,4 (d, J = 6 Hz, 1H); 9,22 (d, J = 8 Hz, 1H); 8,7 (dd, J = 12 Hz, J = 3 Hz, 1H); 8,56 (d, J = 12 Hz, 1H); 8,25 - 8,4 (m, 2H); 8,15 (dd, J = 8 Hz, J = 6 Hz, 1H); 4,8 - 4,95 (m, 1H); 4,62 (s, 3H); 4,33 (t, J = 10 Hz, 1H); 3,95 (dd, J = 10 Hz, J = 7 Hz, 1H); 3,45 - 3,57 (m, 2H); 1,83 (s, 3H).

Wie für Beispiel 63 beschrieben werden die in der Tabelle 8 aufgeführten Verbindungen hergestellt:

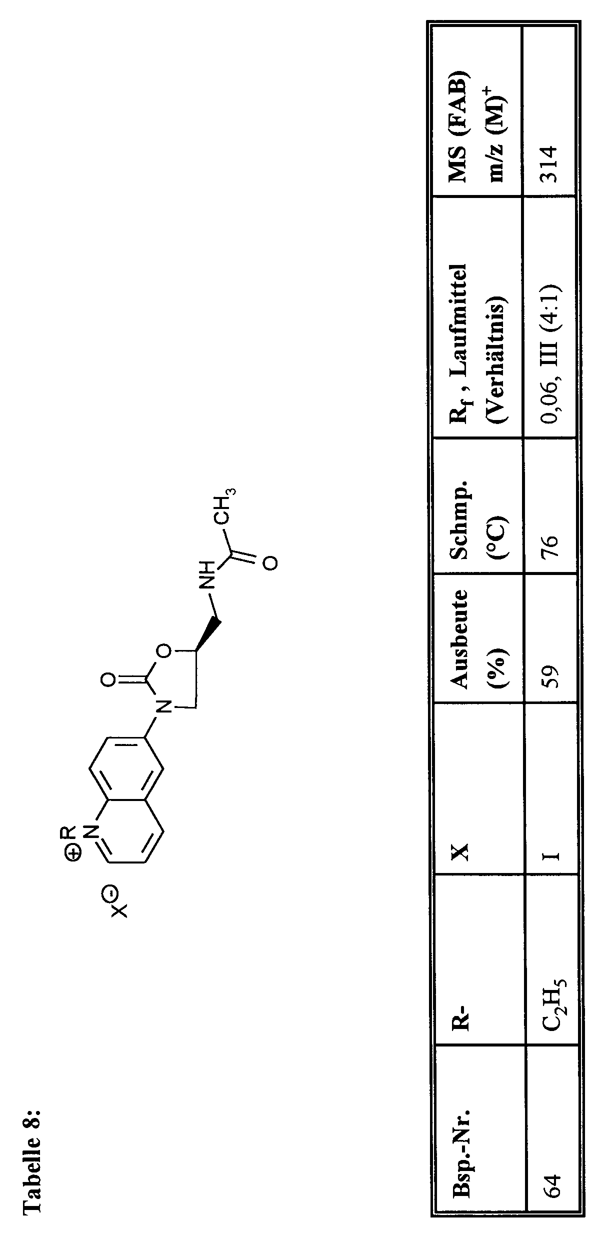

### Beispiel 65

### (5R)-3-(Chinolin-6-yl)-5-acetylaminomethyl-oxazolidin-2-on-N-1-oxid

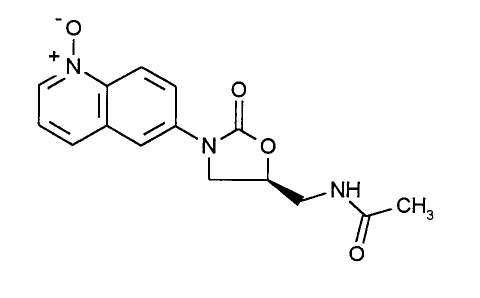

Eine gerührte Lösung von 500 mg (1,75 mmol) der Verbindung aus Beispiel 29 in 5 ml Dichlormethan wird mit 832 mg (3,85 mmol) 80%iger n-Chlorperbenzosäure versetzt und 16 h bei Raumtemperatur gerührt. Danach wird die Reaktionsmischung in 20 ml 10%iger wäßriger Na₂SO₃-Lösung eingerührt. Die wäßrige Phase wird abgetrennt und im Vakuum eingedampft. Man gibt 25 ml Toluol und 1,5 g Kieselgel zu und dampft erneut ein. Der Rückstand wird durch Chromatographie an 50 g Kieselgel (Dichlormethan : Methanol 4:1) gereinigt. Die produkthaltigen Fraktionen werden vereinigt und mit 200 ml Ether versetzt. Der entstandene Niederschlag wird durch Filtration abgetrennt und im Hochvakuum getrocknet. Man erhält 453 mg (86%) der Titelverbindung als farblose Kristalle. Schmp.: 191°C (Zers.)
R_{f} = 0,15 (Dichlormethan : Methanol 9:1)
MS (FAB) m/z = 302 (M+H)⁺
¹H-NMR (300 MHz, D₆-DMSO): δ = 1,85 (s, 3H, COCH₃); 3,50 (m, 2H, CH₂N); 3,91 (dd, J = 7, 10 Hz, 1H, H-4 trans); 4,28 (dd, J = 10, 10 Hz, 1H, H-4 cis); 4,82 (m, 1H, H-5); 7,3 - 7,5 (m, 2H); 7,9 (m, 1H); 8,0 (s, 1H, Chinolin H-5); 8,3 (m, 1H); 8,50 (m, 1H, Chinolin H-2).

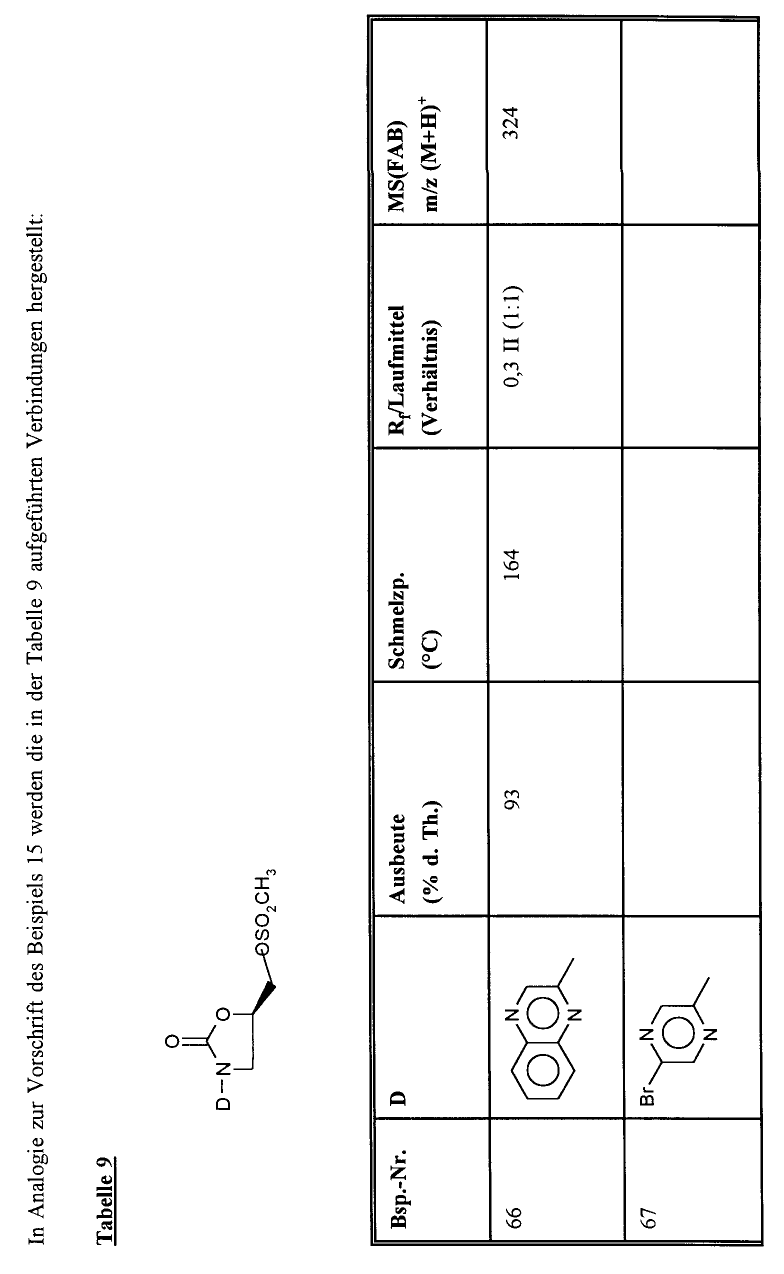

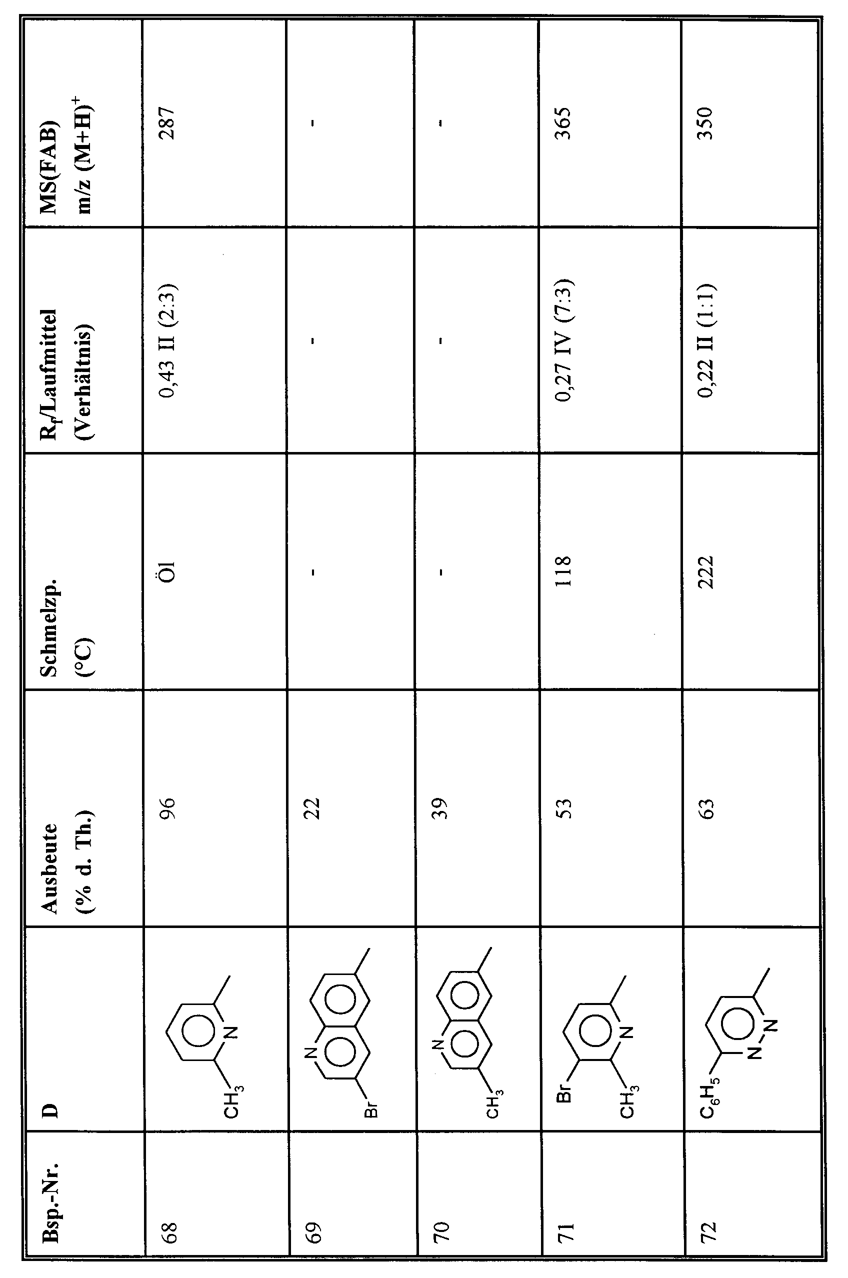

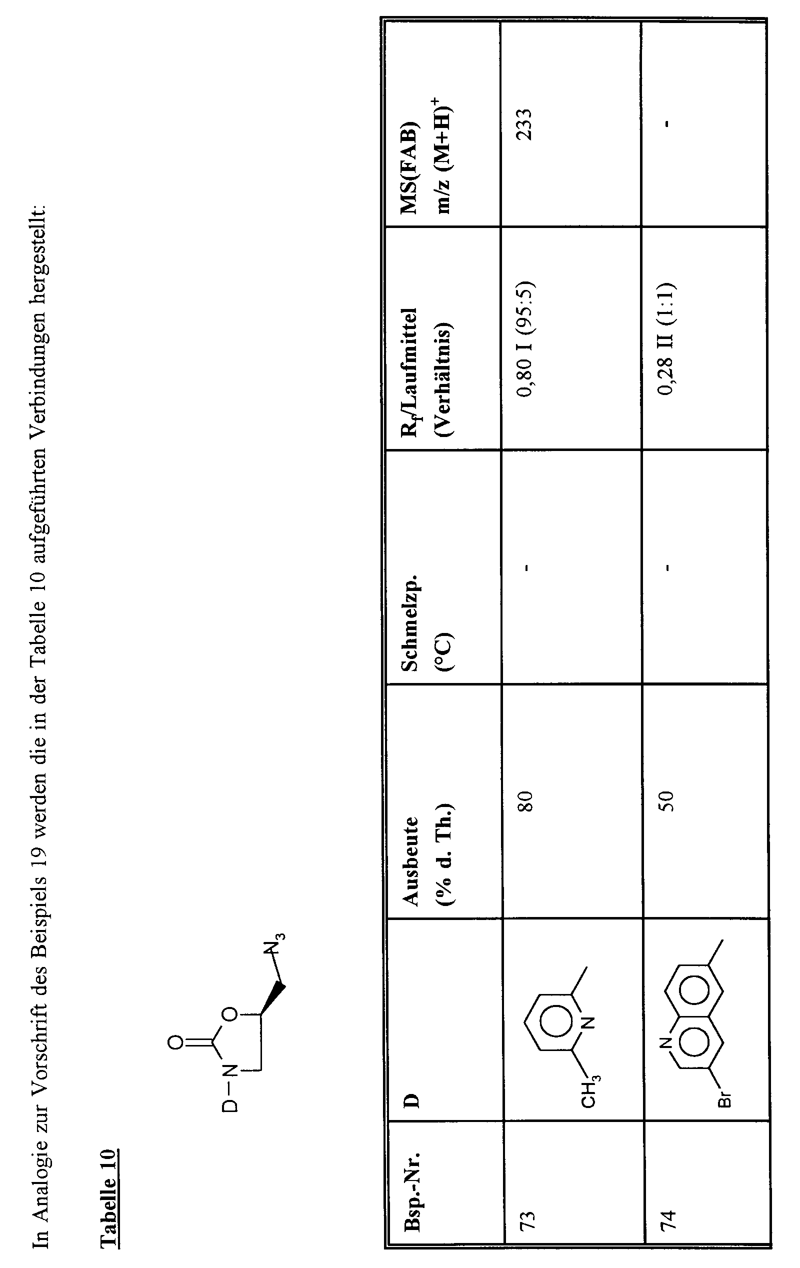

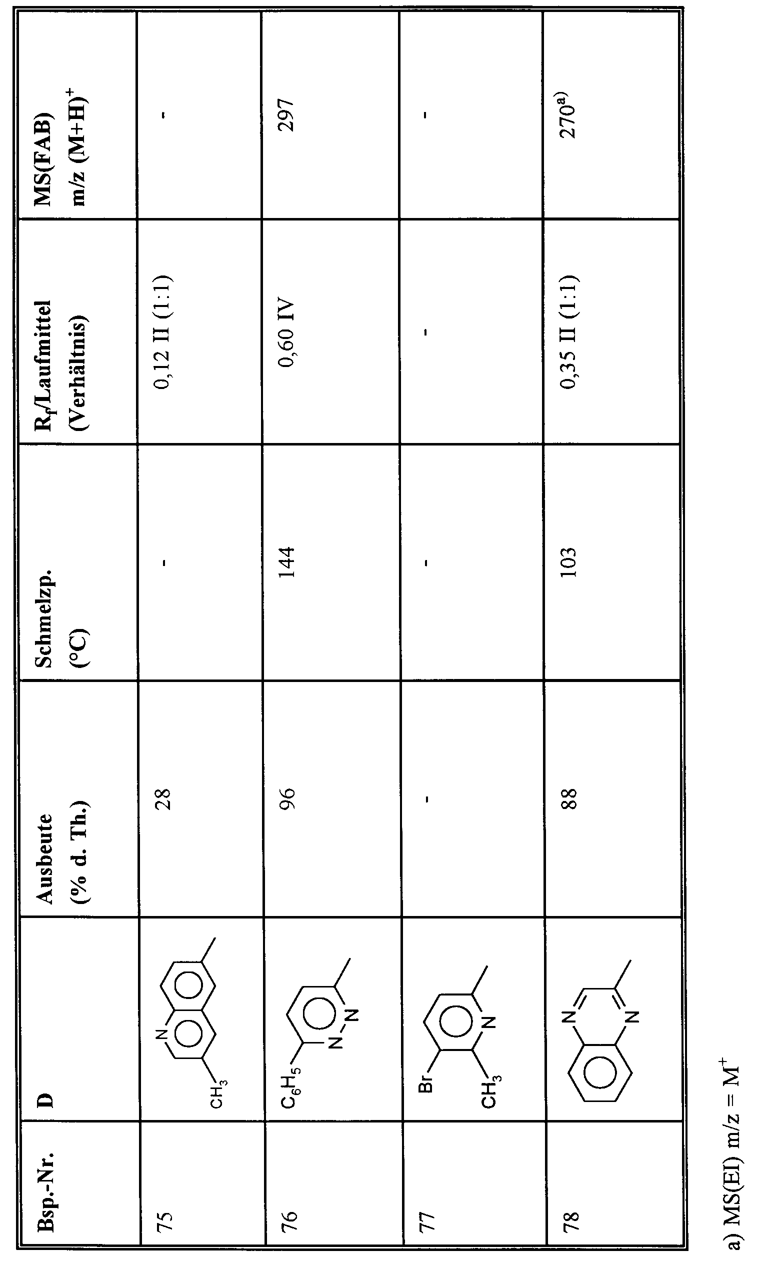

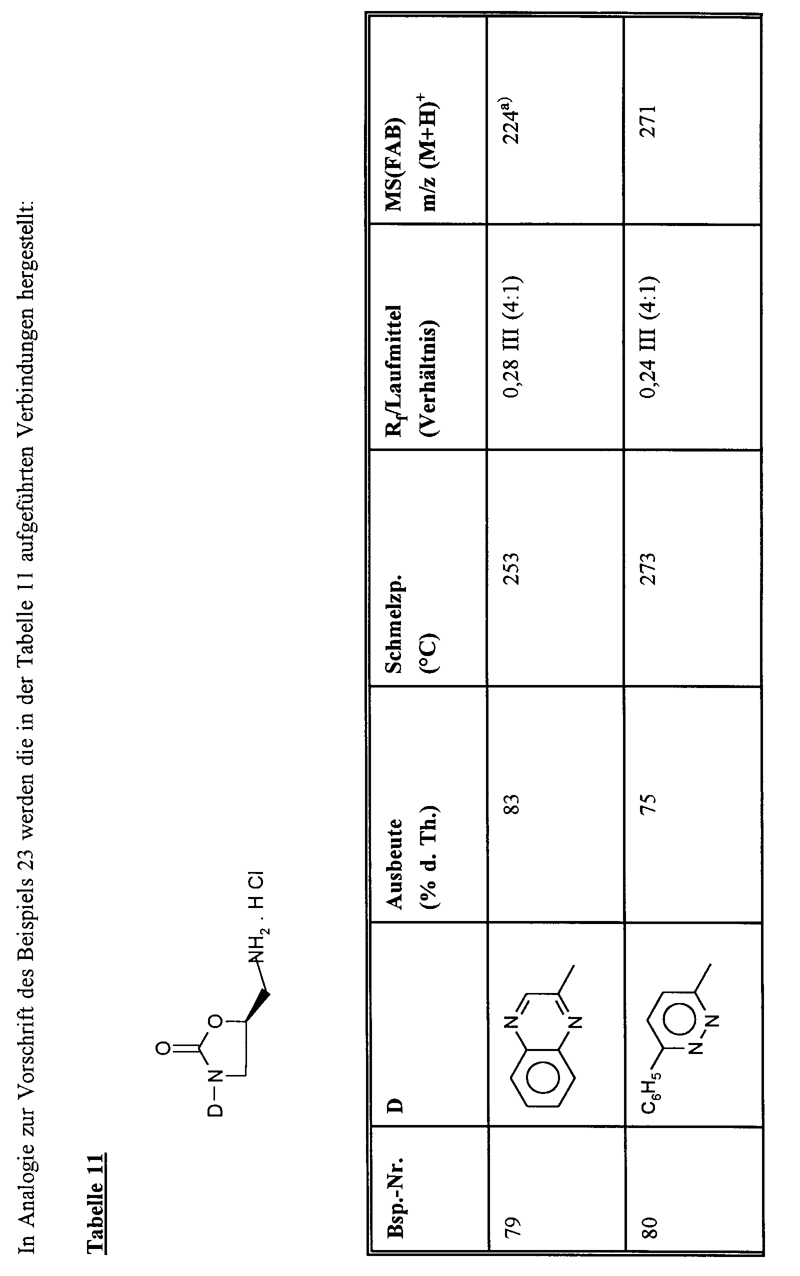

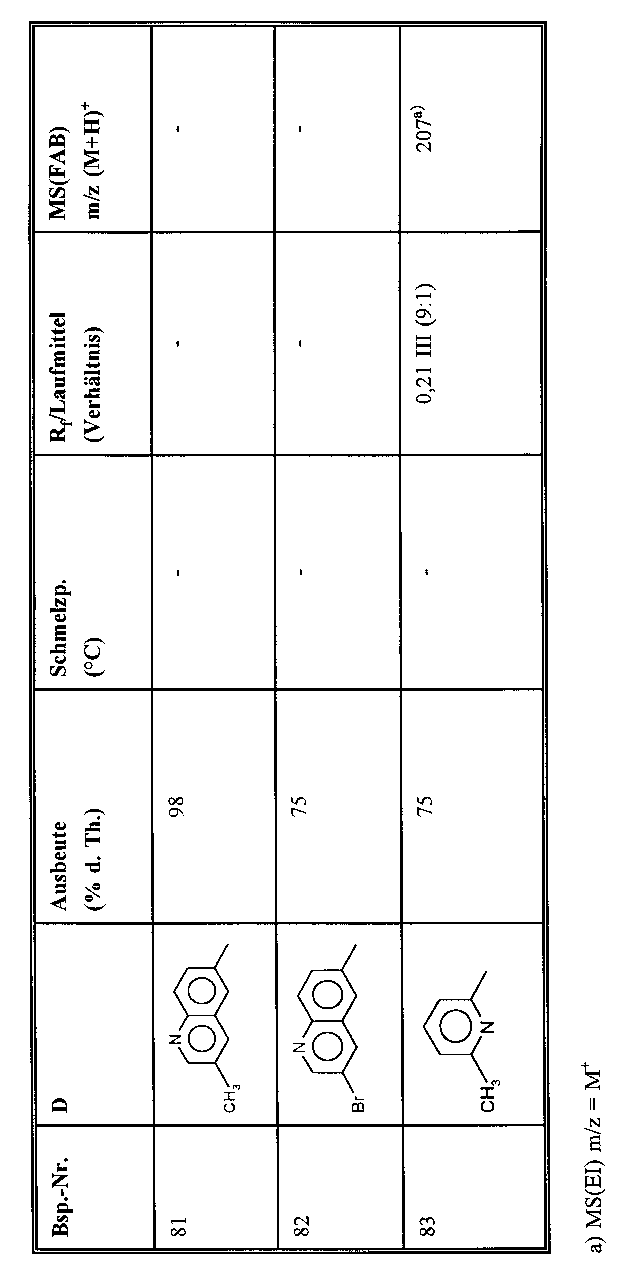

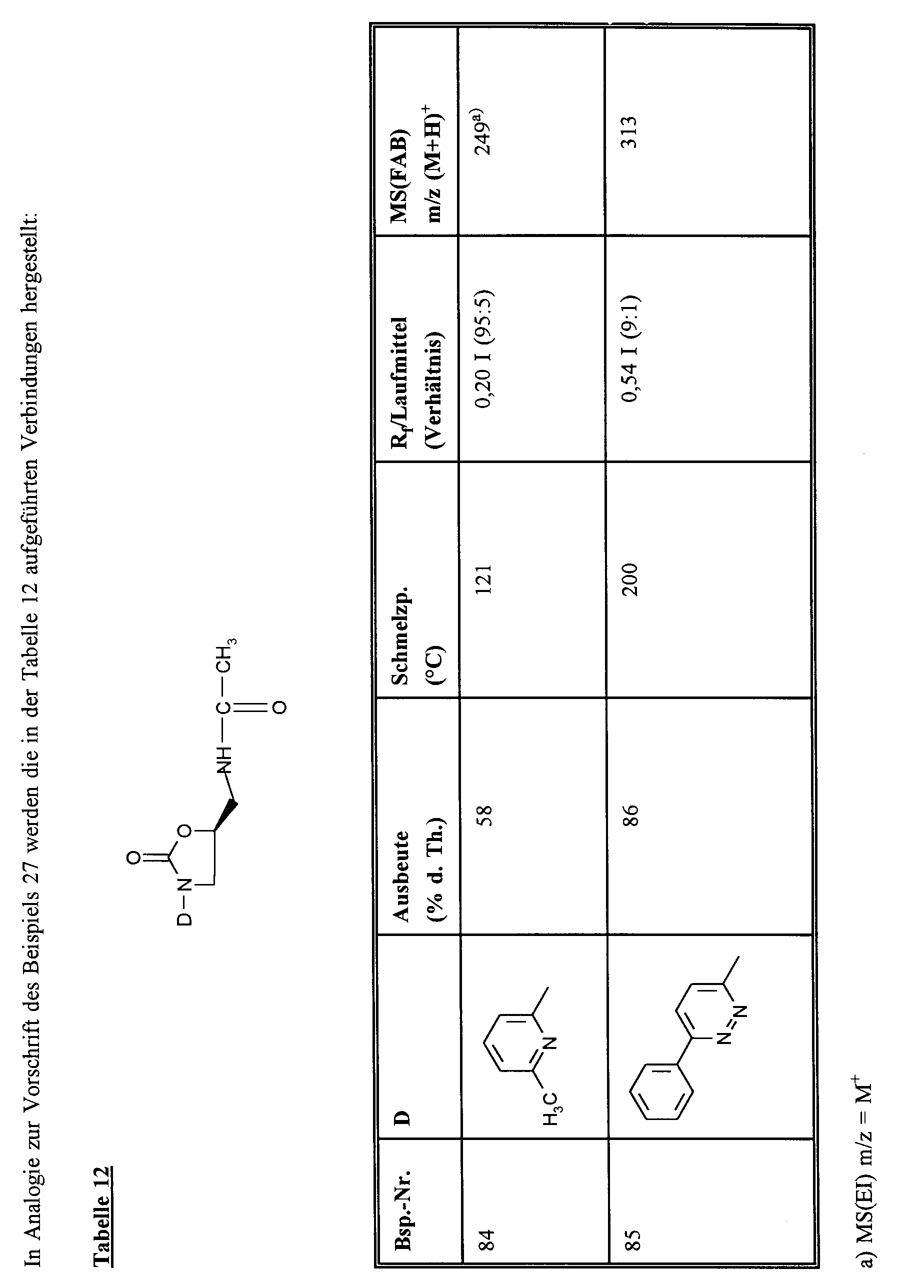

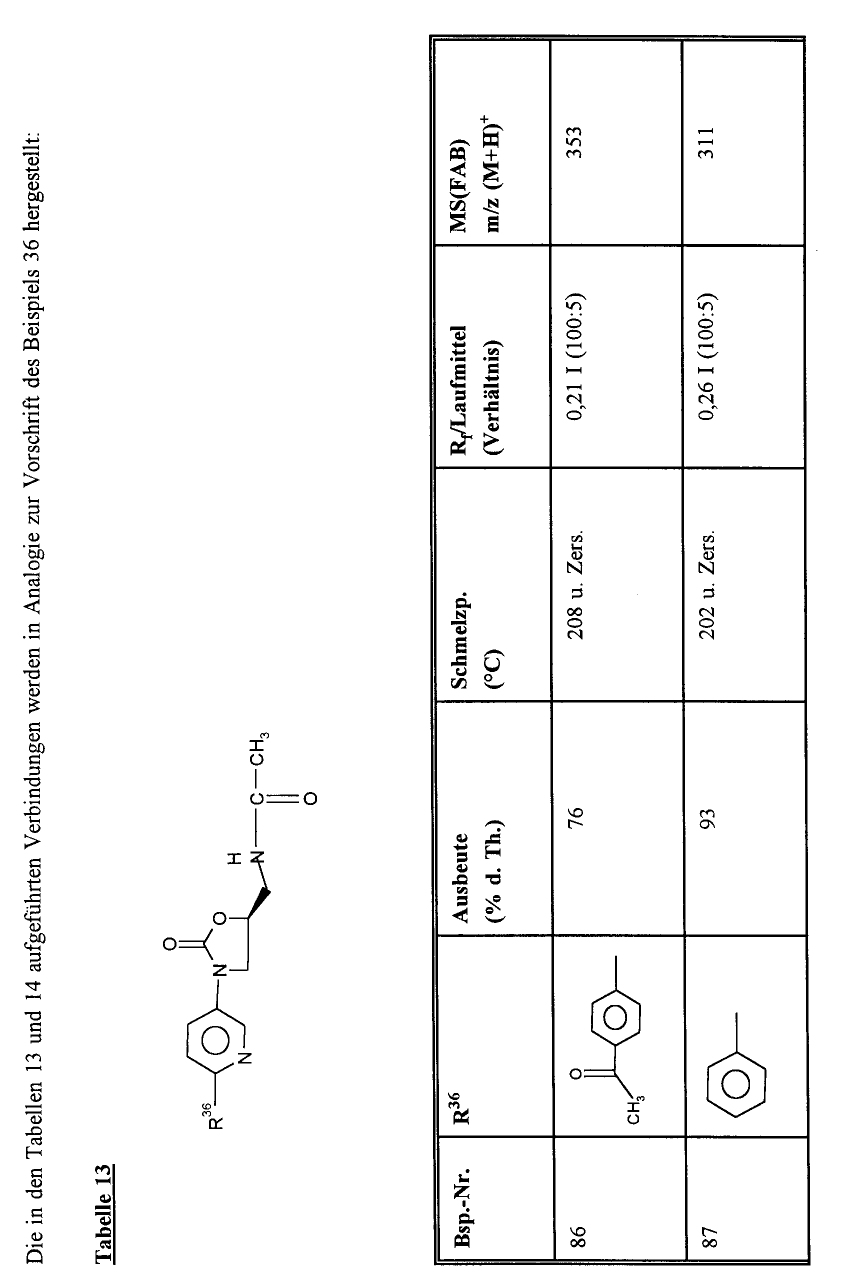

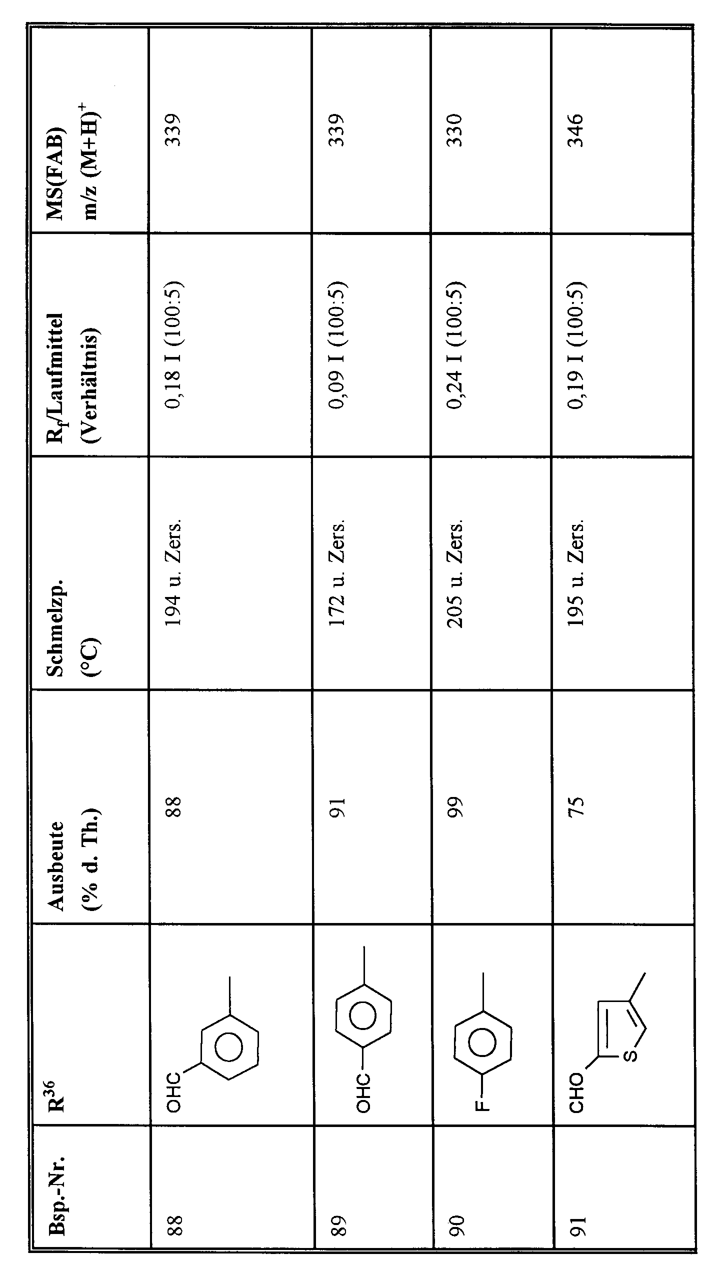

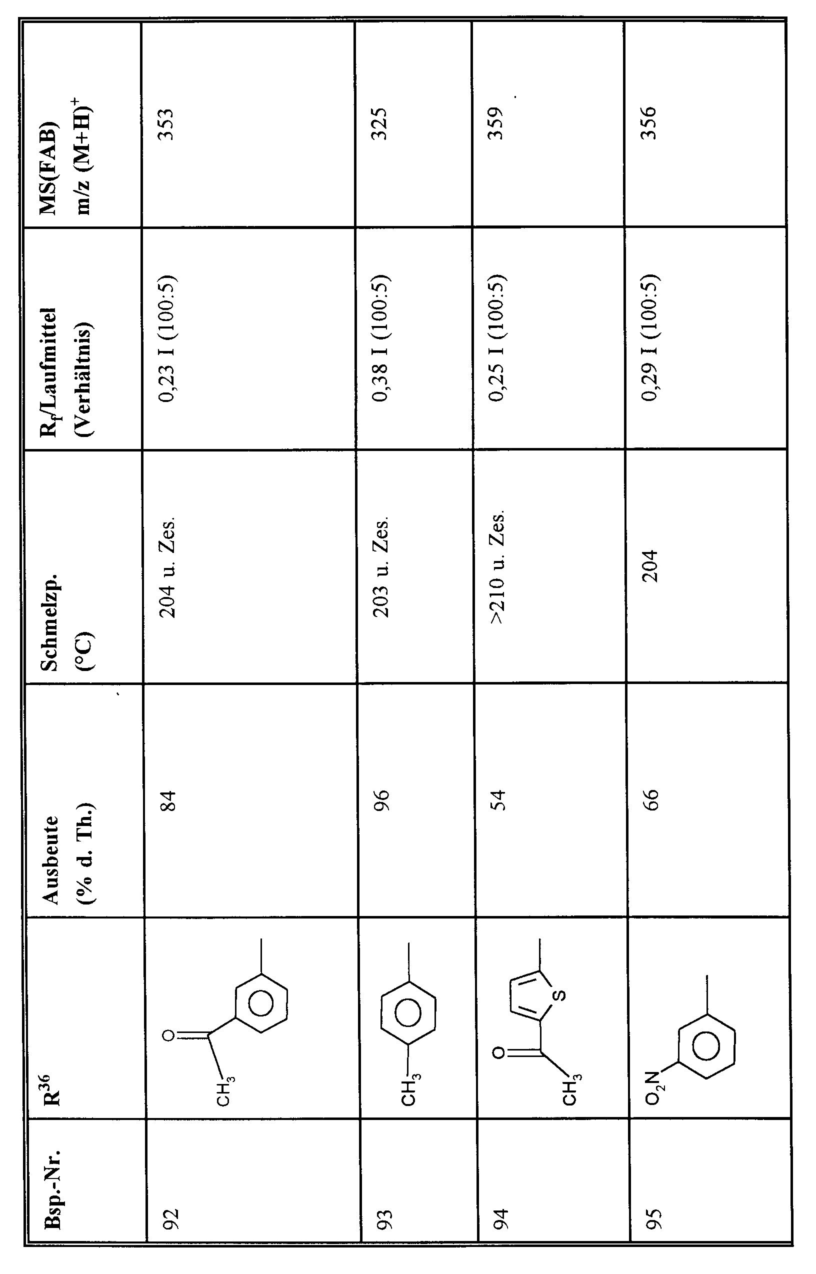

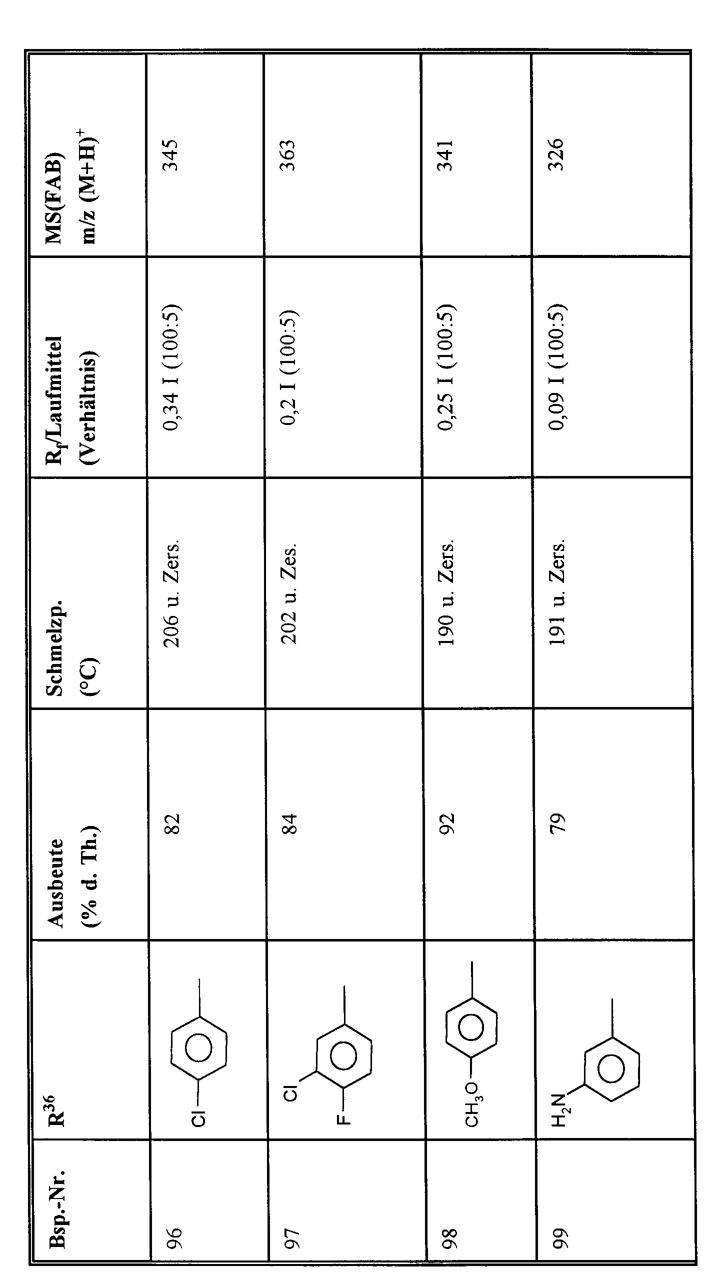

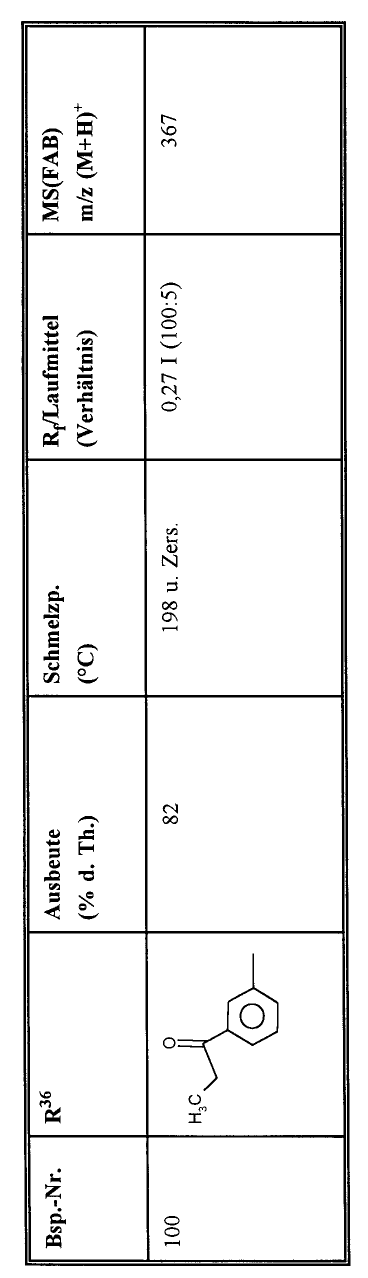

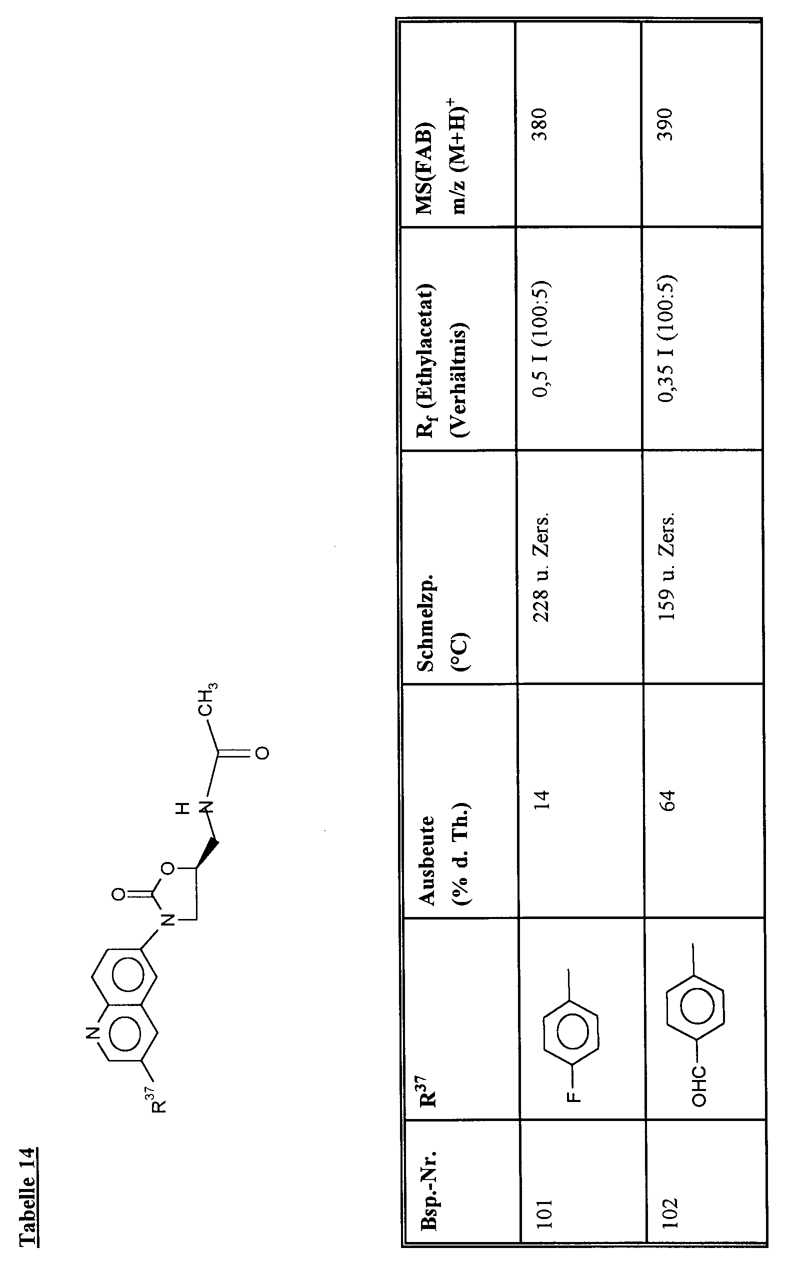

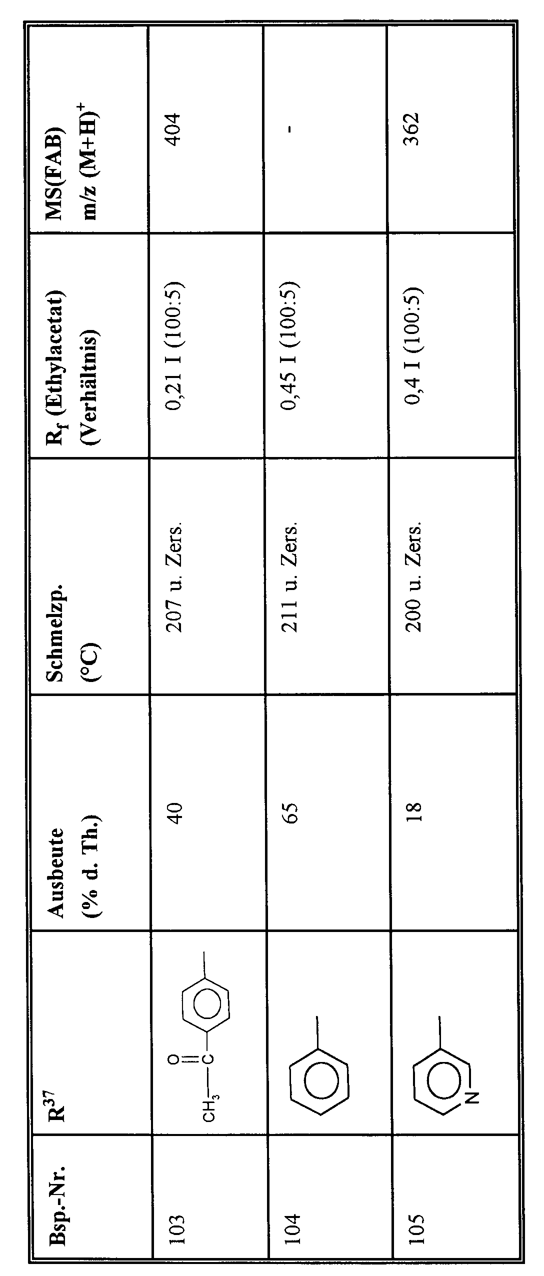

### Beispiel 106

### (5S)-3-[5-(4-(Piperidin-1-yl)-phenyl)-pyridin-2-yl]-5-acety-aminomethyl-oxazolidin-2-on

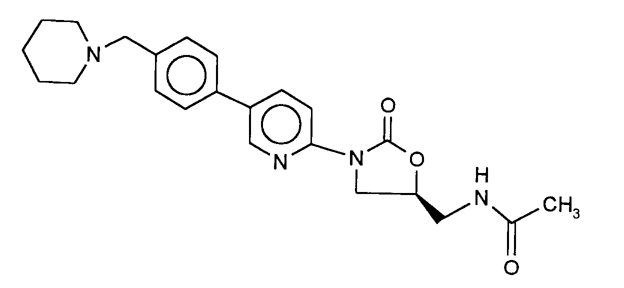

Zu einer gerührten Suspension von 340 mg (1.00 mmol) des Aldehyds aus Beispiel 40 und 86 mg (1.00 mmol) Piperidin in 10 ml Dichlormethan werden 0,37 ml (1.26 mmol) Tetraisopropoxytitan versetzt und 1 Stunde bei Raumtemperatur gerührt, wobei eine klare Lösung entsteht. Danach wird das Lösungsmittel im Vakuum abgedampft, der Rückstand wird in 2 ml Ethanol gelöst, 44 mg (0,67 mmol) Natriumcyanoborhydrid werden zugesetzt, und das Gemisch wird 18 h bei Raumtemperatur gerührt. Das Lösungsmittel wird im Valuum abgedampft und der Rückstand wird in 40 ml eines Gemischs aus Ethylacetat und Wasser 1:1 aufgenommen. Dir organische Phase wird abgetrennt, mit 2 x 10 ml Wasser und 10 ml NaCl-Lösung gewaschen und über MgSO₄ getrocknet. Nach Abdampfen des Lösungsmittels und Chromatographie des Rückstands an 80 g Kieselgel (Dichlormethan:Methanol 9:1) erhält man 187 mg (46 %) der Titelverbindung als farblose Kristalle.
Schmp.: 154-155°C
R_{f}= 0,20 (Dichlormethan:Methanol 9:1)
MS (FAB) m/z = 409 (M+H)⁺
¹H-NMR (200 MHZ, D₆-DMSO): δ = 1,3-1,6 (m, 6H, CH₂); 1,84 (s, 3H, COCH₃); 2,33 (m, 4H, CH₂N); 3,45 (m, 4H, CH₂N); 3,91 (dd, I = 8,10 N₂, 1H, H-4 trans); 4,25 (dd, I = 10,10 N₂, 1H, H-4 cis); 4,78 (m, 9H, H-5); 7,40, 7,68 (AB, I = 9 H₂, Harom); 8,13 (s, 2H, Pyridyl H-3,4); 8,25 (m, 1H, NHCO); 8,70 (m, 1H, Pyridyl H-6).

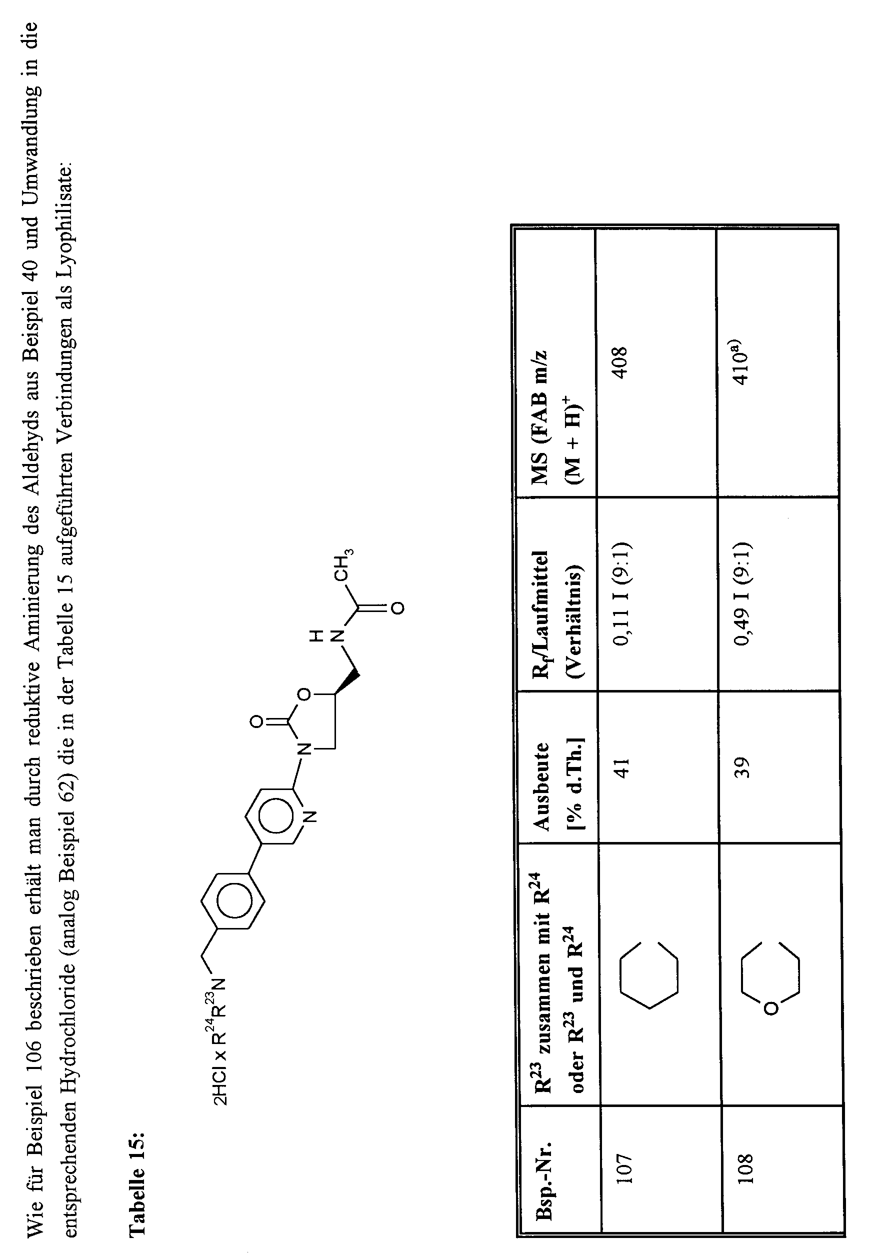

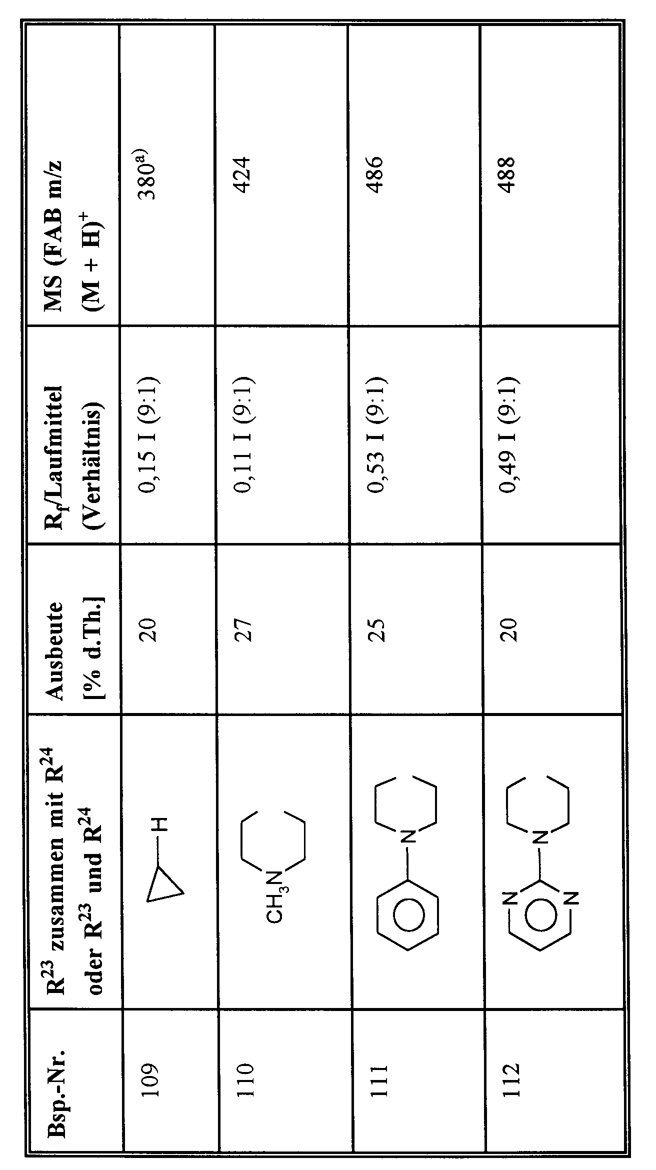

### Beispiel 113

### (5S)-3-[5-(4-(Hydroximinomethyl)-phenyl)-pyridin-2-yl]-5-acetyl-aminomethyl-oxazolidin-2-on

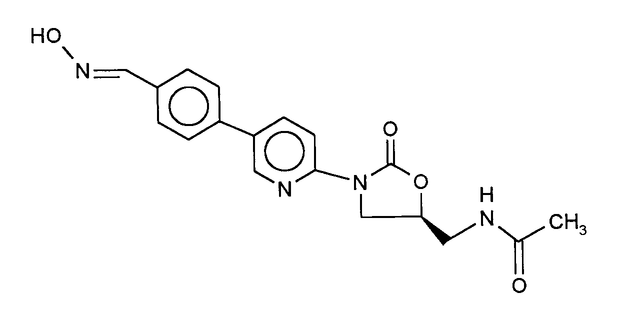

Eine gerührte Suspension von 340 mg (1.00 mmol) des Aldehyds aus Beispiel 40 in 15 ml Ethanol wird mit 0,33 ml (4.00 mmol) Pyridin und 278 mg (4.00 mMol) Hydroxylaminhydrochlorid versetzt und 1 Stunde am Rückfluß erhitzt. Das Gemisch darf abkühlen und wird mit 15 ml Wasser versetzt. Der Niederschlag wird durch Filtration abgetrennt, mehrmals mit Wasser gewaschen und im Vakuum über Sicapent getrocknet. Man erhält 173 mg der Titelverbindung als farblose Kristalle.
Schmp.: 233-234°C
R_{f}= 0,29 (Dichlormethan:Methanol 9:1)
MS (DCl, NH₃) m/z = 355 (M+H)⁺
¹H-NMR (250 MHZ, D₆-DMSO): δ = 1,85 (s, 3H, COCH₃); 3,46 (m, 2H, CH₂N); 3,92 (dd, I = 8,10 Hz, 1H, H-4 trans); 4,27 (dd, I = 10,10 Hz, 1H, H-4 cis); 4,78 (m, 1H, H-5); 7,71, 7,79 (AB, I = 11Hz, 4H, Harom); 8,20 (m, 2H, Pyridyl H-3,4); 8,26 (m, 1H, NHCO); 8,72 (bs, 1H, Pyridyl H-6).

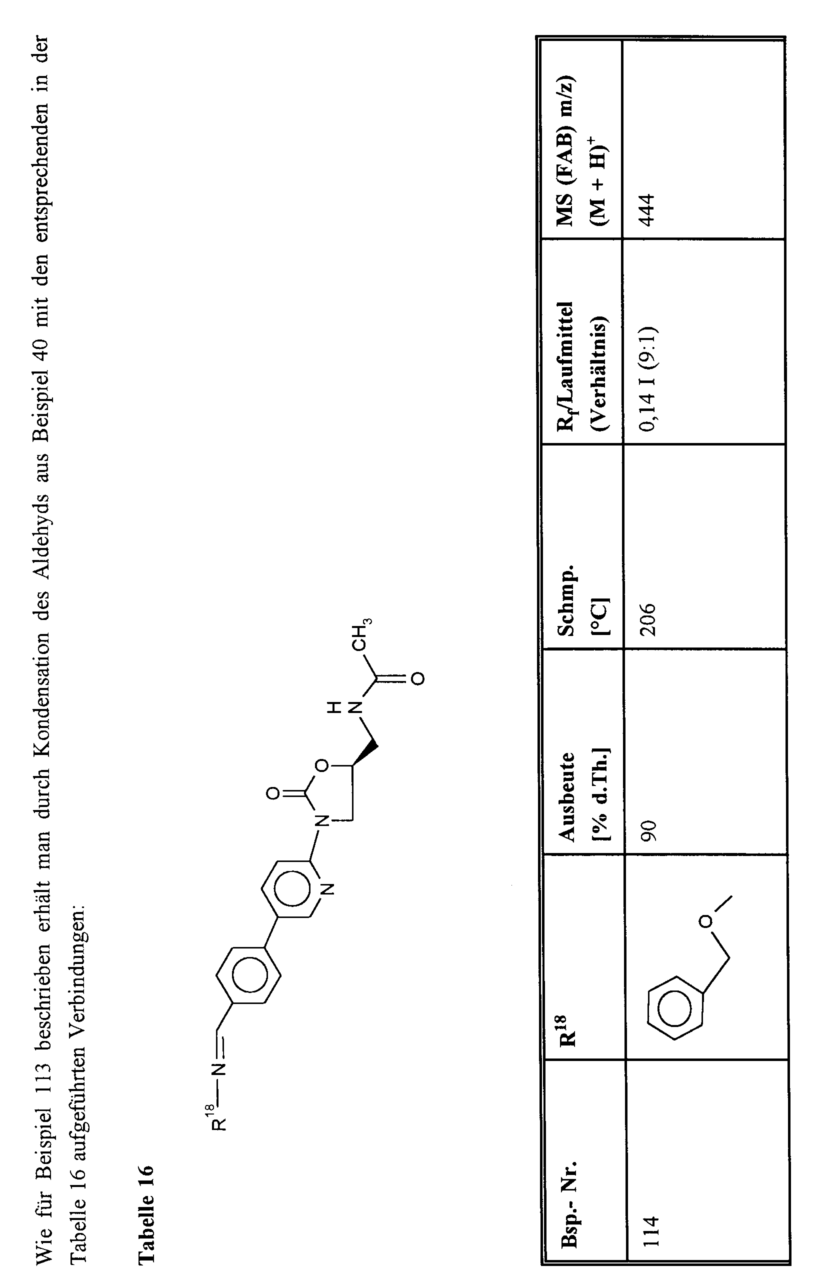

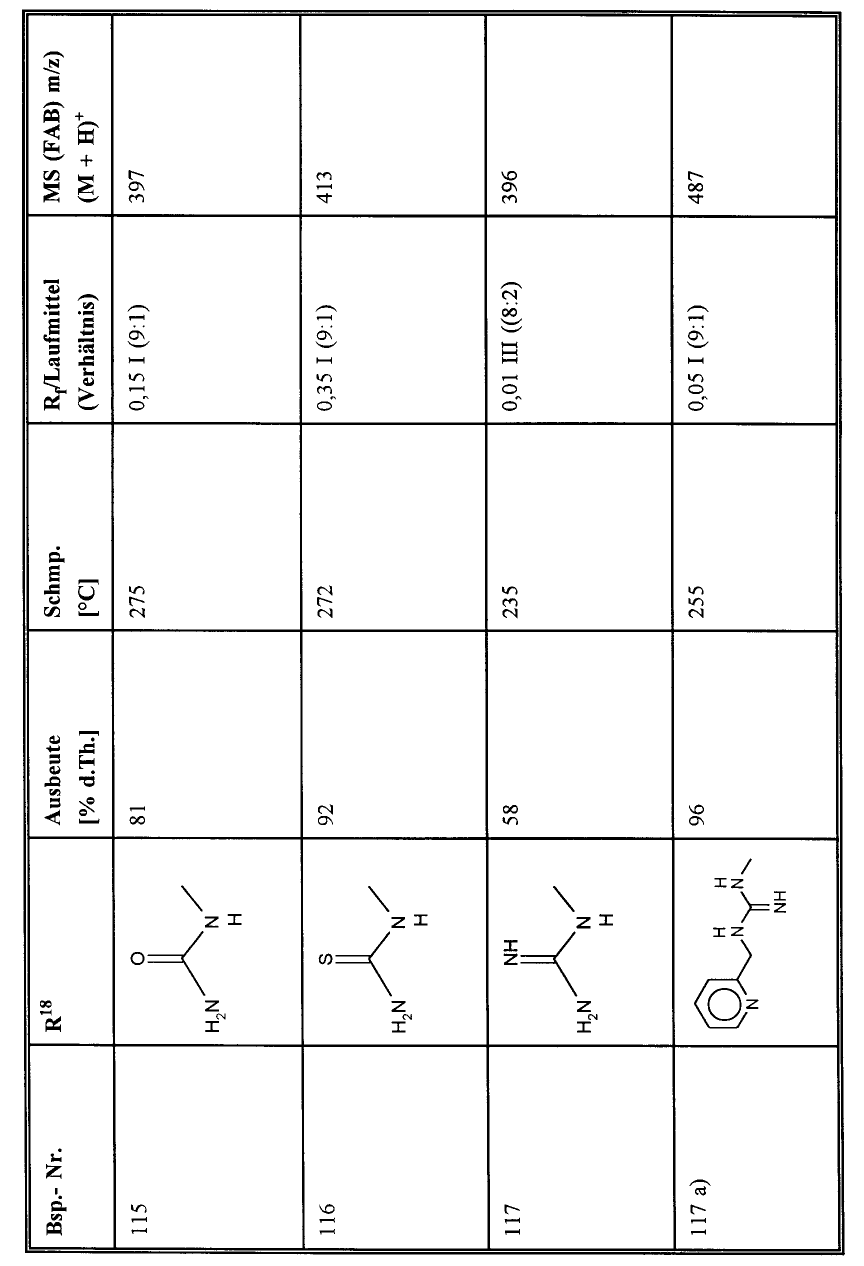

### Beispiel 118

### (5S)-3-[5-(4-Formyl-phenyl)-pyridin-2-yl]-5-acetyl-aminomethyl-oxazolidin-2-on Bisulfitaddukt

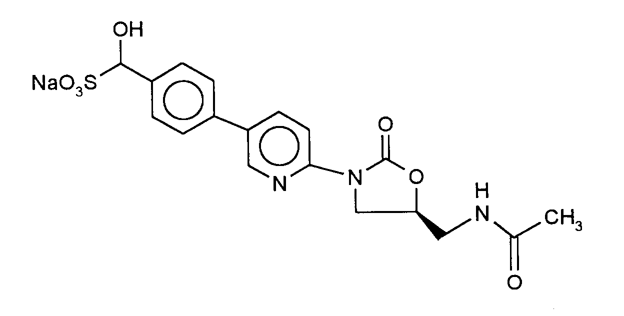

Ein gerührtes Gemisch aus 232 mg (0.50 mmol) des Aldehyds aus Beispiel 40 und 0,2 ml 39 %ige wässrige NaHSO₃-Lösung in 20 ml Ethanol wird im Rückfluß erhitzt. Nach dem Abkühlen wird der Niederschlag durch Filtration abgetrennt, mit Ethanol gewaschen und im Vakuum über Sicapent getrocknet. Man erhält 225 mg (98 %) der Titelverbindung als farblose Kristalle.
Schmp.: >310°C
R_{f}= 0,29 (Dichlormethan:Methanol 9:1)
MS (FAB) m/z = 420 (M)⁺
¹H-NMR (200 MHZ, D₆-DMSO): δ = 1,90 (s, 3H, COCH₃); 3,48 (m, 2H, NCH₂); 3,92 (dd, I = 8,10 Hz, 1H, H-4 trans); 4,26 (dd, I = 10,10 Hz, 1H, H-4 cis); 4,75 (m, 1H, H-5); 5,01 (d, I = 5Hz, 1H, CHOH); 5,92 (d, 5 Hz, 1H, CHOH); 7,57 (m, 2H, Harom); 8,18 (s, 2H, Pyridyl H-3,4); 8,30 (m, 1H, NHCO); 8,70 (s, 1H, Pyridyl H-6).

### Beispiel 119

### (5S)-3-[5-((4-(-n-Butyloxycarbonyl-phenyl)-aminocarbonyl)oxymethyl)-phenyl)-pyridin-2-yl]-5-acetyl-aminomethyl-oxazolin-2-on

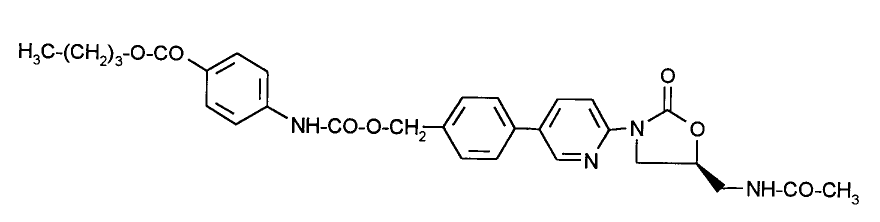

Eine gerührte Lösung von 80 mg (0.25 mmol) des Alkohols aus Beispiel 120 in 35 ml Dichlormethan wird mit 0,11 ml (0,77 mmol) Triethylamin und mit 61 mg (0,28 mmol) 4-Isocyanato-n-butylbenzoat versetzt, wobei ein voluminöser Niederschlag entsteht. Man rührt 1 Stunde bei Raumtemperatur nach, trennt den Niederschlag durch Filtration ab, wäscht mit 3 x 5 ml Dichlormethan und trocknet im Hochvakuum über Sicapent. Man erhält 82 mg (77 %) der Titelverbindung als farblose Kristalle.
Schmp.: 233-234°C
R_{f}= 0,43 (Dichlormethan:Methanol 9:1)
MS (DCI, NH₃) m/z = 561 (M+H)⁺
¹H-NMR (200 MHZ, D₆-DMSO): δ = 0,93 (t, I = 6,5 Hz, 3H, CH₃); 1,40 (m, 2H, CH₂); 1,70 (m, 2H, CH₂), 1,82 (s, 3H, COCH₃); 3,46 (m, 2H, CH₂N); 3,92 (dd, I 8,10 Hz, 1H, h-3 trans); 4,25 (m, 3H, 4 cis, CH₂OCO); 4,75 (m, 1H, H-5); 5,25 (2, 2H, CH₂O); 7,55, 7,61, 7,77, 7,90 (AB, I 10 Hz, 8H, C₆NH₄); 8,18 (s, 2H, Pyridyl H-3,4); 8,28 (m, 1H, NHCO); 8,73 (s, 1H, Pyridyl H-6); 10,23 (s, 1H, NHCOO).

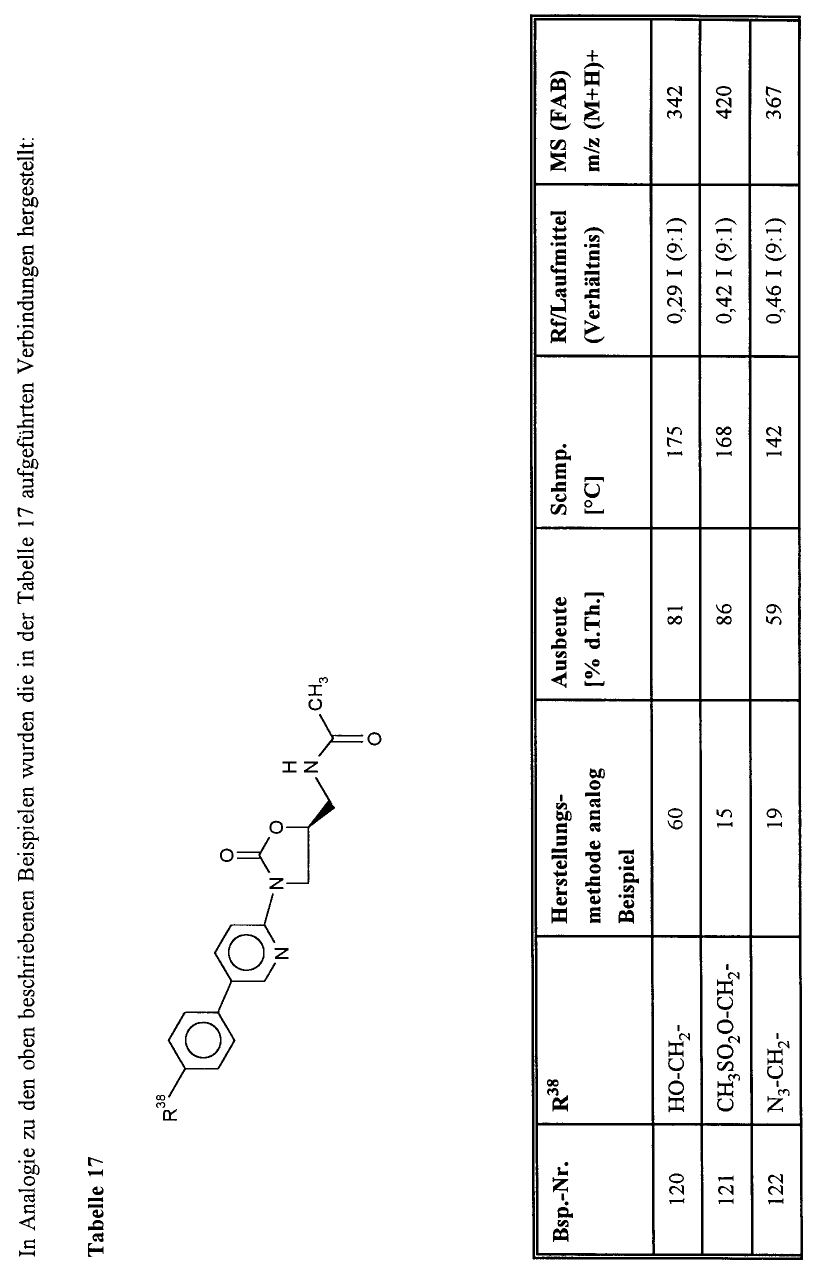

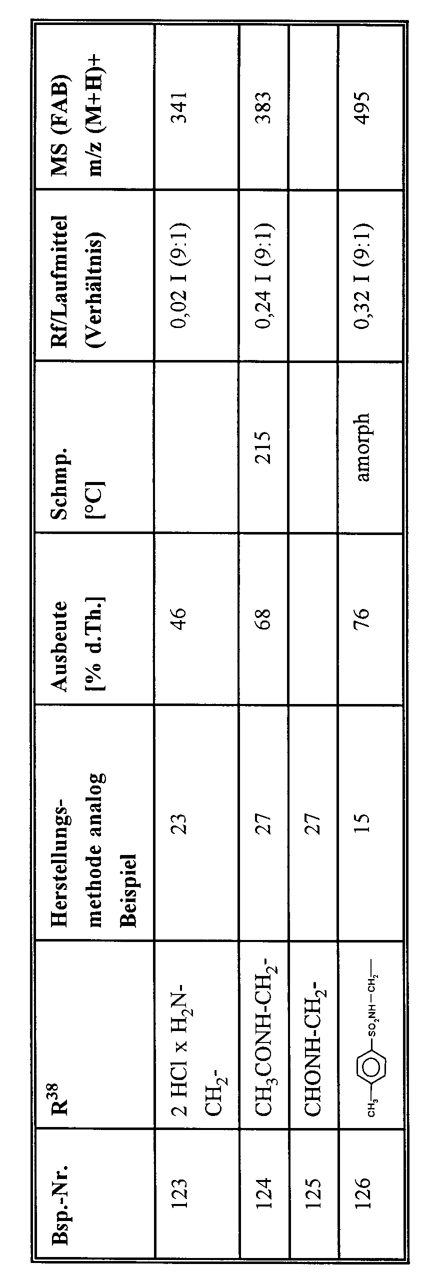

### Beispiel 127

### (5S)-3-[5-(4-(Dimethoxyphosphorylamino-methyl)-phenyl)-pyridin-2-yl]-5-acetyl-aminomethyl-oxazolidin-2-on

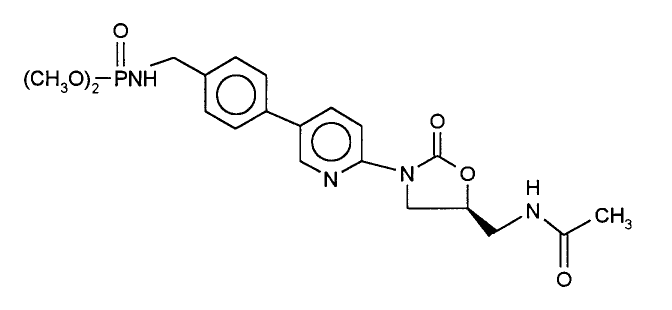

Eine gerührte Lösung von 310 mg (0.85 mmol) des Azids aus Beispiel 122 in 1,5 ml Dimethoxymethan wird auf 70°C erwärmt und man tropft langsam 0,12 ml (1,02 mmol) Trimethylphosphit zu (Gasentwicklung!). Danach rührt man 2 Stunden bei 70°C, laßt das Gemisch abkühlen und gießt dieses in 30 ml Ethylacetat:Wasser 1:1. Die organische Phase wird abgetrennt und über MgSO₄ getrocknet. Nach Abdampfen des Lösungsmittels und Chromatographie des Rückstands an 15 g Kieselgel (Dichlormethan:Methanol 97:3) erhält man 213 mg (56 %) der Titelverbindung als farblose Kristalle.
Schmp.: 131-132°C
R_{f}= 0,28 (Dichlormethan:Methanol 9:1)

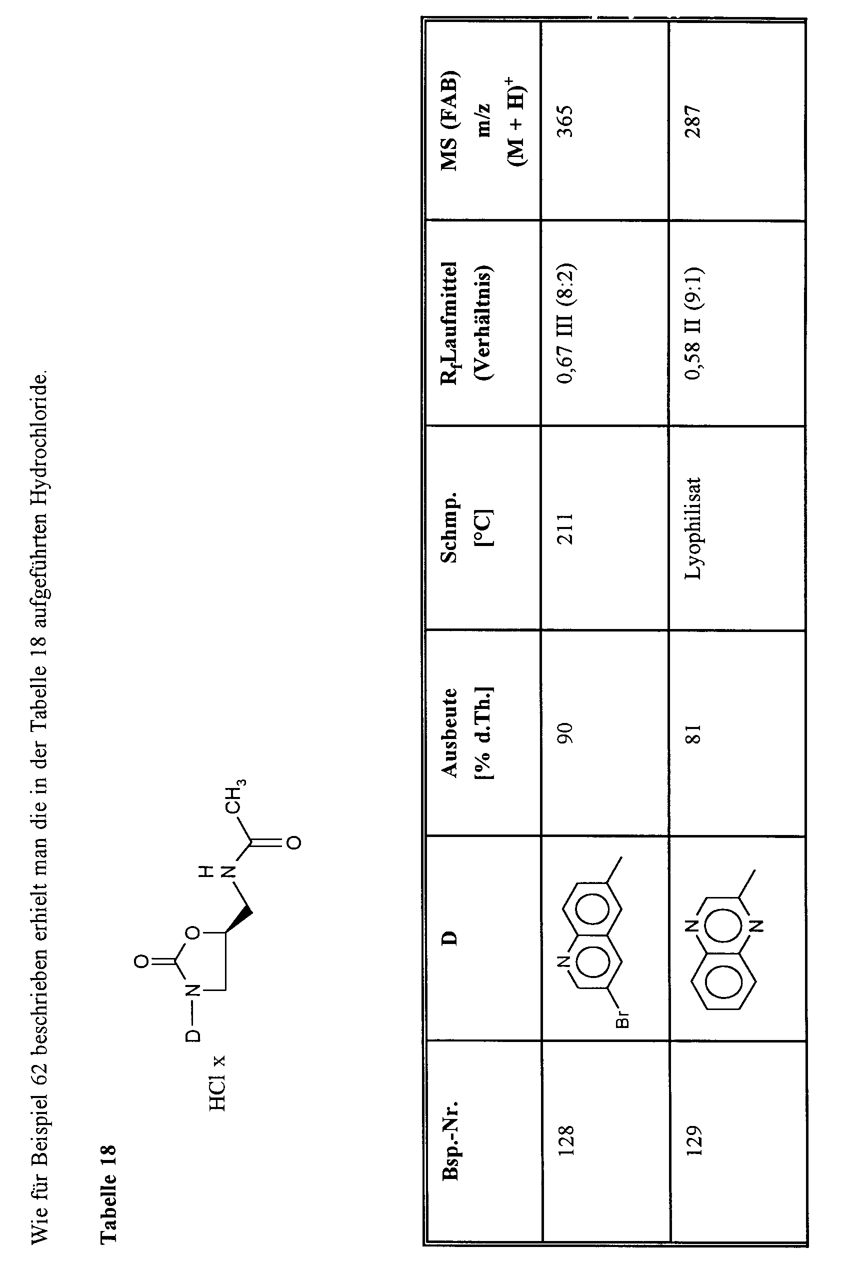

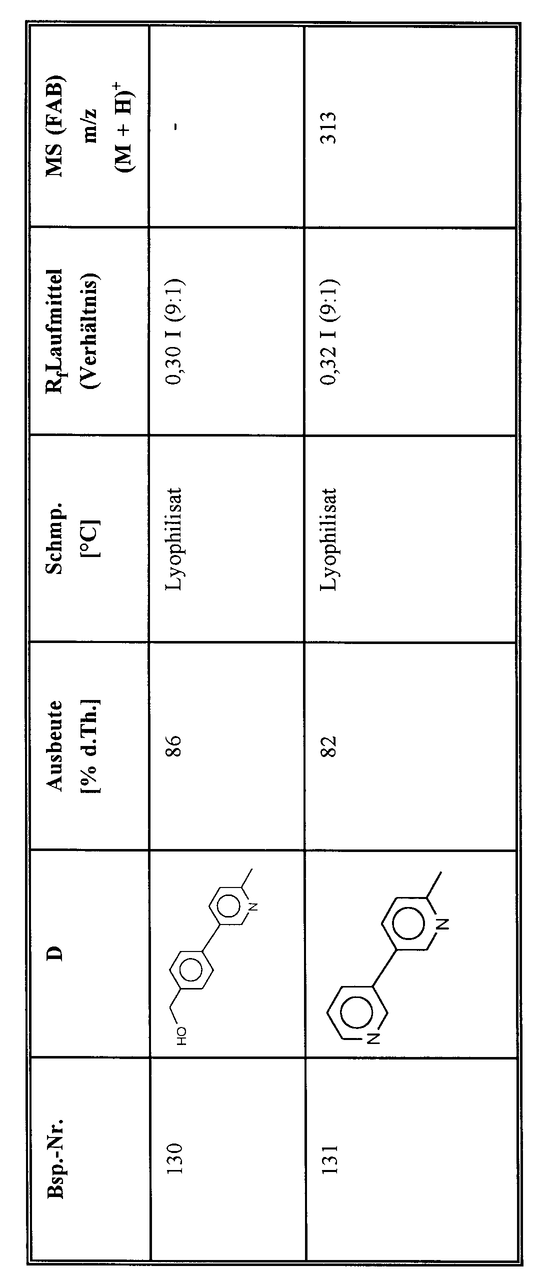

### Beispiel 132

### (5S)-3-[5-(4-(1-Methyl-1H-tetrazol-5-yl-thiomethyl)-phenyl)-pyridin-2-yl]-5-acetyl-aminomethyl-oxazolidin-2-on

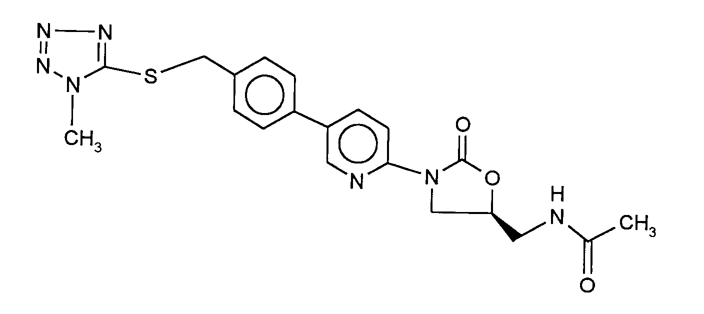

Eine Lösung von 122 mg (0.29 mmol) des Mesylats aus Beispiel 121 in 2 ml Acetonitril wird mit 0,05 ml (0,32 mmol) Triethylamin und 36 mg (0,31 mmol) 1-Methyltetrazol-5-thiol versetzt und 1 Stunde bei Raumtemperatur gerührt. Danach wird 1 g Kieselgel zugegegeben, das Lösungsmittel wird im Vakuum abgedampft und der Rückstand durch Chromatographie an 10 g Kieselgel (Ethylacetat) gereinigt.
Man erhält man 72 mg (57 %) der Titelverbindung als farblose Kristalle.
Schmp.: 154-155°C
R_{f}= 0,10 (Ethylacetat)
MS (DCI, NH₃)m/z = 440 (M + H)⁺
¹H-NMR (300 MHz, D₆-DMSO): δ = 1,85 (s, 3H, COCH₃); 3.45 (m, 2H, CH₂N); 3,89 (s, 3H, NCH₃); 3,91 (m, 1H, H-4 trans); 4,26 (dd, I . 10,10 Hz, 1H, H-4 cis); 4,58 (s, 2H, CH₂); 4,77 (m, 1H, H-5); 7,50, 7,68 (AB, I = 9 Hz, 4H, Harom); 8,16 (s, 2H, Pyridyl H-3,4); 8,25 (m 1H, NHCO); 8,70 (s, 1H, Pyridyl H-6).

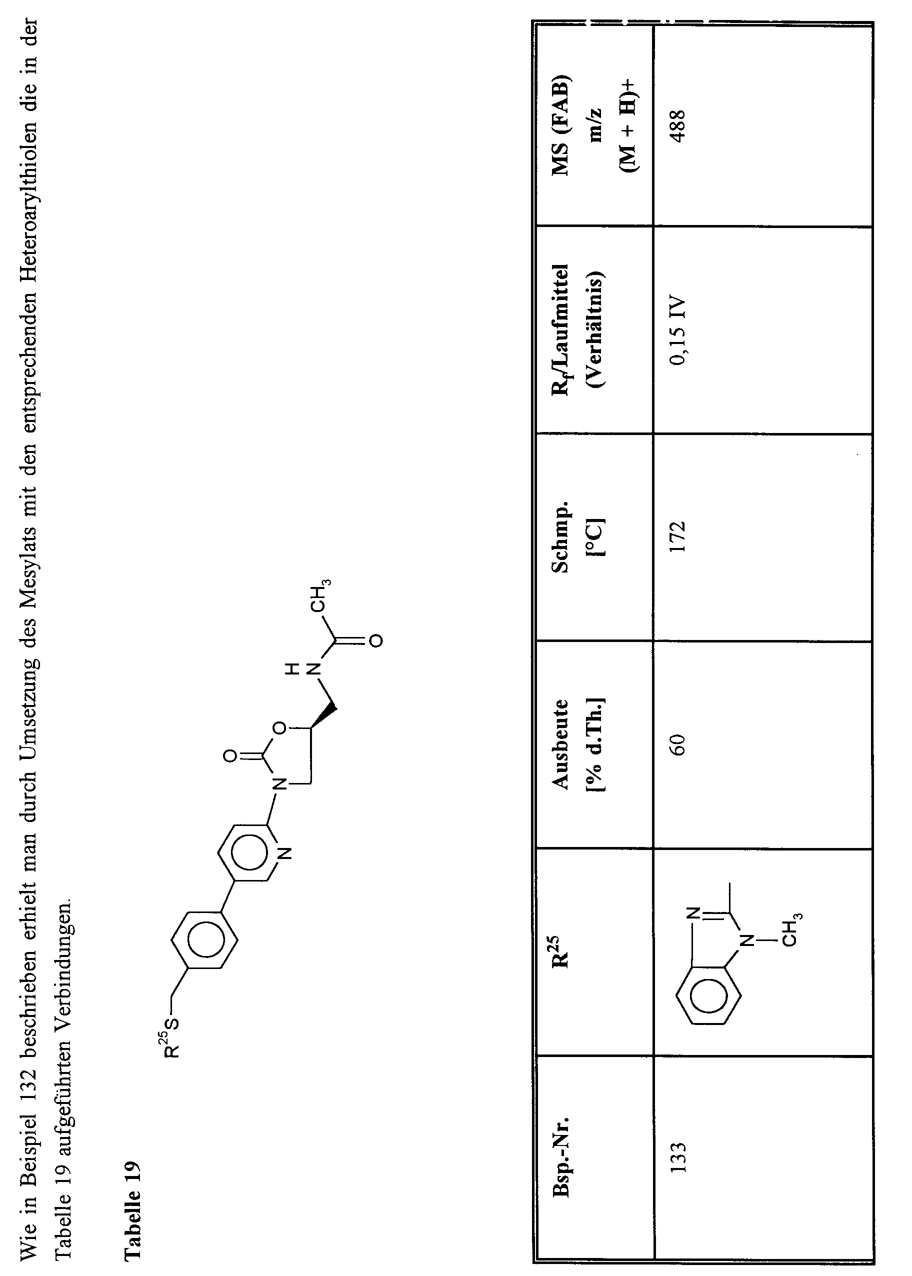

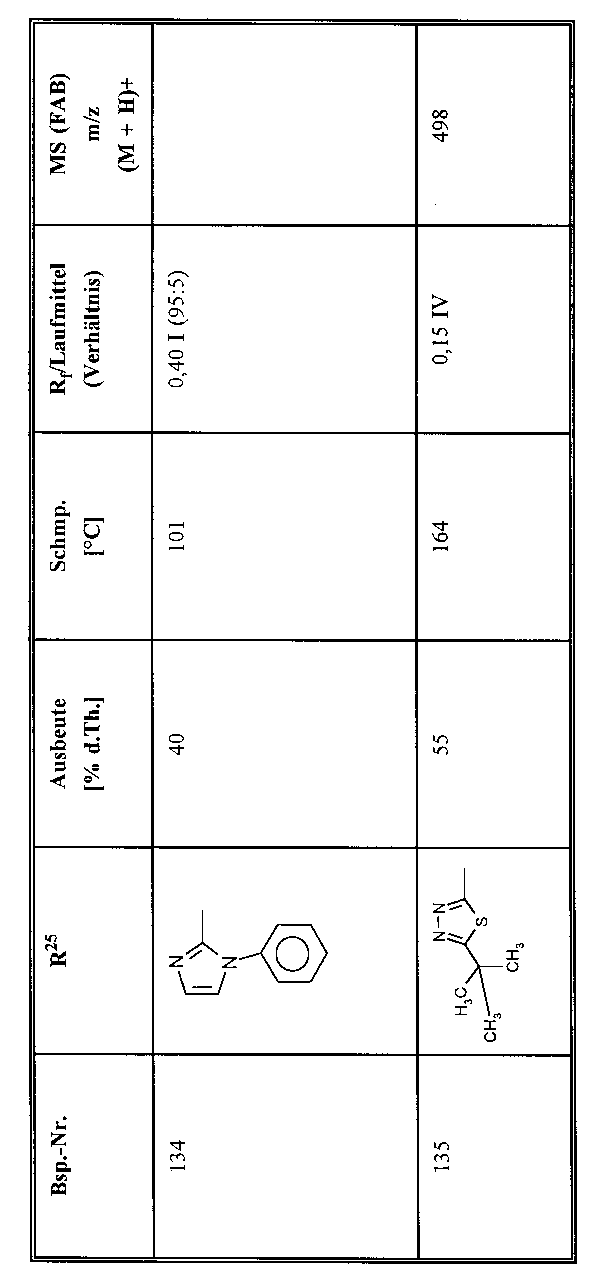

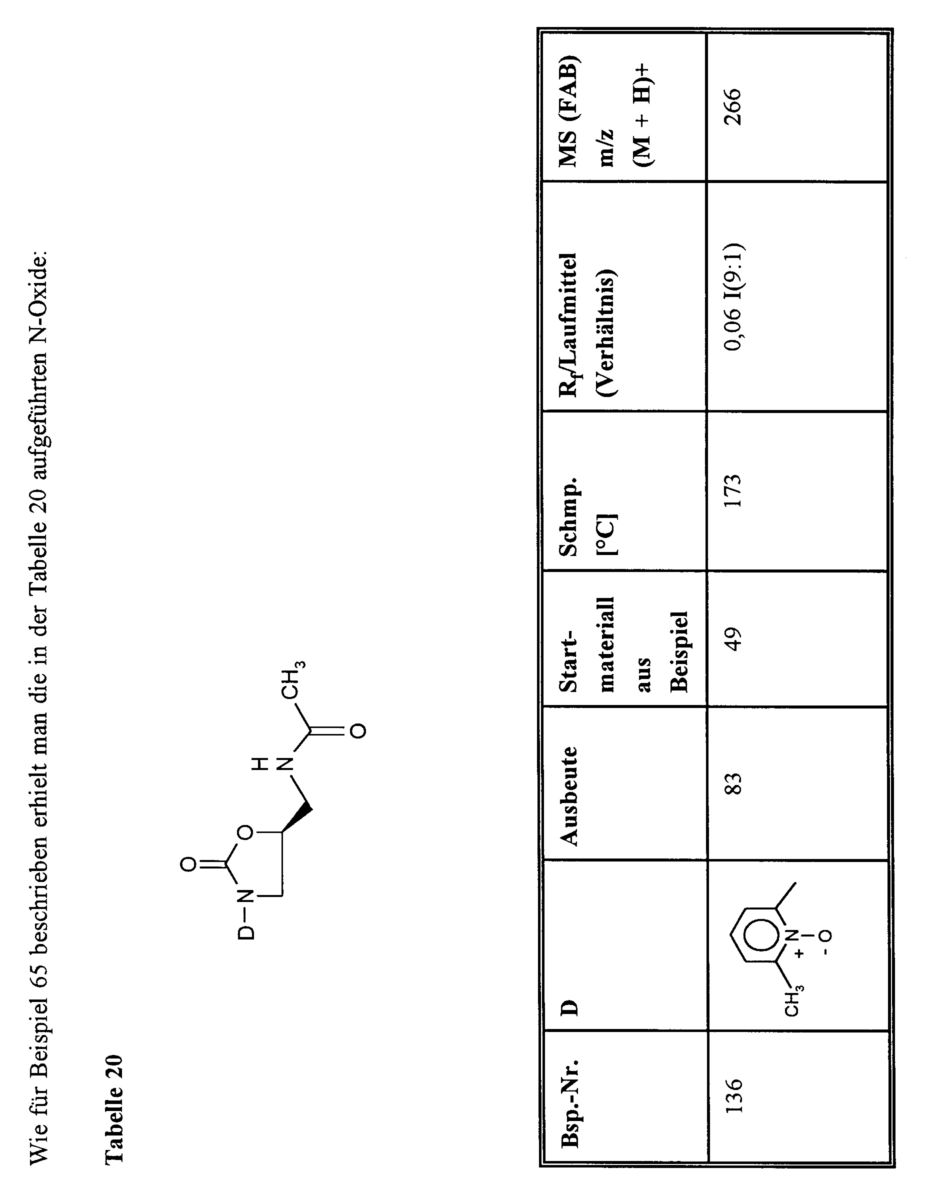

### Beispiel 137

### (5S)-3-[6-(Hydroxymethyl)-pyridin-2-yl]-5-acetyl-aminomethyl-oxazolidin-2-on

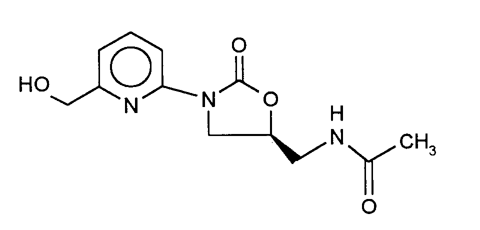

Eine gerührte auf 0°C gekühlte Lösung von 5,80 g (21,88 mmol) als N-Oxids aus Beispiel 138 in 50 ml wasserfreiem DMF wird innerhalb von 6 min mit 18,50 ml (131.00 mmol) Trifluoressigsäurehydrid versetzt. Danach wird das Kühlbad entfernt und man rührt 1 Stunde bei Raumtemperatur nach. Danach wird das Reaktionsgemisch in 130 ml eiskalte gesättigte NO₂CO₃-Lösung eingerührt und 1 h gut durchgerührt, wobei sich das Gemisch auf Raumtemperatur erwärmen darf. Die wässrige Phase wird mit 50 ml Ethylacetat (8x) und mit 50 ml Dichlormethan (5x) extrahiert und die vereinigten Extrakte werden über MgSO₄ getrocknet. Nach Abdampfen des Lösungsmittels im Vakuum und Chromatographie des Rückstandes an 100 g Kieselgel (Dichlormethan:Methanol 9:1) erhält man 5,79 g (98 %) der Titelverbindung als hellgelbe Kristalle.
Schmp.: 138°C
R_{f}= 0,26 (Dichlormethan:Methanol 9:1)
MS (DCI, NH₃)m/z = 266 (M + H)⁺
¹H-NMR (200 MHz, D₆-DMSO): δ = 1,84 (s, 3H, COCH₃); 3.43 (m, 2H, CH₂N); 3,82 (dd, I = 8,10 Hz, 1H, H-4 trans); 4,20 (dd, I = 10,20, 1H, H-4 cis); 4,50 (d, I = 6 Hz, 2H, CH₂OH); 4,72 (m, 1H, H-5); 5,42 (t, I = 6 Hz, 1H, CH₂OH); 7,21 (d, I = 8 Hz, 1H, Pyridyl-H); 7,90 (m, 2H, Pyridyl-H); 8,25 (bt, I = 7 Hz, 1H, NHCO).

## Patentansprüche

1. Heteroaryl-oxazolidinone der allgemeinen Formel (I) 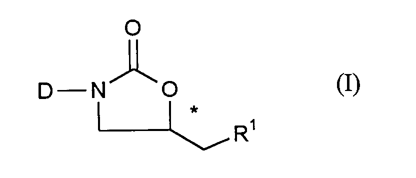 in welcher
R¹ für Azido, Hydroxy oder für eine Gruppe der Formel -OR², O-SO₂R³ oder -NR⁴R⁵ steht,
worin
R² geradkettiges oder verzweigtes Acyl mit bis zu 8 Kohlenstoffatomen oder eine Hydroxyschutzgruppe bedeutet,
R³ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
R⁴ und R⁵ gleich oder verschieden sind und
Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten,
oder
R⁴ oder R⁵ eine Gruppe der Formel -CO-R⁶ bedeutet, worin
R⁶ Cycloalkyl mit 3 bis 6 Kohlenstoffatomen, geradkettiges oder verzweigtes Alkyl mit bis zu 8 Kohlenstoffatomen, Phenyl oder Wasserstoff bedeutet,
D für einen über ein Kohlenstoffatom direkt gebundenen 6-gliedrigen, aromatischen Heterocyclus mit mindestens einem Stickstoffatom steht, oder
für einen über ein Kohlenstoffatom, jeweils 6-gliedrigen, bi- oder tricyclischen aromatischen Rest mit mindestens einem stickstoffhaltigem Ring steht, oder
für β-Carbolin-3-yl oder für über den 6-Ring direkt gebundenes Indolizinyl steht, wobei die Cyclen gegebenenfalls jeweils bis zu 3-fach gleich oder verschieden durch Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR⁷R⁸ substituiert sein kann,
worin
R⁷ und R⁸ gleich oder verschieden sind und
Wasserstoff Formyl, geradkettiges oder verzweigtes Alkyl oder Acyl mit jeweils bis zu 4 Kohlenstoffatomen, Phenyl oder Cycloalkyl mit 3 bis 6 Kohlenstoffatomen bedeuten, oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch Phenyl, Pyrimidyl oder geradkettiges oder verzweigtes Alkyl oder Acyl mit jeweils bis zu 3 Kohlenstoffatomen substituiert sein kann,
und/oder
die Cyclen gegebenenfalls durch eine Gruppe der Formel -NR⁷'R⁸' substituiert sind,
worin
R^{7'} und R^{8'} gleich oder verschieden sind und die oben angegebene Bedeutung von R⁷ und R⁸ haben und mit dieser gleich oder verschieden sind,
und/oder
die Cyclen gegebenenfalls durch (C₂-C₈)-Alkenylphenyl, Phenyl oder durch einen 5- oder 6-gliedrigen gesättigten oder ungesättigten Heterocyclus mit bis zu 3 Heteroatomen aus der Reihe S, N und/oder O substituiert sind, die ihrerseits gegebenenfalls durch eine Gruppe der Formel -CO-NR⁹R¹⁰, -NR¹¹R¹², -NR¹³-SO₂-R¹⁴, R¹⁵R¹⁶N-SO₂-, R¹⁷⁻S(O)ₐ-, R¹⁸-N=CH- oder durch den Rest -CH(OH)-SO₃R²⁰ substituiert sind,
worin
a eine Zahl 0, 1 oder 2 bedeutet,
R⁹, R¹⁰, R¹³, R¹⁵ und R¹⁶ gleich oder verschieden sind und Wasserstoff, geradkettiges oder verzweigtes Alkyl mit bis zu 6 Kohlenstoffatomen, Tolyl oder Phenyl bedeuten,
R¹¹ und R¹² gleich oder verschieden sind und die oben angegebene Bedeutung von R⁷ und R⁸ haben und mit dieser gleich oder verschieden sind,
R¹⁴ und R¹⁷ gleich oder verschieden sind und die oben angegebene Bedeutung von R³ haben und mit dieser gleich oder verschieden sind,
R¹⁸ Hydroxy, Benzyloxy oder einen Rest der Formel -NH-CO-NH₂, 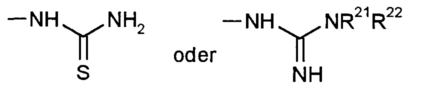 bedeutet,
worin
R²¹ und R²² gleich oder verschieden sind und Wasserstoff oder geradkettiges oder verzweigtes Alkyl mit bis zu 4 Kohlenstoffatomen bedeuten, das seinerseits durch Phenyl oder Pyridyl substituiert sein kann,
R²⁰ Wasserstoff oder ein Natriumion bedeutet,
und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, Azido durch geradkettiges oder verzweigtes Alkoxy oder Acyl mit bis zu 5 Kohlenstoffatomen oder durch eine Gruppe der Formel -NR²³R²⁴, R²⁵-S-, R²⁶-SO₂O- oder 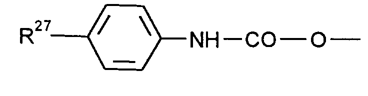 substituiert sein kann,
worin
R²³ und R²⁴ die oben angegebene Bedeutung von R⁷ und R⁸ haben und mit dieser gleich oder verschieden sind, oder einen Rest der Formel -P(O) (OR²⁸) (OR²⁹) oder R³⁰-SO₂- bedeuten,
worin
R²⁸ und R²⁹ gleich oder verschieden sind und Wasserstoff oder geradkettiges oder verzweigtes Alkyl mit bis zu 3 Kohlenstoffatomen bedeuten
R³⁰ Methyl, Phenyl oder Tolyl bedeutet,
R²⁵ einen Rest der Formel 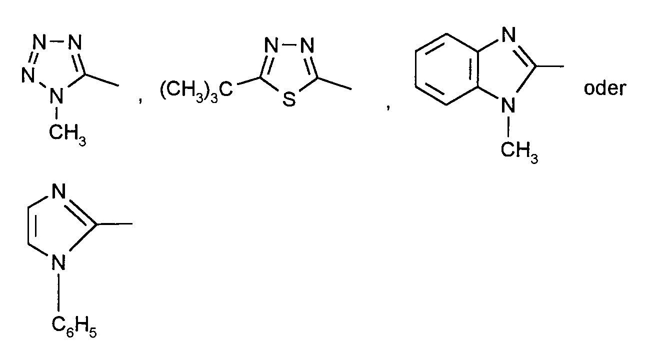 bedeutet,
R²⁶ geradkettiges oder verzweigtes Alkyl mit bis zu 3 Kohlenstoffatomen bedeutet,
R²⁷ geradkettiges oder verzweigtes Alkoxycarbonyl mit bis zu 6 Kohlenstoffatomen oder Carboxy bedeutet
und/oder
die Cyclen gegebenenfalls durch einen Rest der Formel 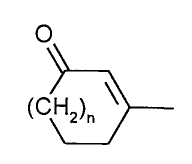 substituiert sind,
worin
n eine Zahl 0, 1 oder 2 bedeutet
und deren Salze und N-Oxide.

2. Heteroaryl-oxazolidinone nach Anspruch 1, in denen
R¹ für Azido, Hydroxy oder für eine Gruppe der Formel -OR², -OSO₂R³ oder -NR⁴R⁵ steht,
worin
R² geradkettiges oder verzweigtes Acyl mit bis zu 6 Kohlenstoffatomen oder Benzyl bedeutet,
R³ geradkettiges oder verzweigtes Alkyl mit bis zu 3 Kohlenstoffatomen, Phenyl oder Toluolyl bedeutet,
R⁴ und R⁵ gleich oder verschieden sind und
Cyclopropyl, Cycylopentyl, Cyclohexyl, Wasserstoff, Phenyl oder geradkettiges oder verzweigtes Alkyl mit bis zu 6 Kohlenstoffatomen, tert. Butoxycarbonyl oder Benzyloxycarbonyl bedeuten,
oder
R⁴ oder R⁵ eine Gruppe der Formel -CO-R⁶ bedeutet,
worin
R⁶ Cyclopropyl, Cyclobutyl, Cyclopentyl, Cyclohexyl oder geradkettiges oder verzweigtes Alkyl mit bis zu 6 Kohlenstoffatomen, Phenyl oder Wasserstoff bedeutet,
D für Cinnolinyl, Pteridinyl, Acridinyl, Chinazolinyl, Chinoxalinyl, Naphthyridinyl, Phthalaziny, Chinolyl, Isochinolyl, Pyridyl, Pyrazinyl, Pyrimidinyl oder Pyridazinyl steht, wobei die Cyclen gegebenenfalls jeweils bis zu 3-fach gleich oder verschieden durch durch Carboxy, Fluor, Chlor, Brom, Jod, Cyano, Mercapto, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR⁷R⁸ gegebenenfalls substituiert sein kann,
worin
R⁷ und R⁸ gleich oder verschieden sind und
Wasserstoff, Formyl geradkettiges oder verzweigtes Alkyl oder Acyl mit jeweils bis zu 3 Kohlenstoffatomen, Phenyl, Cyclopropyl, Cyclopentyl oder Cyclohexyl bedeuten, oder gemeinsam mit dem Stickstoffatom einen Morpholinyl-, Pyrrolidinyl-, Piperazinyl- oder Piperidylring bilden, die gegebenenfalls, auch über die freie N-Funktion, durch Phenyl, Pyrimidyl, Methyl, Ethyl oder Acetyl substituiert sind,
und/oder die Cyclen gegebenenfalls durch einen Rest der Formel durch eine Gruppe der Formel -NR^{7'}R^{8'} substituiert sind,
worin
R^{7'} und R^{8'} die oben angegebene Bedeutung von R⁷ und R⁸ haben und mit dieser gleich oder verschieden sind, und/oder die Cyclen gegebenenfalls durch (C₂-C₄)-Alkenylphenyl, Phenyl, Pyridyl oder Thienyl substituiert sind, die ihrerseits gegebenenfalls durch eine Gruppe der Formel -CO-NR⁹R¹⁰, -NR¹¹R¹², -NR¹³-SO₂-R¹⁴, R¹⁵R¹⁶N-SO₂-, R¹⁷-S(O)ₐ-, R¹⁸-N=CH- oder durch den Rest -CH(OH)-SO₃R²⁰ substituiert sind,
worin
a eine Zahl 0, 1 oder 2 bedeutet,
R⁹, R¹⁰, R¹³, R¹⁵ und R¹⁶ gleich oder verschieden sind und Wasserstoff, geradkettiges oder verzweigtes Alkyl mit bis zu 4 Kohlenstoffatomen, Tolyl oder Phenyl bedeuten,
R¹¹ und R¹² gleich oder verschieden sind und die oben angegebene Bedeutung von R⁷ und R⁸ haben und mit dieser gleich oder verschieden sind,
R¹⁴ und R¹⁷ gleich oder verschieden sind und die oben angegebene Bedeutung von R³ haben und mit dieser gleich oder verschieden sind,
R¹⁸ Hydroxy, Benzyloxy oder einen Rest der Formel -NH-CO-NH₂, 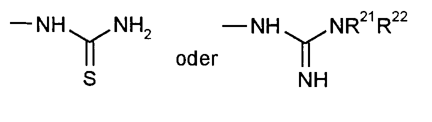 bedeutet,
worin
R²¹ und R²² gleich oder verschieden sind und Wasserstoff oder geradkettiges oder verzweigtes Alkyl mit bis zu 3 Kohlenstoffatomen bedeutet, das seinerseits durch Phenyl oder Pyridyl substituiert sein kann,
R²⁰ Wasserstoff oder ein Natriumion bedeutet
und/oder ihrerseits gegebenenfalls bis zu 2-fach gleich oder verschieden durch Carboxy, Fluor, Chlor, Brom, Jod, Cyano, Mercapto, Trifluormethyl, Formyl, Nitro, Phenyl, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, Azido durch geradkettiges oder verzweigtes Alkoxy oder Acyl mit bis zu 4 Kohlenstoffatomen oder durch eine Gruppe der Formel -NR²³R²⁴ , R²⁵-S-, R²⁶-SO₂O- oder 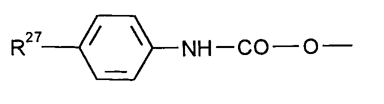 substituiert sein kann,
worin
R²³und R²⁴ die oben angegebene Bedeutung von R⁷ und R⁸ haben und mit dieser gleich oder veschieden sind, oder einen Rest der Formel -(P) (O) (OR²⁸) (OR²⁹) oder R³⁰-SO₂- bedeuten,
worin
R²⁸ und R²⁹ gleich oder verschieden sind und Wasserstoff, Methyl oder Ethyl bedeuten,
R³⁰ Methyl, Phenyl oder Tolyl bedeutet,
R²⁵ einen Rest der Formel 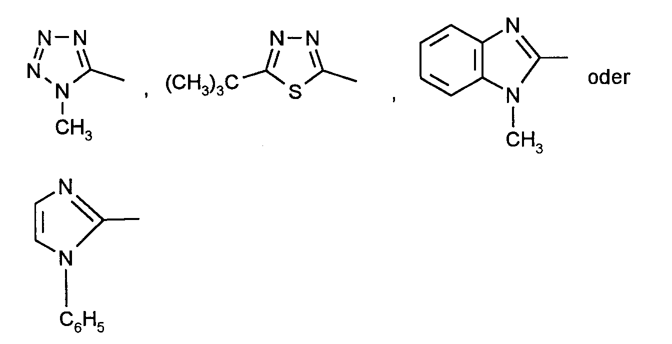 bedeutet,
R²⁶ Methyl, Ethyl, Propyl oder Isopropyl bedeutet,
R²⁷ geradkettiges oder verzweigtes Alkoxycarbonyl mit bis zu 5 Kohlenstoffatomen oder Carboxy bedeutet
und/oder
die Cyclen gegebenenfalls durch einen Rest der Formel 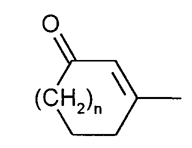 substituiert sind,
worin
n eine Zahl 0, 1 oder 2 bedeutet
und deren Salze und N-Oxide.

3. Heteroaryl-oxazolidinone nach Anspruch 1, in denen
R¹ für Azido, Hydroxy oder für eine Gruppe der Formel -OR², -OSO₂R³ oder -NR⁴R⁵ steht, worin
R² geradkettiges oder verzweigtes Acyl mit bis zu 6 Kohlenstoffatomen bedeutet,
R³ Methyl, Ethyl, Phenyl oder Toluolyl bedeutet,
R⁴ und R⁵ gleich oder verschieden sind und Cyclopropyl, Cyclopentyl, Cyclohexyl, Wasserstoff, Phenyl oder geradkettiges oder verzweigtes Alkyl mit bis zu 5 Kohlenstoffatomen bedeuten,
oder
R⁴ oder R⁵ eine Gruppe der Formel -CO-R⁶ bedeutet,
worin
R⁶ Cyclopropyl, Cyclopentyl, Cyclohexyl oder geradkettiges oder verzweigtes Alkyl mit bis zu 4 Kohlenstoffatomen, Wasserstoff oder Phenyl bedeutet,
D für Cinnolinyl, Chinoxalinyl, Naphthyridinyl, Phthalazinyl, Chinolyl, Isochinolyl, Pyridyl, Pyrazinyl, Pyrimidinyl oder Pyridazinyl steht, wobei die Cyclen gegebenenfalls jeweils bis zu 2-fach gleich oder verschieden durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR⁷R⁸ gegebenenfalls substituiert sein kann,
worin
R⁷ und R⁸ gleich oder verschieden sind und Wasserstoff, Formyl, Acetyl, Methyl oder Cylopropyl bedeuten,
oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl, Phenyl, Pyrimidyl oder Acetyl substituiert sind,
und/oder die Cyclen gegebenenfalls durch eine Gruppe der Formel -NR⁷'R^{8'} substituiert sind,
worin
R^{7'} und R^{8'} die oben angegebene Bedeutung von R⁷ und R⁸ haben und mit dieser gleich oder verschieden sind,
und/oder
die Cyclen gegebenenfalls durch 2-Phenylvinyl, Phenyl, Pyridyl oder Thienyl substituiert sind, die ihrerseits gegebenenfalls durch eine Gruppe der Formel -CO-NR⁹R¹⁰ , -NR¹¹R¹², R¹⁸-N=CH- oder durch den Rest -CH(OH)-SO₃-R²⁰ substituiert sind,
worin
R⁹ und R¹⁰ gleich oder verschieden sind und Wasserstoff oder Methyl bedeuten,
R¹¹ und R¹² gleich oder verschieden sind und die oben angegebene Bedeutung von R⁷ und R⁸ haben und mit dieser gleich oder verschieden sind,
R¹⁸ Hydroxy, Benzyloxy oder einen Rest der Formel -NH-CO-NH₂, 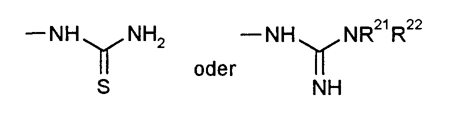 oder bedeutet,
worin
R²¹ und R²² gleich oder verschieden sind und Wasserstoff, Methyl oder Ethyl bedeuten, die ihrerseits durch Phenyl oder Pyridyl substituiert sein können,
R²⁰ Wasserstoff oder ein Natriumion bedeutet und/oder ihrerseits gegebenenfalls bis zu 2-fach gleich oder verschieden durch Carboxy, Fluor, Chlor, Brom, Jod, Cyano, Formyl, Trifluormethyl, Nitro, Phenyl, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, Azido, durch geradkettiges oder verzweigtes Alkoxy oder Acyl mit bis zu 4 Kohlenstoffatomen oder durch eine Gruppe der Formel NR²³R²⁴ , R²⁵-S-, R²⁶-SO₂O- oder 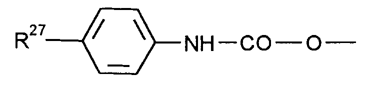 substituiert sein kann,
worin
R²³ und R²⁴ die oben angegebene Bedeutung von R⁷ und R⁸ haben und mit dieser gleich oder verschieden sind, oder einen Rest der Formel -P(O) (OCH₃)₂ oder R³⁰-SO₂- bedeuten
worin
R³⁰ Methyl, Phenyl oder Tolyl bedeutet,
R²⁵ einen Rest der Formel 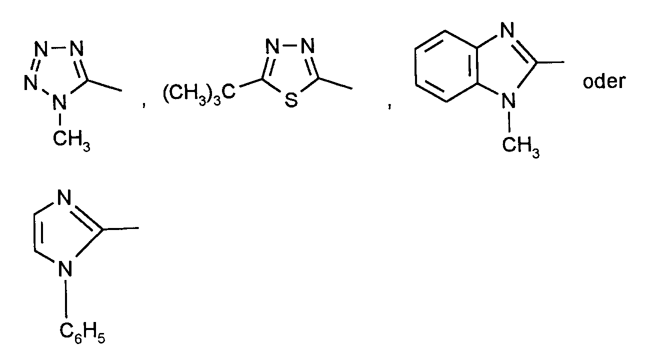 bedeutet,
R²⁶ Methyl, Ethyl oder Propyl bedeutet,
R²⁷ geradkettiges oder verzweigtes Alkoxycarbonyl mit bis zu 4 Kohlenstoffatomen bedeutet
und/oder
die Cyclen gegebenenfalls durch einen Rest der Formel 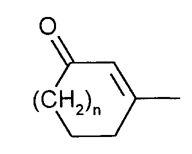
substituiert sind,
worin
n eine Zahl 0, 1 oder 2 bedeutet
und deren Salze und N-Oxide.

4. Verwendung der Heteroaryl-oxazolidinone nach den Ansprüchen 1 bis 3 zur Herstellung von Arzneimitteln.

5. Arzneimittel enthaltend Heteroaryl-oxazolidinone gemäß den Ansprüchen 1 bis 3.
